# EUROPEAN PATENT SPECIFICATION

(11) **EP 1 222 194 B1**
(45) Date of publication and mention of the grant of the patent: **15.06.2005**
(21) Application number: 00966807.0
(22) Date of filing: 21.09.2000
(51) Int. Cl.: C07D 501/59, A61K 31/546, A61P 31/04, C07D 213/70, C07D 285/08, C07F 9/6506

(54) **7-ACYLAMINO-3-HETEROARYLTHIO-3-CEPHEM CARBOXYLIC ACID ANTIBIOTICS AND PRODRUGS THEREOF**
7-ACYLAMINO-3-HETEROARYLTHIO-3-CEPHEMCARBONSÄURE ANTIBIOTIKA UND DEREN VORWIRKSTOFFE
ANTIBIOTIQUES A L'ACIDE 7-ACYLAMINO-3-HETEROARYLTHIO-3-CEPHEM CARBOXYLIQUE ET PROMEDICAMENTS DE CES DERNIERS

(30) Priority: 22.09.1999 US 155496 P
(43) Date of publication of application: 17.07.2002
(73) Proprietor: Trine Pharmaceuticals, Inc., Mountainview, CA 94043 (US)
(72) Inventor: HECKER, Scott, J., Los Gatos, CA 95032 (US); CHO, Aesop, Mountain View, CA 94040 (US); GLINKA, Tomasz, W., Cupertino, CA 95014 (US); CALKINS, Trevor, Londonderry, NH 03053 (US); LEE, Ving, J., Los Altos, CA 94024 (US)
(74) Representative: Viering, Jentschura & Partner
(86) International application number: PCT/US2000/026069
(87) International publication number: WO 2001/021623

(56) References cited:
- EP-A- 0 022 245
- EP-A- 0 025 017
- WO-A-92/05156
- WO-A-95/26966
- WO-A-96/02548
- WO-A-96/38450
- WO-A-97/13772
- WO-A-99/58535
- B. BLANK ET AL: "Mercaptopyridinecarboxylic acids, synthesis and hypoglycemic activity" JOURNAL OF MEDICINAL CHEMISTRY, vol. 17, no. 10, 1974, pages 1065-1071, XP002158204 WASHINGTON US
- CHEMICAL ABSTRACTS, vol. 131, no. 10, 6 September 1999 (1999-09-06) Columbus, Ohio, US; abstract no. 131249, KAYASATO J.: "Manufacture of halotriazoles as intermediates for photographic dyes and color couplers" XP002158205 & JP 11 199568 A (SANKYO KAGAKU K.K.) 27 July 1999 (1999-07-27)

## Description

### RELATED APPLICATION

### FIELD OF THE INVENTION

The present invention relates to novel cephalosporin antibiotics and their methods of production and use, as well as prodrugs thereof These compounds exhibit antibiotic activity against a wide spectrum of organisms, including organisms which are resistant to conventional β-lactam antibiotics.

### BACKGROUND OF THE INVENTION

Over the past three decades a large variety of antibiotics has become available for clinical use. One class of antibiotics which has seen remarkable growth are the cephalosporins, over 70 of which have entered clinical use for the treatment of bacterial infections in mammals since 1965. The cephalosporins exhibit their antibacterial activity by inhibiting bacterial peptidoglycan biosynthesis, and have been extremely effective in treating a wide variety of bacterial infections. Cephalosporins that are said to have antibacterial activity are described in U.S. Patent 3,992,377 and U.S. Patent 4,256,739.

Unfortunately, the wide-spread and indiscriminant use of these antibiotics has led to a rapid increase in the number of bacterial strains which are resistant to these compounds. Most importantly, this resistance has emerged among clinically important microorganisms which threaten to limit the utility of presently available cephalosporin antibiotics. In particular, resistant strains of *Salmonella, S. pneumonioe, Enterobacteriaceoe, Staphylococcus aureus*, and *Pseudomonas* have emerged which threaten to undo many of the strides made in reducing mortality and morbidity from bacterial infections.

Bacterial resistance to cephalosporins follows three major pathways: (a) the development of β-lactamases capable of inactivating the β-lactam ring of the cephalosporin; (b) decreased cephalosporin penetration into the bacteria due to changes in bacterial cell wall composition; and (c) poor binding to penicillin-binding proteins (PBPs). The latter pathway is especially important, as the binding of β-lactams to PBPs is essential for inhibiting bacterial cell-wall biosynthesis. Certain Gram-positive bacteria, namely methicillin-resistant Staphylococcus aureus ("MRSA") and Enterococci are highly resistant to β-lactam antibiotics. Resistance in MRSA is due to the presence of high levels of an unusual PBP, PBP2a, which is insensitive, or binds poorly, to β-lactam antibiotics. The activity of β-lactam antibiotics against PBP2a-containing organisms has been shown to correlate well with the binding affinity of the antibiotic to PBP2a. Currently, the glycopeptides vancomycin and teicoplanin are primarily used for MRSA bacteremia. The quinolone antibacterials and some carbapenems, such as imipenem, have been reported to be active against a few MRSA strains, but their use is restricted due to emerging resistant MRSA strains.

Experimental compounds which may possess utility as anti-MRSA or anti-enterococcal bactericides include the glycylcyclines (see, e.g., P.-E. Sum et al., J. Med. Chem., 37, (1994)), FK-037 (see, e.g., H. Ohki et al., J. Antibiotics, 46:359-361 (1993)), RP-59,500 (see, e.g., S.K. Spangler et al., Antimicro. Agents Chemother., 36:856-9 (1992)), the eveminomycin complex (see, e.g., W.E. Sanders et al., Antimicro. Agents Chemother., 6: 232-8 (1974)), the 2-(biaryl)carbapenems (see, e.g., U.S. Patent No. 5,025,006), 3-(benzothiazolylthio)cephems (see, e.g., EP Application No. 527686), 3-(thiazolylthio)carbacephems (see, e.g., R.J. Temansky et al., J. Med. Chem., 36:1971 (1993) and U.S. Patent No. 5,077,287) and arbekacin (S. Kondo, et al. J. Antibiotics 46:531 (1993).

Recent advances in the compounds, compositions and methods useful for treating infections in mammals arising from β-lactam antibiotic resistant bacteria are described in commonly owned International Application No. PCT/US95/03976 and U.S. patent applications Serial Nos. 08/222,262, filed April 1, 1994; 08/369,798, filed January 6, 1995; 08/413,713, 08/413,714, 08/415,065, 08/413,712, 08/415,064, and 08/415,069, all of which were filed on March 29, 1995; 08/455,969, filed May 31, 1995; 08/457,673, filed June 1, 1995; 08/940,508 and 08/937,812, both of which were filed September 29, 1997; 08/730,041, 08/730,439, 08/728,232, 08/430,042, 08/728,233, and 08/730,040, all of which were filed October 11, 1996; and 08/842,915, filed April 17, 1997; all of which are incorporated herein by reference in their entirety, including any drawings.
WO 97/13772 discloses cephalosporin antibiotics which exhibit activity against a wide spectrum of organisms including organisms which are resistant to β-lactam antibiotics and are useful as antibacterial agents. The described compounds, however, show an increased decomposition in rat serum and increased rat clearance rates.
Therefore, there is an need for compounds with better pharmacokinetics.

### SUMMARY OF THE INVENTION

The present invention includes compounds, compositions and methods effective to treat infections in mammals arising from β-lactam antibiotic resistant bacteria. Preferred compounds will have a minimum inhibitory concentration (MIC) that is less that 50%, more preferably less than 10%, and most preferably less than 1% of the MIC of cefotaxime or imipenem for a beta-lactam resistant organism, preferably a methicillin-resistant Staphylococcal organism. Other preferred compounds will be able to prevent or reduce mortality in mice infected with the beta-lactam resistant organism to a greater extent than cefotaxime or imipenem.

Compounds from the class of 7-acylamino-3-heteroarylthio-3-cephem carboxylic acids of this invention have higher chemical reactivity and lower stability towards chemical or enzymatic decomposition than other cephalosporin compounds known in the art. Without wishing to be bound by any particular theory of operation of the invention, it is believed that this is due to an unusual type of substitution at the 3-position of the cephalosporin system. One aspect of the present invention features certain compounds from the class of 7-acylamino-3-heteroarylthio-3-cephem carboxylic acids which display an unexpected advantage over other compounds of this class, by virtue of lowered susceptibility to decomposition by enzymes present in mammalian serum. Compounds having this property are described below, and data is presented showing their improved stability in mammalian serum. In addition to this increased stability, there is also an improvement in pharmacokinetic parameters of such compounds and especially a lowered clearance of such compounds from the body. Pharmacological data demonstrating this lowered clearance is shown below, as well as improved efficacy in an animal model of infection due to this lowered clearance. One aspect of the present invention features certain compounds from the class of 7-acylamino-3-heteroarylthio-3-cephem carboxylic acids which combine the above mentioned improved characteristics of increased stability in mammalian serum and lowered clearance with low binding to human serum proteins.

In one aspect the invention features compounds of the chemical formula: 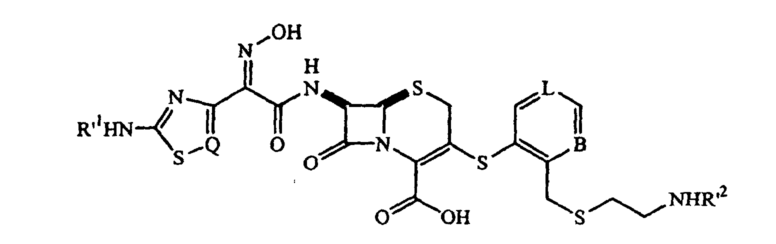 or a pharmaceutically acceptable salt thereof, wherein:
R'¹ is selected from the group consisting of hydrogen and -C (O)CH (NH₂)CH₃;
R'² is hydrogen,
B or L is nitrogen and the other is carbon; and,
Q is selected from the group consisting of nitrogen and -CX,
wherein X is selected from the group consisting of hydrogen and chlorine.

In these and other preferred embodiments, the invention feature compounds of the above chemical formula, wherein the compound is selected from the group consisting of
Cmpd 1. (7*R*)-7[(Z)-2-(2-amino-5-chlorothiazol-4-yl)-2-(hydroxyimino)acetamido]-3-[2-(2-aminoethylthiomethyl)pyrid-3-ylthio]-3-cephem-4-carboxylic acid,
Cmpd 2. (7*R*)-7[(Z)-2-(5-amino-1,2,4-thiadiazol-3-yl)-2-(hydroxyimino)acetamido]-3-[2-(2-aminoethylthiomethyl) pyrid-3-ylthio]-3-cephem-4-carboxylic acid,
Cmpd 3. (7*R*)-7[(Z)-2-(2-amino-5-chlorothiazol-4-yl)-2-(hydroxyimino)acetamido]-3-[4-(2-aminoethylthiomethyl)pyrid-3-ylthio]-3-cephem-4-carboxylic acid,
Cmpd 4. (7R)-7-[(Z)-2-(5-amino-1,2,4-thiadiazol-3-yl)-2-(hydroxyimino)acetamido]-3-[4-(2-aminoethylthiomethyl) pyrid-3-ylthio]-3-cephem-4-carboxylic acid.
Cmpd 5. (7R)-7-[(Z)-2-(2-amino-5-chlorothiazol-4-yl)-2-(hydroxylamino)acetamido]-3-[2-(2-aminoethylthiomethyl) pyrid-3-ylthio]-3-cephem-4-carboxylic acid,
Cmpd 6. (7R)-7-[(Z)-2-(5-amino-1,2,4-thiadiazol-3-yl)-2-(hydroxylamino)acetamido]-3-[2-(2-aminoethylthiomethyl) pyrid-3-ylthio]-3-cephem-4-carboxylic acid,
Cmpd 7. (7R)-7-[(Z)-2-(2-amino-5-chlorothiazol-4-yl)-2-(hydroxylamino)acetamido]-3-[4-(2-aminoethylthiomethyl) pyrid-3-ylthio]-3-cephem-4-carboxylic acid,
Cmpd 8. (7R)-7-[(Z)-2-(5-amino-1,2,4-thiadiazol-3-yl)-2-(hydroxylamino)acetamido]-3-[9-(2-aminoethylthiomethyl) pyrid-3-ylthio]-3-cephem-4-carboxylic acid,

In another preferred embodiment the invention features a prodrug compound having the chemical formula: 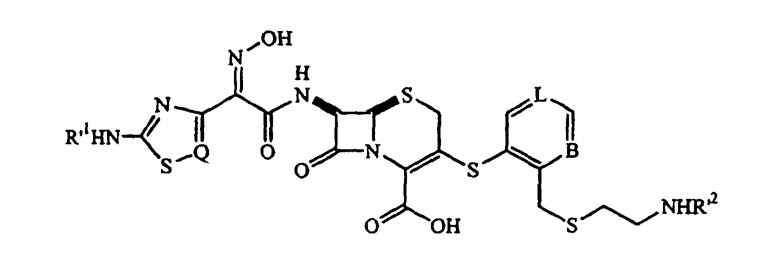 or a pharmaceutically acceptable salt thereof, wherein: R'¹ is selected from the group consisting of hydrogen and -C (O)CH(NH₂)CH₃;
R'² is an acyl group that is cleaved by an enzyme found in mammals and is selected from the group consisting of -C(O)-R⁸⁸, -C (O)-OR⁸⁹, -C (O)-CH(NHR'³)-alk₄, and 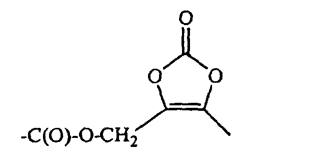 wherein: 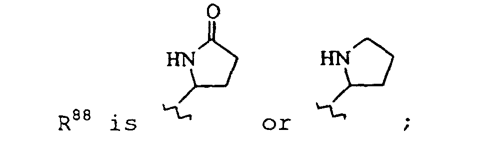 R'³ is selected from the group consisting of hydrogen, alkyl, -C(O)-OR⁸⁹, and -C(O)-CH(NH₂)-alk₄;
alk₄ is selected from the group consisting of hydrogen, and optionally substituted alkyl, wherein said alkyl is optionally substituted with one or more substituents selected from the group consisting of hydrogen, phenyl, -COOH, -C(O)-OR⁸⁹, -C(O)NH₂, -OH, -SH, -NH₂, and 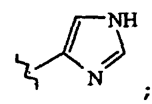 and,
R⁸⁹ is selected from the group consisting of benzhydryl, *t*-butyl, allyl, *p*-nitrobenzyl, benzyl, *p-* or *o*-nitrobenzyl, 2,2,2-trichloroethyl, allyl, cinnamyl, benzhydryl, 2-chloroallyl, *t*-amyl, trityl, 4-methoxytrityl, 4,4'-dimethoxytrityl, trimethylsilyl, *t*-butyldimethylsilyl, β-(trimethylsilyl)ethyl, 4- or 2-methoxybenzyl, 2,4-dimethoxybenzyl, 3,4-dimethoxybenzyl, 2,4,6-trimethoxybenzyl, methoxymethyl, and 3,3-dimethylallyl.
B or L is nitrogen and the other is carbon; and,
Q is selected from the group consisting of nitrogen and -CX, wherein X is selected from the group consisting of hydrogen and chlorine.

In a further preferred embodiment of the invention the prodrug compound of the above formula is a compound, wherein R'³ is selected from the group consisting of hydrogen, methyl, and -C(O)-CH(NH₂)CH₃.

In another preferred embodiment, alk₄ is selected from the group consisting of hydrogen, -CH₃, -CH(CH₃)₂, -CH₂OH, -CH₂NH₂, -CH₂CH₂NH₂, -CH₂CH₂CH₂NH₂, -CH₂CH₂CH₂CH₂NH₂, -CH₂COOH, -CH₂CH₂COOH, -CH₂-C(O)NH₂, -CH₂CH₂-C(O)NH₂, and 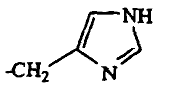 in the compounds of the above mentioned formulas.

In more preferred embodiments, the invention features a prodrug compound of the above formula, wherein the compound is selected from the group consisting of
Cmpd 2. (7*R*)-7[(Z)-2-(5-amino-1,2,4-thiadiazol-3-yl)-2-(hydroxyimino)acetamido)-3-[2-(2-aminoethylthiomethyl) pyrid-3-ylthio]-3-cephem-4-carboxylic acid,
Cmpd 2-A. (7R)-7-[(Z)-2-(5-amino-1,2,4-thiadiazol-3-yl)-2-(hydroxyimino)acetamido]-3-{2-[2-N-(L)-ornithylaminoethylthiomethyl]pyrid-3-ylthio}-3-cephem-4-carboxylic acid,
Cmpd 2-B. (7R)-7-[(Z)-2-(5-amino-1,2,4-thiadiazol-3-yl)-2-(hydroxyimino)acetamido]-3-{2-[2-N-(L)-prolylaminoethylthiomethyl]pyrid-3-ylthio)-3-cephem-4-carboxylic acid,
Cmpd 2-C. (7R)-7-[(Z)-2-(5-N-(L)-alanylamino-1,2,4-thiadiazol-3-yl)-2-(hydroxyimino)-acetamido]-3-{2-[2-aminoethylthiomethyl]pyrid-3-ylthio}-3-cephem-4-carboxylic acid,
Cmpd 2-D. (7R)-7-[(Z)-2-(5-amino-1,2,4-thiadiazol-3-yl)-2-(hydroxyimino)acetamido]-3-{2-[2-N-(L,L)-alanylalanylaminoethylthiomethyl]pyrid-3-ylthio}-3-cephem-4-carboxylic acid,
Cmpd 2-E. (7R)-7-[(Z)-2-(5-amino-1,2,4-thiadiazol-3-yl)-2-(hydroxyimino)acetamido]-3-{2-[2-N-glycylaminoethylthiomethyl]pyrid-3-ylthio}-3-cephem-4-carboxylic acid,
Cmpd 2-F. (7R)-7-[(Z)-2-(5-amino-1,2,4-thiadiazol-3-yl)-2-(hydroxyimino)acetamido]-3-{2-[2-N-(L)-aspartylaminoethylthiomethyl]pyrid-3-ylthio}-3-cephem-4-carboxylic acid,
Cmpd 2-G. (7R)-7-[(Z)-2-(5-amino-1,2,4-thiadiazol-3-yl)-2-(hydroxyimino)acetamido]-3-{2-[2-N-(L)-alanylaminoethylthiomethyl]pyrid-3-ylthio}-3-cephem-4-carboxylic acid,
Cmpd 2-H. (7R)-7-[(Z)-2-(5-amino-1,2,4-thiadiazol-3-yl)-2-(hydroxyimino)acetamido]-3-{2-[2-N-(L)-(N_{α}-methyl)alanylaminoethylthiomethyl]pyrid-3-ylthio}-3-cephem-4-carboxylic acid,
Cmpd 2-I. (7R)-7-[(Z)-2-(5-amino-1,2,4-thiadiazol-3-yl)-2-(hydroxyimino)acetamido]-3-{2-[2-N-(L)-histidylaminoethylthiomethyl]pyrid-3-ylthio}-3-cephem-4-carboxylic acid,
Cmpd 2-J. (7R)-7-[(Z)-2-(5-amino-1,2,4-thiadiazol-3-yl)-2-(hydroxyimino)acetamido]-3-{2-[2-N-(L)-valylaminoethylthiomethyl]pyrid-3-ylthio}-3-cephem-4-carboxylic acid,
Cmpd 2-K. (7R)-7-[(Z)-2-(5-amino-1,2,4-thiadiazol-3-yl)-2-(hydroxyimino)acetamido]-3-{2-[2-N-(L)-asparagylaminoethylthiomethyl]pyrid-3-ylthio}-3-cephem-4-carboxylic acid,
Cmpd 2-L. (7R)-7-[(Z)-2-(5-amino-1,2,4-thiadiazol-3-yl)-2-(hydroxyimino)acetamido]-3-{2-[2-N-(L)-lysylaminoethylthiomethyl]pyrid-3-ylthio}-3-cephem-4-carboxylic acid,
Cmpd 2-M. (7R)-7-[(Z)-2-(5-amino-1,2,4-thiadiazol-3-yl)-2-(hydroxyimino)acetamido]-3-{2-[2-N-(L)-serylaminoethylthiomethyl]pyrid-3-ylthio}-3-cephem-4-carboxylic acid,
Cmpd 2-N. (7R)-7-[(Z)-2-(5-amino-1,2,4-thiadiazol-3-yl)-2-(hydroxyimino)acetamido]-3-{2-[2-N-(L)-glutaminylaminoethylthiomethyl]pyrid-3-ylthio}-3-cephem-4-carboxylic acid,
Cmpd 2-O. (7R)-7-[(Z)-2-(5-amino-1,2,4-thiadiazol-3-yl)-2-(hydroxyimino)acetamido]-3-{2-[2-(5-methyl-1,3-dioxolan-4-en-2-on-4-yl)methoxycarbonyl)aminoethylthiomethyl]pyrid-3-ylthio}-3-cephem-4-carboxylic acid,
Cmpd 2-P. (7R)-7-[(Z)-2-(5-amino-1,2,4-thiadiazol-3-yl)-2-(hydroxyimino)acetamido]-3-{2-(2-N-(L)-pyroglutamylaminoethylthiomethyl]pyrid-3-ylthio}-3-cephem-4-carboxylic acid,
Cmpd 2-Q. (7R)-7-[(Z)-2-(5-amino-1,2,4-thiadiazol-3-yl)-2-(hydroxylamino)acetamido]-3-[2-(2-guanidinoethylthiomethyl) pyrid-3-ylthio]-3-cephem-4-carboxylic acid,
Cmpd 2-R. (7*R*)-7[(Z)-2-(5-amino-1,2,4-thiadiazol-3-yl)-2-(hydroxyimino)acetamido]-3-[2-(2-guanidinoethylthiomethyl) pyrid-3-ylthio]-3-cephem-4-carboxylic acid,
Cmpd 2-S. (7R)-7-[(Z)-2-(2-amino-5-chlorothiazol-4-yl)-2-(hydroxyimino)acetamido]-3-[2-(2-guanidinoethylthiomethyl) pyrid-3-ylthio]-3-cephem-4-carboxylic acid, aspartylamidoethylthiomethyl]pyrid-3-ylthio}-3-cephem-4-carboxylic acid.

In another aspect, the invention relates to a compound having the chemical formula: 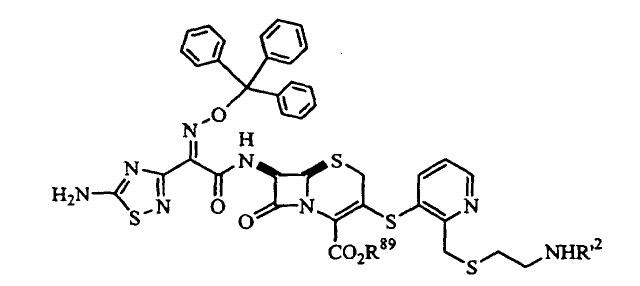 wherein R'² is hydrogen
R⁸⁹ is selected from the group consisting of benzhydryl, *t*-butyl, allyl, *p*-nitrobenzyl, benzyl, *p-* or *o*-nitrobenzyl, 2,2,2-trichloroethyl, allyl, cinnamyl, benzhydryl, 2-chloroallyl, *t*-amyl, trityl, 4-methoxytrityl, 4,4'-dimethoxytrityl, trimethylsilyl, *t*-butyldimethylsilyl, β-(trimethylsilyl)ethyl, 4- or 2-methoxybenzyl, 2,4-dimethoxybenzyl, 3,4-dimethoxybenzyl, 2,4,6-trimethoxybenzyl, methoxymethyl, and 3,3-dimethylallyl.

In a further aspect, the invention relates to a prodrug compound having the chemical formula: 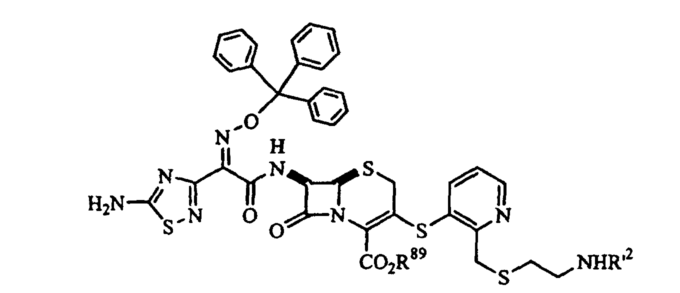 wherein R'² is selected from the group consisting of -C(O)-R⁸⁸, -C(O)-OR⁸⁹, -C(O)-CH(NHR'³)-alk₄, and 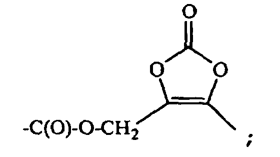 wherein,
R⁸⁸ is 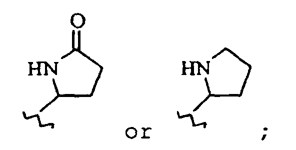 R'³ is selected from the group consisting of hydrogen, -C(O)-OR⁸⁹, and -C(O)-CH(NH₂)-alk₄;
alk₄ is selected from the group consisting of hydrogen, and optionally substituted alkyl, wherein said alkyl is optionally substituted with one or more substituents selected from the group consisting of hydrogen, phenyl, -COOH, -C(O)-OR⁸⁹, -C(O)NH₂, -OH, -SH, -NH₂, and 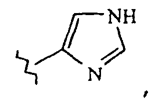 and,
R⁸⁹ is selected from the group consisting of benzhydryl, *t*-butyl, allyl, *p*-nitrobenzyl, benzyl, *p*- or *o*-nitrobenzyl, 2,2,2-trichloroethyl, allyl, cinnamyl, benzhydryl, 2-chloroallyl, *t*-amyl, trityl, 4-methoxytrityl, 4,4'-dimethoxytrityl, trimethylsilyl, t-butyldimethylsilyl, β-(trimethylsilyl)ethyl, 4- or 2-methoxybenzyl, 2,4-dimethoxybenzyl, 3,4-dimethoxybenzyl, 2,4,6-trimethoxybenzyl, methoxymethyl, and 3,3-dimethylallyl.

It is understood that the above-named compounds may be synthesized, purified, and used in their neutral form, as the above names suggest, or as pharmaceutically acceptable salts. The pharmaceutically acceptable salt comprises the above compounds in their charged form, either as cations or anions, along with a counterion. Preferred pharmaceutically acceptable salts include (1) inorganic salts such as sodium, potassium, chloride, bromide, iodide, nitrate, phosphate or sulfate; (2) carboxylate salts such as acetate, propionate, butyrate, maleate, or fumarate; (3) alkylsulfonates such as methanesulfonate, ethanesulfonate, 2-hydroxyethylsulfonate, n-propylsulfonate or isopropylsulfonate; and (4) hydroxycarboxylates such as lactate, malate, and citrate. Generally, pharmaceutically acceptable salts may be obtained by reacting any one of the compounds of the invention with an organic or inorganic acid, such as hydrochloric acid, hydrobromic acid, sulfuric acid, nitric acid, phosphoric acid, methanesulfonic acid, ethanesulfonic acid, p-toluenesulfonic acid, salicylic acid, and the like. Pharmaceutically acceptable salts may also be obtained by reacting any one of the compounds of the invention with an organic or inorganic base, such as benzathene, chloroprocaine, choline, diethanolamine, ethylenediamine, meglumine, procain, and the hydroxide, alkoxide, carbonate, bicarbonate, sulfate, bisulfate, amide, alkylamide, or dialkylamide salts of the following metal cations: lithium, sodium, potassium, magnesium, calcium, aluminum, and zinc.

As explained herein, the compounds of the invention have biological activity. The invention features a compound of the chemical formulas as described above which is active against methicillin-resistant Staphylococci, as demonstrated by a lower minimum inhibitory concentration than methicillin, where the bacteria are selected from the group consisting of *S. aureus* Col (Meth^{R})(lac-), *S. aureus* 76 (Meth^{R})(Iac+), *S. aureus* ATCC 33593 (Meth^{R}), *S. aureus* Spain #356 (Meth^{R}), and *S. haemolyticus* 05 (Meth^{R}).

In another embodiment, the present invention provides for compositions comprising an amount of a compound of formula I or II effective to treat bacterial infections in mammals arising from bacteria resistant to β-lactam antibiotics.

In still another embodiment, the present invention includes methods for treating a bacterial infection in a mammal arising from bacteria resistant to β-lactam antibiotics, or a mammal suffering from a methicillin-resistant Staphylococcal bacterial infection, comprising administering to such mammal a therapeutically effective amount of a compound of the chemical formulas as described above. Of course, the compounds of the present invention also have utility in compositions and methods to treat mammals infected with bacteria that are sensitive to conventional β-lactam antibiotics. Thus, the invention also features an antibacterial composition for treating a methicillin-resistant Staphylococcal bacterial infection, comprising a therapeutically effective amount of a compound of formula I or II in a pharmaceutically acceptable carrier or diluent.

### DETAILED DESCRIPTION OF THE PREFERRED EMBODIMENTS

### I. Definitions

As used herein, the term "alkyl" denotes a branched, unbranched, or cyclic hydrocarbon group, preferably containing between one and six, more preferably one and four, carbon atoms, such as methyl, ethyl, *n*-propyl, *iso*-propyl, *n*-butyl, *sec*-butyl, *iso*-butyl, *tert*-butyl, and 2-methylpentyl. These groups may be optionally substituted with one or more functional groups which are attached commonly to such chains, such as hydroxyl, bromo, fluoro, chloro, iodo, mercapto, cyano, alkylthio, heterocycle, aryl, heteroaryl, carboxyl, alkoxycarbonyl, alkyl, alkenyl, nitro, amino, alkoxyl, amido, and optionally substituted isothioureido, amidino, guanidino, and the like to form alkyl groups such as trifluoromethyl, 3-hydroxyhexyl, 2-carboxypropyl, 2-fluoroethyl, carboxymethyl, 4-cyanobutyl, 2-guanidinoethyl, 3-N,N'-dimethylisothiouroniumpropyl, and the like.

The term "acyl" denotes groups -C(O)R, where R is hydrogen or alkyl as defined above, such as formyl, acetyl, propionyl, or butyryl.

The term "β-lactam resistant bacteria" refers to bacteria against which a β-lactam antibiotic has a minimum inhibitory concentration (MIC) of greater than 32 mg/mL.

The term "methicillin-resistant bacteria" refers to bacteria that are resistant to methicillin. Examples of such bacteria arc provided in Table 1 and are identified Meth^{R}. The term "methicillin sensitive bacteria" refers to bacteria that are sensitive to methicillin. Examples of such bacteria are provided in Table 1 and are identified Meth^{S}.

A "prodrug" refers to an agent that is converted into the parent drug *in vivo*. Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. An example, without limitation, of a prodrug would be a compound of the present invention which is administered as an ester (the "prodrug") to facilitate transmittal across a cell membrane where water solubility is detrimental to mobility but which then is metabolically hydrolyzed to the carboxylic acid, the active entity, once inside the cell where water solubility is beneficial. A further example of a prodrug might be a short peptide (polyaminoacid) bonded to an acid group where the peptide is metabolized to reveal the active moiety.

### II. Compounds of the Invention

The present invention provides compounds, methods and compositions effective to treat bacterial infections, and, especially, infections arising from bacteria which have developed resistance to conventional β-lactam antibiotics. The present invention alsoprovides compounds, methods and compositions effective to treat bacterial infections arising from bacteria which have developed resistance to conventional cephalosporin antibiotics.

It is understood to those skilled in the art that the compounds of the present invention can be prepared or be present as their pharmaceutically acceptable salts or as salts that may not be pharmaceutically acceptable. Such salts are within the scope and contemplation of the claims of the present invention. Such salts can exist as the combination of the compounds of the present invention and acids or bases. These acids may include trifluoroacetic acid, hydrochloric acid, methanesulfonic acid, and other organic or inorganic acids. The bases may include benzathene, chloroprocaine, choline, diethanolamine, ethylenediamine, meglumine, procain, and the hydroxide, alkoxide, carbonate, bicarbonate, sulfate, bisulfate, amide, alkylamide, or dialkylamide salts of the following metal cations: lithium, sodium, potasium, magnesium, calcium, aluminum, and zinc and other organic or inorganic bases. These salts may exist as a combination of one or more equivalents of acid or base per compound or one or more equivalents of compound per acid or base.

The names and structures of some of the compounds of the invention are shown below.

### Cmpd 1 (7R)-7-[(Z)-2-(2-amino-5-chlorothiazol-4-yl)-2-(hydroxyimino)acetamido]-3-[2-(2-aminoethylthiomethyl)pyrid-3-ylthio]-3-cephem-4-carboxylic acid

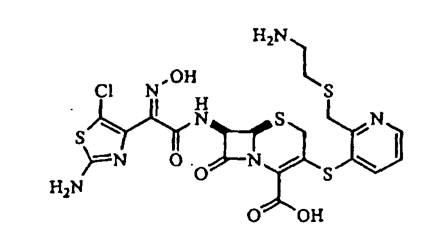

### Cmpd 2-R. (7R)-7-[(Z)-2-(5-amino-1,2,4-thiadiazol-3-yl)-2-(hydroxyimino)acetamido]-3-[2-(2-guanidinoethylthiomethyl)pyrid-3-ylthio]-3-cephem-4-carboxylic acid

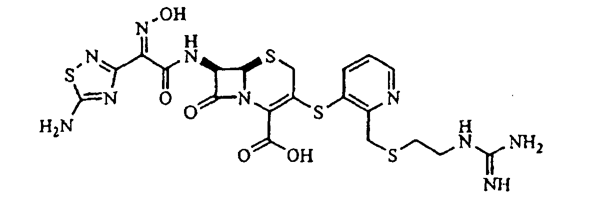

### Cmpd 2-5.. (7R)-7-[(Z)-2-(2-amino-5-chlorothiazol-4-yl)-2-(hydroxyimino)acetamido]-3-[2-(2-guanidinoethylthiomethyl)pyrid-3-ylthio]-3-cephem-4-carboxylic acid

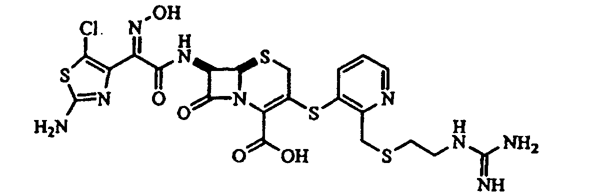

### Cmpd 2 (7R)-7-[(Z)-2-(5-amino-1,2,4-thiadiazol-3-yl)-2-(hydroxyimino)acetamido]-3-[2-(2-aminoethylthiomethyl)pyrid-3-ylthio]-3-cephem-4-carboxylic acid

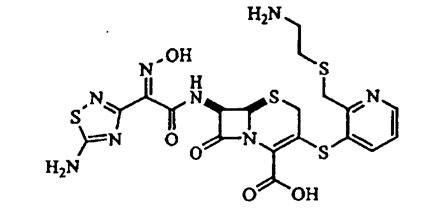

### Cmpd 3 (7R)-7-[(Z)-2-(2-amino-5-chlorothiazol-4-yl)-2-(hydroxyimino)acetamido]-3-[4-(2-aminoethylthiomethyl)pyrid-3-ylthio]-3-cephem-4-carboxylic acid

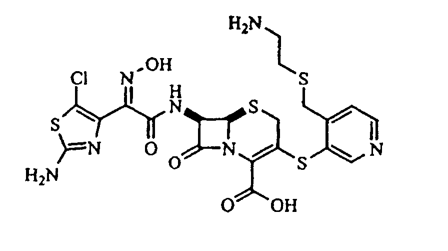

### Cmpd 4 (7R)-7-[(Z)-2-(5-amino-1,2,4-thiadiazol-3-yl)-2-(hydroxyimino)acetamido]-3-[4-(2-aminoethylthiomethyl)pyrid-3-ylthio]-3-cephem-4-carboxylic acid

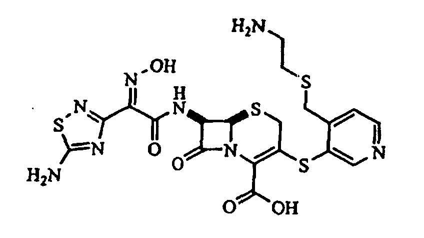

### A. Synthesis of Compounds of Formula I and II

The compounds of the present invention may be readily prepared in accordance with the following schemes. However, it will be appreciated that other synthetic pathways for forming the compounds of the invention are available and that the following is offered merely by way of example, and not limitation. It will be further recognized that various protecting and deprotecting strategies will be employed which are standard in the art *(see, e.g.,* Greene and Wuts, Protective Groups in Organic Synthesis, 2^{nd} Ed., John Wiley & Sons, New York, NY, 1991). Those of skill in the art will recognize that the selection of any particular protecting group (*e.g.*, a carboxyl protecting group) will depend on the stability of the protected moiety with respect to subsequent reaction conditions.

Generally, the synthesis of the cephalosporins of the present invention may be achieved using well-known methods and readily available materials (*see, e*.*g*., March; Larock, COMPREHENSIVE ORGANIC TRANSFORMATIONS (VCH Publishers, 1989); and G.I. Georg, THE ORGANIC CHEMISTRY OF β-LACTAMS, (VCH 1992), each of which is incorporated herein by reference).

Cephalosporin intermediates bearing an appropriate acylamino substituent R¹ and carboxyl protecting group R² and leaving group R"¹ can be reacted with a heterocyclic thiol, displacing R"¹.

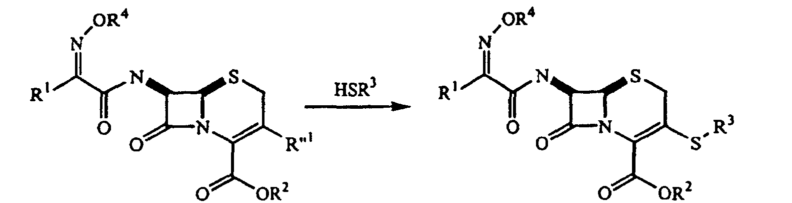

In the above structures, R"¹ is a leaving group, which may be selected from the group consisting of *p*-toluenesulfonate, methylsulfonate, fluorosulfonate, chloro, bromo, and (R"²O)₂PO-, where R"² is selected from the group consisting of hydrogen and alkyl, as defined herein; and

R² is a carboxyl protecting group, which may be selected from the group consisting of *p*-methoxybenzyl, benzhydryl, *t*-butyl, allyl, and *p*-nitrobenzyl. Those skilled in the art realize that other suitable leaving groups or carboxyl protecting groups may be used in place of those mentioned here for R"¹ and R², respectively. For example, the carboxyl protecting group R² may be those protecting groups amenable to reductive cleavage, such as benzyl,*p*- or *o*-nitrobenzyl, 2,2,2-trichloroethyl, allyl, cinnamyl, benzhydryl, 2-chloroallyl and the like. Alternatively, R² may be a protecting group amenable to acidic cleavage, such as *t*-butyl, *t*-amyl, trityl, 4-methoxytrityl, 4,4'-dimethoxytrityl, trimethylsilyl, *t*-butyldimethylsilyl, phenacyl, β-(trimethylsilyl)ethyl, benzyl, 4- or 2-methoxybenzyl, 2,4-dimethoxybenzyl, 3,4-dimethoxybenzyl, 2,4,6-trimethoxybenzyl, methoxymethyl, benzhydryl, or 3,3-dimethylallyl. Preferred protecting groups are *t*-butyl, *p*-methoxybenzyl, *p*-nitrobenzyl, allyl and benzhydryl. Such groups may be attached to the unprotected carboxyl group of the cephalosporin starting material using known reagents and techniques, such as those described in Greene and Wuts.

The above reaction may be carried out at room temperature, at temperatures higher than room temperature, or at temperatures lower than room temperature. The reaction is preferably carried out in the range of about -78 °C to about 50 °C, more preferably in the range of about -10 °C to about 40 °C, and most preferably in the range of about 0 °C to room temperature. "Room temperature" is generally in the range of about 20 °C to about 25 °C. By "about" a certain temperature it is meant that the temperature range is preferably within 10 °C of the listed temperature, more preferably within 5 °C of the listed temperature, and most preferably within 2 °C of the listed temperature. Therefore, by way of example, by "about 40 °C" it is meant that the temperature range is preferably 40±10 °C, more preferably 40±5 °C, and most preferably 40±2 °C.

The reaction can also be carried out with or without an external base. If a base is used, the base is preferably a nitrogen base, an organic base, or an inorganic base. "Nitrogen bases" are commonly used in the art and are selected from acyclic and cyclic amines. Examples of nitrogen bases include, but are not limited to, ammonia, methylamine, trimethylamine, triethylamine, aniline, 1,8-diazabicyclo[5.4.0]undec-7-ene, diisopropylethylamine, pyrrolidine, piperidine, and pyridine or substituted pyridine (*e.g.*, 2,6-di-*tert*-butylpyridine). "Organic bases" are bases that contain carbon atoms. Examples of organic bases include, but are not limited to, carbonate, bicarbonate, acetate, and formate anions. "Inorganic bases" are bases that do not contain any carbon atoms. Examples of inorganic bases include, but are not limited to, hydroxide, phosphate, bisulfate, hydrosulfide, and amide anions. Those skilled in the art know which nitrogen base or inorganic base would match the requirements of the reaction conditions. In certain embodiments of the invention, the base used may be pyrrolidine or piperidine. In other embodiments the base may be the hydroxide, carbonate, bicarbonate or anion, preferably used as the sodium or potassium salt.

The solvent in which the reaction is carried out may be a homogeneous solvent system, in which case no phase transfer catalyst is used. In other cases, the solvent may be a heterogeneous solvent system, in which case a phase transfer catalyst is used. By "homogeneous solvent system" it is meant a solvent system which uses one or more solvents that are fully miscible, and therefore, form one phase. The solvents in a homogeneous solvent system are all other hydrophobic or hydrophilic. By "heterogeneous solvent system" it is meant a solvent system which uses two or more solvents that are not fully miscible, and therefore, form more than one phase, usually two phases consisting of an aqueous phase and an organic phase. Some of the solvents in a heterogeneous solvent system are hydrophobic while others are hydrophilic.

If a heterogeneous solvent system is used, then the reaction may be carried out in the presence of a phase transfer catalyst. Those skilled in the art can select a suitable phase transfer catalyst by knowing the reaction conditions or by further experimentation. Common phase transfer catalysts include, but are not limited to, quaternary ammonium salts.

Manipulation of the 7-acyl substituent can be performed after the thio-linked heterocyclic substituent is attached to the cephalosporin:

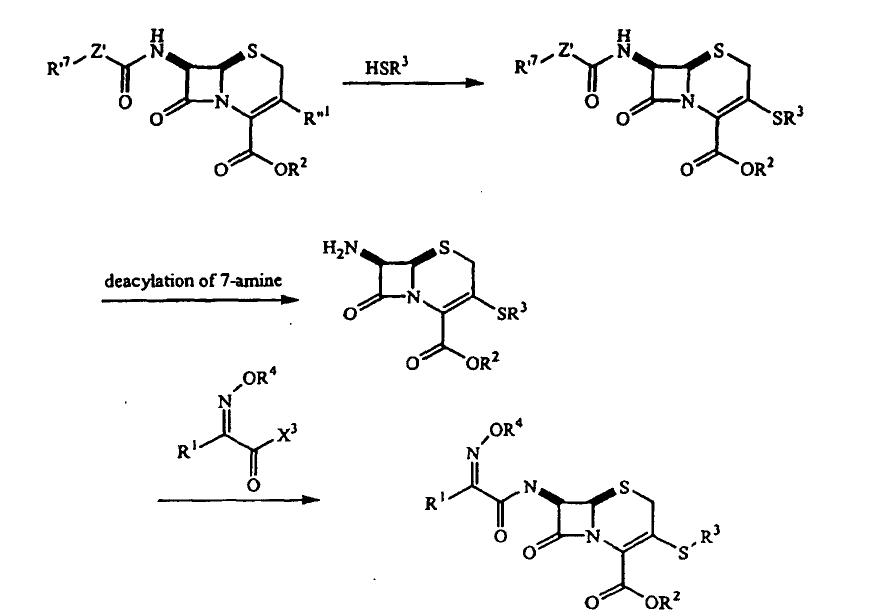

In the above scheme R¹, R², R³, and R⁴ are as defined herein. R'⁷ is preferably selected from the group consisting of alkyl and aryl, as those terms are defined herein. R'⁷ is more preferably selected from the group consisting of phenyl, *tert*-butyl, and benzyl. Z' is preferably selected from the group consisting of methylene (-CH₂-), oxygen, sulfur, and -NH-. More preferably, Z' is selected from the group consisting of methylene and oxygen. X³ in the above scheme is preferably selected from the group consisting of -OP(O)-(O-phenyl)₂, and -OP(O)-Cl₂.

Alternatively, the displacement of the leaving group at the 3-position of cephalosporin can be performed at the stage of the 7-amino intermediate and then followed by acylation of the amine with appropriate acylating reagent: 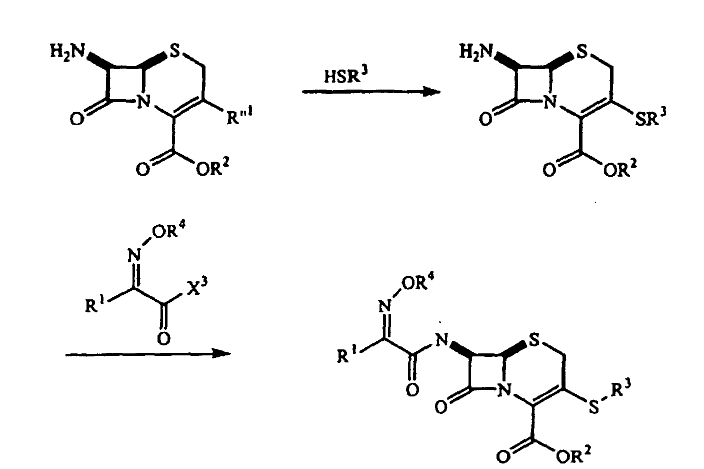 In the above scheme R¹, R², R³, R⁴, and X³ are as defined herein.

Finally, the one-step or multi-step deprotection of the fully assembled cephalosporin, removing protecting group R², and other protecting groups present at substituents R³, Z, and R⁷ using conditions appropriate for the removal of all protecting groups is used for obtaining the biologically active cephalosporin.

The substituent R' in all of the above schemes may be any of the groups described above and are either available commercially (e.g., from Aldrich, Milwaukee, WI) or can be formed using known techniques and starting materials (*see, e.g.,* March; Larock). These groups can be substituted for those present on the starting material by variety of well known techniques (*see, e.g.,* Barrett, J.C.S. Perkin I, 1629 (1979) or Chauvette, J. Org. Chem. **36**:1259 (1971), both of which are incorporated herein by reference), such as by transamination of an existing substituent for the desired substituent, or hydrolytic removal of the existing substituent followed by reaction with a suitably reactive form of desired substituent, such as an acyl chloride. Again, the appropriate reagents and techniques will be apparent to those of skill in the art.

### B. Side Chain Synthesis:

Side chains on C-7 and C-3 of the cephem core are synthesized by the procedures described below. These procedures are modified from the procedures found in chemical literature, in particular, Tatsuda, K. et al., *Bull. Chem. Soc. Jpn.,* **1994,** *67*, 1701-1707; Csendes, B. et al., *Journal of Antibiotics*, **1983**, *36,* 1020; Memoli, K.A., *Tetrahedron Lett,* **1996**., 37, 3617; and Bjoork, P., et al., *J. Heterocycl. Chem,* **1995.** *32 (3)*, 751.

The following diagrams depict the synthetic schemes for the side chains.

### a. C-7 Side Chain Synthesis:

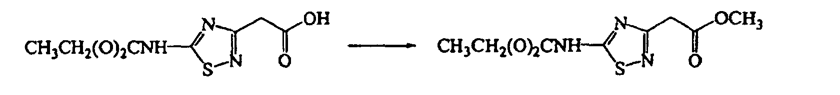

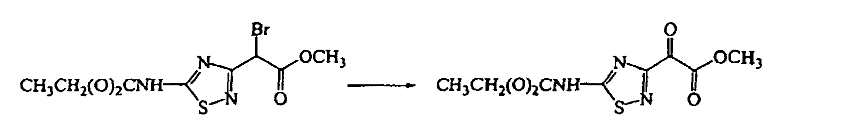

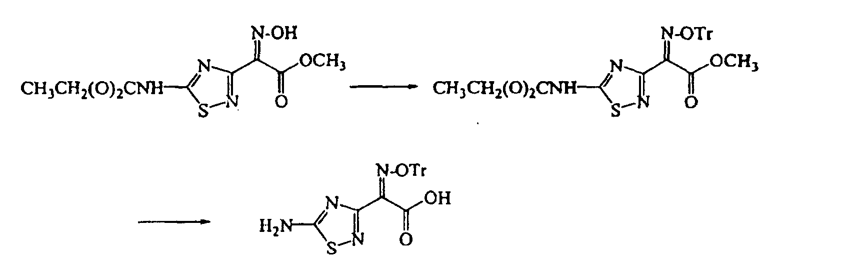

### b. C-3 Side Chain Synthesis (Memoli, et al., route):

As shown below, 3-mercapto-2-hydroxymethylpyridine is synthesized using the Memoli, et al., route.

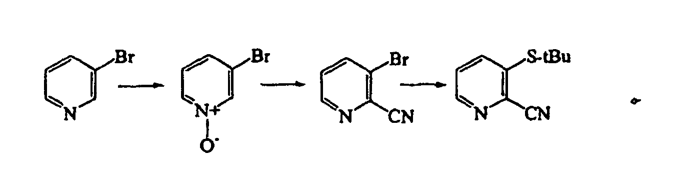

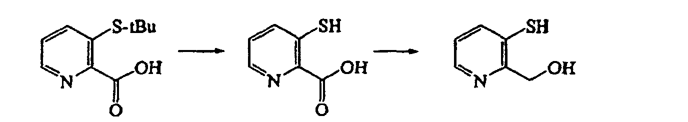

The product of the above synthesis can be used to synthesize the C-3 side chains for the compounds of the present invention, using the synthetic schemes shown below.

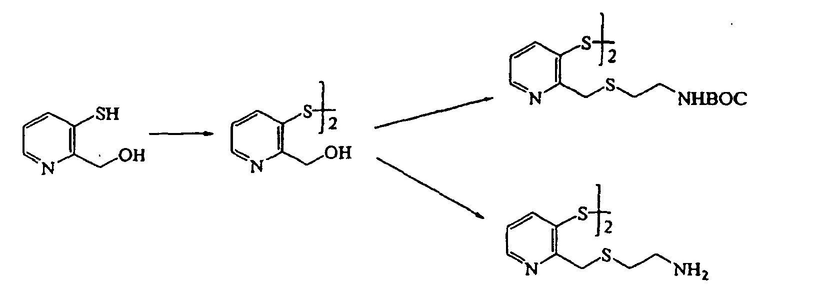

Or the following alternative pathway.

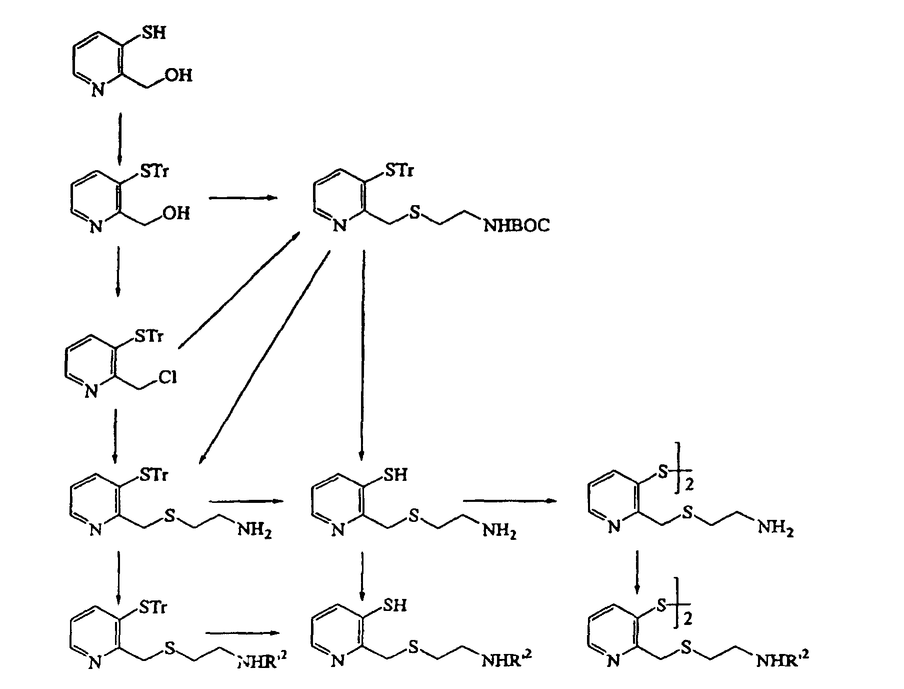

### C. Other Synthetic Schemes

Some of the cephalosporin compounds of the invention can be synthesized using the scheme shown below.

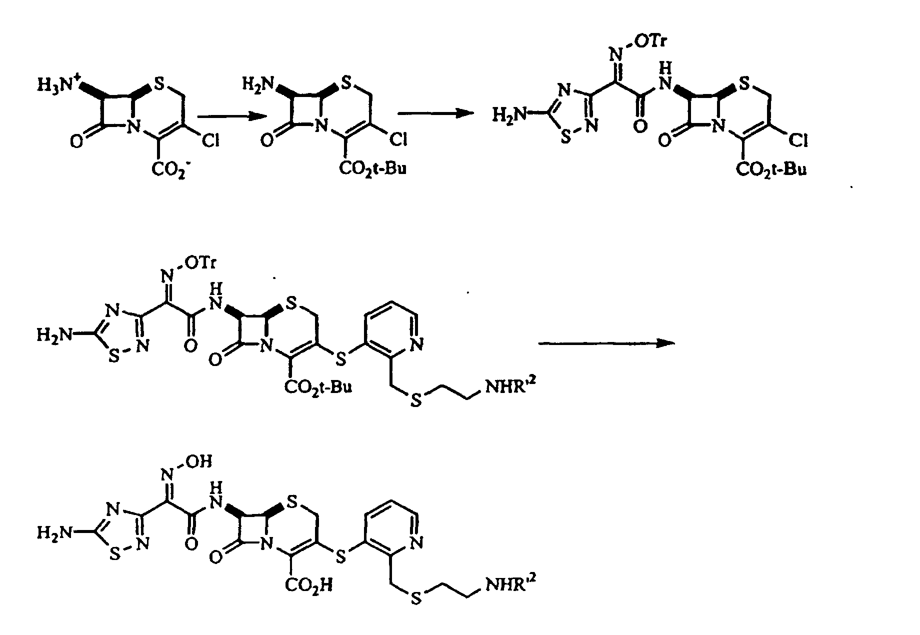

### III. Pharmaceutical Applications and Preparations

According to this invention, a therapeutically or pharmaceutically effective amount of a cephalosporin and particularly, a compound of Formula I, II or III, is administered to a mammal suffering from a methicillin-resistant bacterial infection (or other β-lactam resistant bacterial infections, such as vancomycin-resistant or ampicillin-resistant infections), especially resistant *S*. *aureus*, in an amount effective to at least partially relieve the infection. Especially important are infections resulting from strains having similar activity to strains such as *S. aureus* Col (Meth^{R})(lac⁻), *S. aureus* 76 (Meth^{R}) (lac⁺), *E. foecium* ATCC 35667, or *E. foecalis* ATCC 29212. Again, such compounds are also effective against bacteria sensitive to methicillin, vancomycin, and/or ampicillin and therefore have utility in such compositions and methods.

The compositions containing the compound(s) of the invention can be administered for prophylactic and/or therapeutic treatments. In therapeutic applications, the compositions are administered to a patient already suffering from an infection, as described above, in an amount sufficient to cure or at least partially arrest the symptoms of the infection. An amount adequate to accomplish this is defined as "therapeutically effective amount or dose." Amounts effective for this use will depend on the severity and course of the infection, previous therapy, the patient's health status and response to the drugs, and the judgment of the treating physician. In prophylactic applications, compositions containing the compounds of the invention are administered to a patient susceptible to or otherwise at risk of a particular infection. Such an amount is defined to be a "prophylactically effective amount or dose." In this use, the precise amounts again depend on the patient's state of health, weight, and the like.

Once improvement of the patient's conditions has occurred, a maintenance dose is administered if necessary. Subsequently, the dosage or the frequency of administration, or both, can be reduced, as a function of the symptoms, to a level at which the improved condition is retained. When the symptoms have been alleviated to the desired level, treatment can cease. Patients can, however, require intermittent treatment on a long-term basis upon any recurrence of the disease symptoms.

In general, a suitable effective dose of the compound of the invention will be in the range of 0.1 to 1000 milligram (mg) per recipient per day, preferably in the range of I to 100 mg per day. The desired dosage is preferably presented in one, two, three, four or more subdoses administered at appropriate intervals throughout the day. These subdoses can be administered as unit dosage forms, for example, containing 5 to 1000 mg, preferably 10 to 100 mg of active ingredient per unit dosage form. Preferably, the compounds of the invention will be administered in amounts of between about 2.0 mg/kg to 250 mg/kg of patient body weight, between about one to four times per day.

While it is possible to administer the active ingredient of this invention alone, it is preferable to present it as part of a pharmaceutical formulation. The formulations of the present invention comprise at least one compound or inhibitor of this invention in a therapeutically or pharmaceutically effective dose together with one or more pharmaceutically or therapeutically acceptable carriers. Solid carriers include, e.g., starch, lactose, dicalcium phosphate, microcrystalline cellulose, sucrose, and kaolin, and optionally other therapeutic ingredients. Liquid carriers include, e.g., sterile water, polyethylene glycols, non-ionic surfactants, and edible oils such as corn, peanut and sesame oils. In addition, various adjuvants such as are commonly used in the art may be included. For example: flavoring agents, coloring agents, preservatives, and antioxidants, e.g., vitamin E, ascorbic acid, BHT and BHA. Various other considerations are described, e.g., in Gilman et al. (eds) (1990) Goodman and Gilman's: The Pharmacological Basis of Therapeutics, 8th Ed., Pergamon Press; and Remington's *supra*. Methods for administration are discussed therein, e.g., for oral, intravenous, intraperitoneal, or intramuscular administration, and others. Pharmaceutically acceptable carriers will include water, saline, buffers, and other compounds described, e.g., in the MERCK INDEX, Merck & Co., Rahway, NJ. Generally, preferred routes of administration are intravenous and intraperitoneal.

These pharmacological agents can be in a variety of forms. These include, for example, solid, semi-solid and liquid dosage forms, such as tablets, pills, powders, liquid solutions or suspensions, liposomes, injectable and infusible solutions. The preferred form depends on the intended mode of administration and therapeutic application. Generally, a pharmacologically acceptable salt of the compound will be used to simplify preparation of the composition. Preferred salts include sodium, potassium, arginine, glycine, alanine, threonine, and lysine. These are prepared, preferably, in water suitably mixed with a surfactant such as hydroxypropylcellulose.

Depending on the specific conditions being treated, such agents may be formulated and administered systemically or locally. Techniques for formulation and administration may be found in Remington's Pharmaceutical Sciences, 18th ed., Mack Publishing Co., Easton, PA (1990). Suitable routes may include oral, rectal, transdermal, vaginal, transmucosal, or intestinal administration; parenteral delivery, including intramuscular subcutaneous, intramedullary injections, as well an intrathecal, direct intraventricular, intravenous, intraperitoneal, intranasal, or intraocular injections, just to name a few.

For injection, the agents of the invention may be formulated in aqueous solutions, preferably in physiologically compatible buffers such as Hanks' solution, Ringer's solution, or physiological saline buffer. For such transmucosal administration, penetrants appropriate to the barrier to be permeated are used in the formulation. Such penetrants are generally known in the art.

In soft capsules, the active compounds may be dissolved or suspended in suitable liquids, such as fatty oils, liquid paraffin, or liquid polyethylene glycols. In addition, stabilizers may be added.

### IV. Prodrugs

Some of the compounds of the invention can be used as prodrugs. As explained above, a prodrug is an agent that is converted to the parent drug *in vivo*. The prodrugs of the present invention have the unusual and surprising characteristic of being more soluble than the parent compound at or near physiological pH. These prodrugs are converted to the parent compound in the body of the mammal that has received the prodrug. As can be seen from the structures of these prodrugs, a substituent either on C-5 of the thiadiazole group, or on C-2 of the pyridyl group can by cleaved by hydrolysis or enzymatic action in order to afford the parent compound. For instance, the amide group on the C-2 side chain of the pyridyl group of compounds **17-A, 17-B, 17-D - 17-Q** or the amide group on the C-5 side chain of the thiadiazole group of compound **17-C** can be hydrolyzed and form an amine side chain, which is the parent compound **17**.

The names and the structures of some of the prodrugs of the invention are shown below.

### Cmpd 2-A (7R)-7-[(Z)-2-(5-amino-1,2,4-thiadiazol-3-yl)-2-(hydroxyimino)acetamido]-3-{2-[2-N-(L)-ornithylaminoethylthiomethyl]pyrid-3-ylthio}-3-cephem-4-carboxylic acid

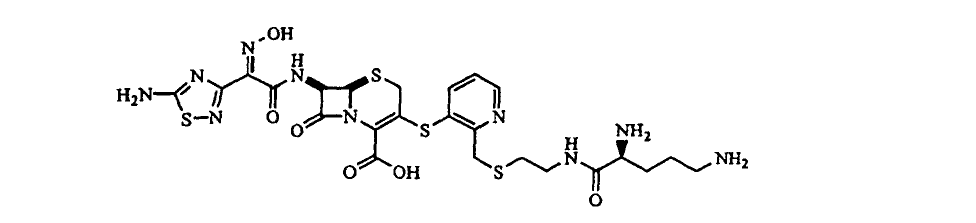

### Cmpd 2-B (7R)-7-[(Z)-2-(5-amino-1,2,4-thiadiazol-3-yl)-2-(hydroxyimino)acetamido]-3-{2-[2-N-(L)-prolylaminoethylthiomethyl]pyrid-3-ylthio)-3-cephem-4-carboxylic acid

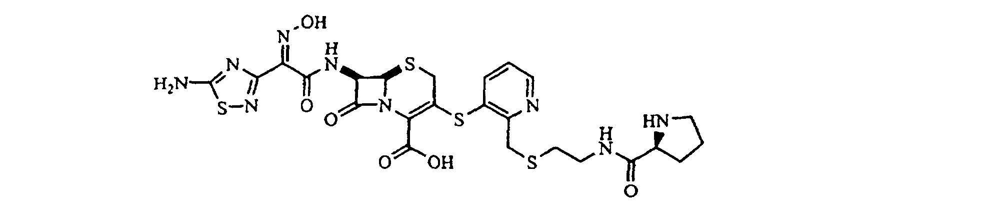

### Cmpd 2-C (7R)-7-[(Z)-2-(5-N-(L)-alanylamino-1,2,4-thiadiazol-3-yl)-2-(hydroxyimino)acetamido]-3-{2-[2-aminoethylthiomethyl]pyrid-3-ylthio}-3-cephem-4-carboxylic acid

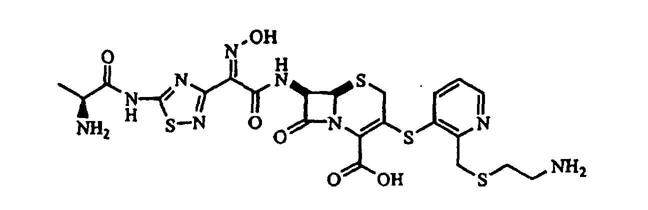

### Cmpd 2-D (7R)-7-[(Z)-2-(5-amino-1,2,4-thiadiazol-3-yl)-2-(hydroxyimino)acetamido]-3-{2-[2-N-(L,L)-alanylalanylaminoethylthiomethyl]pyrid-3-ylthio}-3-cephem-4-carboxylic acid

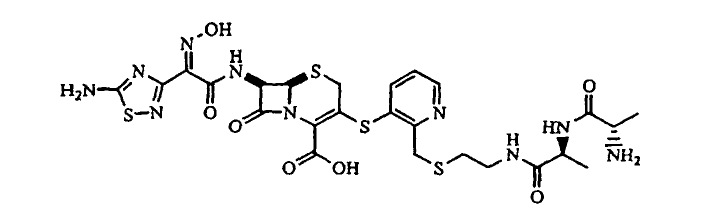

### Cmpd 2-E (7R)-7-[(Z)-2-(5-amino-1,2,4-thiadiazol-3-yl)-2-(hydroxyimino)acetamido]-3-{2-[2-glycylaminoethylthiomethyl]pyrid-3-ylthio}-3-cephem-4-carboxylic acid

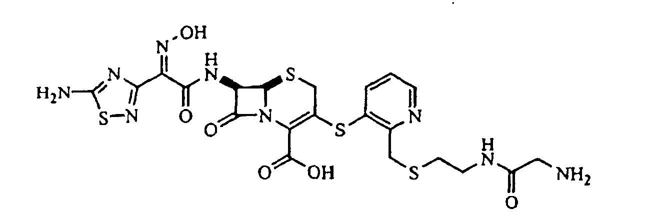

### Cmpd 2-F (7R)-7-[(Z)-2-(5-amino-1,2,4-thiadiazol-3-yl)-2-(hydroxyimino)acetamido]-3-{2-[2-N-(L)-aspartylaminoethylthiomethyl]pyrid-3-ylthio}-3-cephem-4-carboxylic acid

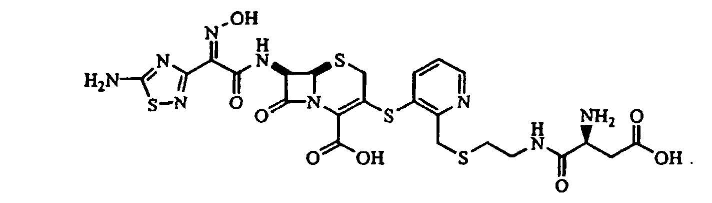

### Cmpd 2-G (7R)-7-[(Z)-2-(5-amino-1,2,4-thiadiazol-3-yl)-2-(hydroxyimino)acetamido]-3-{2-[2-N-(L)-alanylaminoethylthiomethyl]pyrid-3-ylthio}-3-cephem-4-carboxylic acid

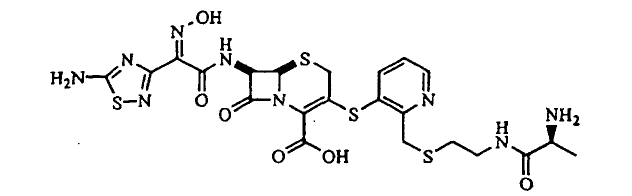

### Cmpd 2-H (7R)-7-[(Z)-2-(5-amino-1,2,4-thiadiazol-3-yl)-2-(hydroxyimino)acetamido]-3-{2-[2-N-(L)-(N_{α}-methyl)alanylaminoethylthiomethyl]pyrid-3-ylthio}-3-cephem-4-carboxylic acid

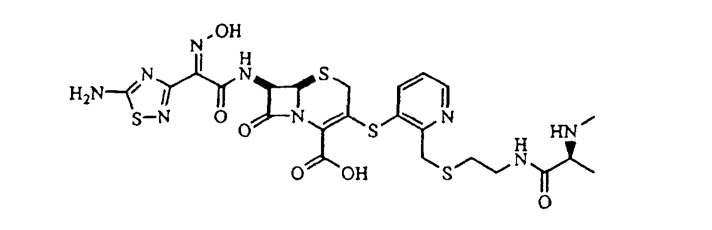

### Cmpd 2-I (7R)-7-[(Z)-2-(5-amino-1,2,4-thiadiazol-3-yl)-2-(hydroxyimino)acetamido]-3-{2-[2-N-(L)-histidylaminoethylthiomethyl]pyrid-3-ylthio}-3-cephem-4-carboxylic acid

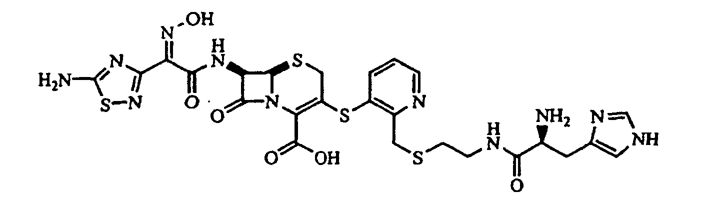

### Cmpd 2-J (7R)-7-[(Z)-2-(5-amino-1,2,4-thiadiazol-3-yl)-2-(hydroxyimino)acetamido]-3-{2-[2-N-(L)-valylaminoethylthiomethyl]pyrid-3-ylthio}-3-cephem-4-carboxylic acid

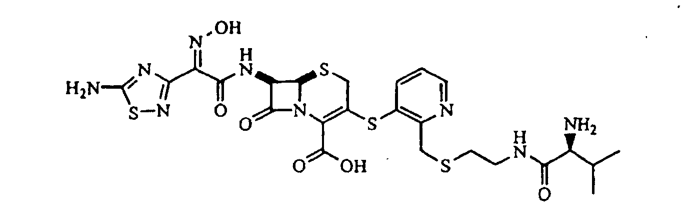

### Cmpd 2-K (7R)-7-[(Z)-2-(5-amino-1,2,4-thiadiazol-3-yl)-2-(hydroxyimino)acetamido]-3-{2-[2-N-(L)-asparagylaminoethylthlomethyl]pyrid-3-ylthio}-3-cephem-4-carboxylic acid

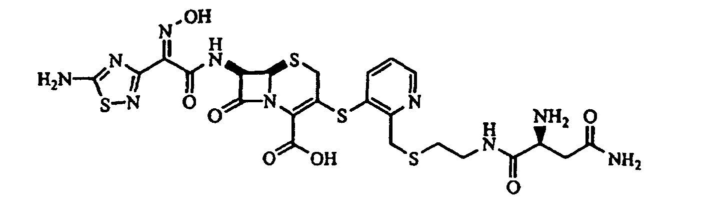

### Cmpd 2-L (7R)-7-[(Z)-2-(5-amino-1,2,4-thiadiazol-3-yl)-2-(hydroxyimino)acetamido]-3-{2-[2-N-(L)-lysylaminoethylthiomethyl]pyrid-3-ylthio}-3-cephem-4-carboxylic acid

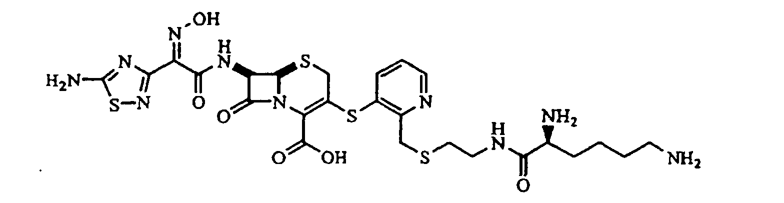

### Cmpd 2-M (7R)-7-[(Z)-2-(5-amino-1,2,4-thiadiazol-3-yl)-2-(hydroxyimino)acetamido]-3-{2-[2-N-(L)-serylaminoethylthiomethyl]pyrid-3-ylthio}-3-cephem-4-carboxylic acid

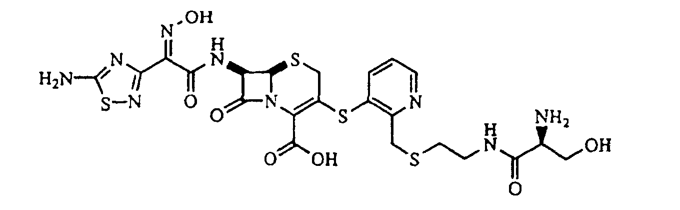

### Cmpd 2-N (7R)-7-[(Z)-2-(5-amino-1,2,4-thiadiazol-3-yl)-2-(hydroxyimino)acetamido]-3-{2-[2-N-(L)-glutaminylaminoethylthiomethyl]pyrid-3-ylthio}-3-cephem-4-carboxylic acid

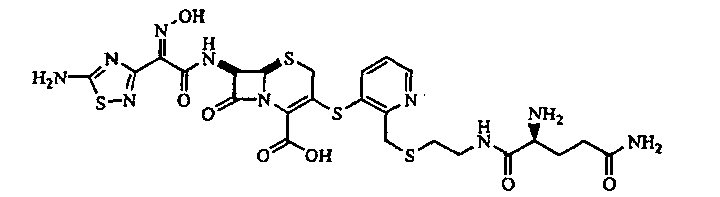

### Cmpd 2-O (7R)-7-[(Z)-2-(5-amino-1,2,4-thiadiazol-3-yl)-2-(hydroxyimino)acetamido]-3-{2-[2-(5-methyl-1,3-dioxolan-4-en-2-on-4-yl)methoxycarbonyl)aminoethylthiomethyl]pyrid-3-ylthio}-3-cephem-4-carboxylic acid

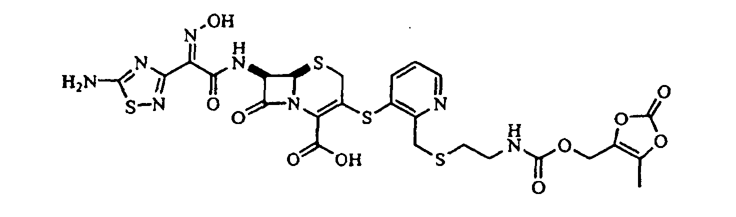

### Cmpd 2-P (7R)-7-[(Z)-2-(5-amino-1,2,4-thiadiazol-3-yl)-2-(hydroxyimino)acetamido]-3-{2-(2-N-(L)-pyroglutamylaminoethylthiomethyl]pyrid-3-ylthio}-3-cephem-4-carboxylic acid

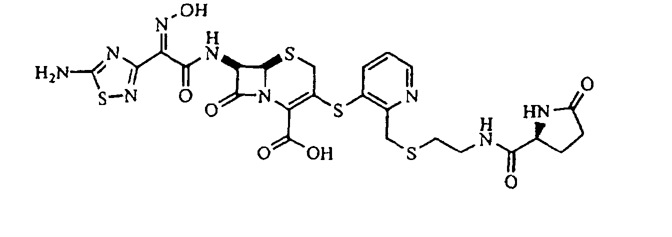

Thus, it will be appreciated that the compounds, methods and compositions of the invention are effective against various β-lactam resistant strains of bacteria which pose an increasing health risk to society.

### EXAMPLES

The examples below are non-limiting and are merely representative of various aspects and features of the present invention. The examples describe methods for synthesizing compounds of the invention and various protocols.

### Example 1:

### 3-t-butylthio-2-hydroxymethylpyridine

To a suspension of 3-t-butylthio-2-carboxypyridine (10.0 g, 47.4 mmol) in tetrahydrofuran (200 mL) cooled to -5 °C was added trethylamine (8.25 mL, 47.4 mmol) followed by addition of ethyl chloroformate (4.38 g, 47.4 mmol) and reaction was stirred for 30 min at 0 °C. Lithium borohydride (2.58 g, 118 mmol) was added in portions, maintaining the temperature below 5 °C. After the addition was complete the reaction was allowed to warm to room temperature and stirred for 1 hour. Temperature was lowered to -5 °C and methanol (10 mL) was added followed by addition of aqueous sodium hydroxide (10 mL, 10%). After addition of ethyl acetate (50 mL) and water (40 mL) dilute hydrochloric acid was added to pH=5.0.

Precipitated inorganic salt was filtered off and organic layer of the filtrate was separated. After washing aqueous layer thoroughly with ethyl acetate combined organic extracts were dried over sodium sulfate and concentrated to produce yelow oil (7.21 g) of title product. ¹H NMR (CDCl₃) δ 1.40 (s, 9H), 4.50 (br s, 1H), 4.90 (s, 2H), 7.20 (m, 1H), 7.80 (d, J=7, 1H), 8.55 (d, J=5, 1H). 1H.).

### Example 2:

### 3-t-butylthio-2-(2-t-butoxycarbonylaminoethylthiomethyl)-pyridine

A solution of Vilsmeier reagent was prepared by addition of thionyl chloride (1.09 g, 9.17 mmol) to dry dimethylformamide (10 mL) at room temperature. After 30 min. the above solution was transferred to a solution of 3-t-butylthio-2-hydroxymethylpyridine (1.20 g, 6.09 mmol) in dry dimethylformamide (5 mL).

After stirring for 30 min at room temperature powdered potassium carbonate (4.15 g, 30 mmol) was added followed by addition of 2-t-butoxycarbonylaminoethanethiol and sodium iodide (0.15 g, 1.05 mmol) and vigorous stirring was continued for 16 hours. Reaction mixture was partitioned between ethyl acetate and water. Organic extract was thoroughly washed with water, then dried over sodium sulfate and evaporated to produce oily residue, which was purified by flash chromatography on silica gel (ethyl acetate-hexane- 1/2) to afford oily title product (1.10 g). ¹H NMR (CDCl₃) δ 1.40 (s, 9H), 1.57 (s, 9H), 2.80 (t, J=6H, 2H), 3.43 (t, J=6H, 2H), 4.35 (s, 2H), 5.40 (br s, 1H), 7.28 (dd, J=6, J=4, 1H), 7.95 (d, J=6, 1H), 8.63 (d, J=4, 1H).

### Example 3:

### 2-(2-aminoethylthiomethyl)-3-mercaptopyridine dihydrochloride

A solution of 3-t-butylthio-2-(2-t-butoxycarbonylaminoethylthiomethyl)-pyridine (0.60 g) in hydrochloric acid (5 mL, 6.0 M) was refluxed for 3 days (until NMR of a sample in D₂O does not show any t-butyl signal) and the reaction mixture was evaporated to dryness to produce the desired solid dihydrochloride (0.40 g), which was used for next step without further purification. ¹H NMR (D₂O) δ 2.85 (t, J=6H, 2H), 3.15 (t, J=6H, 2H), 4.20 (s, 2H), 7.62 (m, 1H), 7.30 (d, J=4, 1H), 7.43 (d, J=6, 1H).

### Example 4:

### Bis(2-(2-aminoethylthiomethyl)pyrid-3-yl) disulfide

A solution of 2-(2-aminoethylthiomethyl)-3-mercaptopyridine dihydrochloride (0.40 g) in water (4 mL) basified with addition of concentrated ammonium hydroxide and a stream of air was bubbled through it for 16 hours. Reaction mixture was evaporated to dryness to produce solid residue of title product and ammonium chloride which was used for next step without further purification. ¹H NMR (D₂O) δ 2.75 (t, J=6H,2H), 3.15 (t, J=6H,2H), 4.00 (s, 2H), 7.38 (dd, J=4, J=6, 1H), 8.21 (d, J=6, 1H), 8.38 (d, J=4, 1H).

### Example 5:

### Bis[2-(2-t-butoxycarbonylaminoethylthiomethyl)pyrid-3-yl] disulfide

To a solution of crude bis(2-(2-aminoethylthiomethyl)pyrid-3-yl) disulfide (0.43 g, 1.08 mmol) in methanol (50 mL) were added di-t-butyldicarbonate (1.19 g, 5.46 mmol) and triethylamine (0.73 g, 7.20 mmol). After 45 min. at room temperature reaction mixture was evaporated to dryness and redissolved in methylene chloride. The insoluble residue was filtered off and the filtrate was concentrated under reduced pressure to produce oily residue. Flash chromatogaphy on silica gel (5% methanol in methylene chloride) yielded oily title product (0.28 g). ¹H NMR (CDCl₃) δ 1.57 (s, 9H), 2.78 (t, J=6H,2H), 3.40 (m, 2H), 4.12 (s, 2H), 5.20 (br s, 1H), 7.30 (dd, J=6, J=4, 1H), 8.03 (d, J=6, 1H), 8.48 (d, J=4, 1H).

### Example 6:

### (7R)-7-[(Z)-2-(2-amino-5-chlorothiazol-4-yl)-2-(triphenylmethoxyimino)acetamido]-3-[2-(2-t-butoxycarbonylaminoethylthiomethyl)pyrid-3-ylthio]-3-cephem-4-carboxylate, diphenylmethyl ester

To a solution bis[2-(2-t-butoxycarbonylaminoethylthiomethyl)pyrid-3-yl] disulfide (83 mg, 0.14 mmol) in dimethylformamide (1 mL) and (7*R*)-7-[(Z)-2-(2-amino-5-chlorothiazol-4-yl)-2-(triphenylmethoxyimino)acetamido]-3-chloro-3-cephem-4-carboxylate, diphenylmethyl ester (175 g, 0.21 mmol) were added triphenyl phosphine (75 g, 0.28 mmol) and water (0.01 mL). After 3 hours at room temperature the reaction mixture was partitioned between water and ethyl acetate. Organic layer was dried over sodium sulfate and concentrated under reduced pressure to afford oily residue which was purified by radial chromatography on silica gel (2% methanol in methylene chloride) to yield title product (205 mg). ¹H NMR (CDCl₃/CD₃OD) δ 1.40 (s, 9H), 2.60 (t, J=6, 2H), 3.12 (d, J=16, 1H), 3.25 (m, 3H), 3.90 (s, 2H), 5.18 (d, J=6, 1H), 6.00 (d, J=6, 1H), 6.98 (s, 1H), 7.20-7.60 (m, 25H), 7.70 (d, J=6, 1H), 8.42 (d, J=6, 1H).

### Example 7:

### (7R)-7-[(Z)-2-(2-amino-5-chlorothiazol-4-yl)-2-(hydroxyimino)acetamido]-3-[2-(2-aminoethylthiomethyl)pyrid-3-ylthio]-3-cephem-4-carboxylate, methanesulfonic acid salt

(7*R*)-7-[(Z)-2-(2-amino-5-chlorothiazol-4-yl)-2-(triphenylmethoxyimino)acetamido]-3-[2-(2-t-butoxycarbonylaminoethylthiomethyl)pyrid-3-ylthio]-3-cephem-4-carboxylate, diphenylmethyl ester was subjected to standard deprotection. Crude product was dissolved in small volume of water and free zwitterion was isolated by loading the solution on HP20 reverse phase column, eluting with water until eluate become neutral and eluting of the product with water/acetonitrile mixture (60/40). The fractions containing pure product were evaporated to dryness under reduced pressure. The solid residue of zwitterionic product was converted into soluble methanesulfonate salt by stirring of a suspension in small volume of water and adding aqueous methanesulfonic acid until all material solubilized. Lyophilization of resulting solution produced methanesulfonate salt of title product. ¹H NMR (D₂O) δ 2.55 (s, 5H), 2.66 (t, J=6, 2H), 3.21 (t, J=6, 2H), 3.30 (d, J=17, 1H), 3.74 (d, J=17, 1H), 4.20 (s, 2 H), 5.33 (d, J=6, 2H), 5.88 (d, J=6, 1H), 7.80 (dd, J=4, J=6, 1H), 8.24 (d, J=6, 1H), 8.50 (d, J=4, 1H).

### Example 8:

### Bis{2-[2-N,N'-bis(t-butoxycarbonyl)guanidinoethylthiomethyl]pyrid-3-yl} disulfide

To a suspension of Bis(2-(2-aminoethylthiomethyl)pyrid-3-yl) disulfide (98 mg, 0.41 mmol) dimethylformamide was added 1-[N,N'-bis(t-butoxycarbonyl)carboxamidino]-1H-pyrazole (141 mg, 0.45 mmol) and the reaction was heated at 40 °C for 16 hours. Reaction mixture was partitioned between ethyl acetate and water. Organic extract was dried over sodium sulfate and concentrated under reduced pressure to afford oily residue which was purified by radial chromatography on silica gel (2% methanol in methylene chloride) to afford oily title product (140 mg). ¹H NMR (CDCl₃) δ 1.60 (s, 18H), 2.80 (t, J=6, 2H), 3.72 (m, 2H), 4.07 (s, 2H), 7.25 (dd, J=6, J=4, 1H), 8.00 (d, J=6, 1H), 8.43 (d, J=4, I H), 8.66 (br s, 1H).

### Example 9:

### (7R)7-[(Z)-2-(5-amino-1,2,4-thiadiazol-3-yl)-2-(triphenylmethoxyimino)acetamido]-3-methanesulfonyloxy-3-cephem-4-carboxylate, diphenylmethyl ester

To a suspension of (Z)-2-(5-amino-1,2,4-thiadiazol-3-yl)-2-(triphenylmethoxyimino)acetic acid (2.52 g, 4.00 mmol) and (7*R*)-7-amino-3-methanesulfonyloxy-3-cephem-4-carboxylate, diphenylmethyl ester, tosylate salt (1.71 g, 4.00 mmol) in tetrahydrofuran cooled to -50 °C was added diisopropylethylamine (1.54 g, 12.00 mmol) followed by phosphorous oxychloride (0.85 g, 5.60 mmol) and the reaction mixture was stirred for 1 hour at -30 °to -35 °C. Dilute hydrochloric acid was added to cold mixture and the reaction was allowed to reach room temperature. After partitioning between water and ethyl acetate the organic layer was thoroughly washed with dilute hydrochloric acid, brine, dried over sodium sulfate and filtered through a plug of silica gel to remove polar impurities. Removing of solvents under reduced pressure afforded yellowish foam of the title product (2.86 g). ¹H NMR (CDCl₃/CD₃OD) δ 2.85 (s, 3H), 3.56 (d, J=16, 1H), 3.83 (d, J=16, 1H), 5.22 (d, J=6, 1H), 6.18 (d, J=6, 1H), 7.00 (s, 1H), 7.30 - 7.50 (m, 25H).

### Example 10:

### (7R)-7-[(Z)-2-(5-amino-1,2,4-thiadiazol-3-yl)-2-(triphenylmethoxyimino)acetamido]-3-{2-[2-N,N'-bis(t-butoxycarbonyl)guanidinoethylthiomethyl]pyrid-3-ylthio}-3-cephem-4-carboxylate, diphenylmethyl ester

(7*R*)-7-[(Z)-2-(5-amino-1,2,4-thiadiazol-3-yl)-2-(triphenylmethoxyimino)acetamido]-3-methanesulfonyloxy-3-cephem-4-carboxylate, diphenylmethyl ester was reacted with di {2-[2-N,N'-bis(t-butoxycarbonyl)guanidinoethylthiomethyl]pyrid-3-yl} disulfide in a manner similar to that described for preparation of 67 to produce title compound. ¹H NMR (CDCl₃/CD₃OD) δ 1.40 (s, 18H), 2.61 (t, J=6,2H), 3.06 (d, J=16, 1H), 3.23 (d, J=16, 1H), 3.53 (t, J=6, 2H), 3.80 (s, 2H), 5.10 (d, J=6, 1H), 5.96 (d, J=6, 1H), 6.92 (s, 1H), 7.10-7.30 (m, 27H), 8.62 (d, J=6, 1H).

### Example 11:

### (7R)-7-[(Z)-2-(5-amino-1,2,4-thiadiazol-3-yl)-2-(hydroxyimino)acetamido]-3-{2-[2-guanidinoethyithiomethyl]pyrid-3-ylthio}-3-cephem-4-carboxylate, trifluoroacetic acid salt

To a solution of (7*R*)-7-[(Z)-2-(5-amino-1,2,4-thiadiazol-3-yl)-2-(triphenylmethoxyimino)acetamido]-3-{2-[2-N,N'-bis(t-butoxycarbonyl)guanidinoethylthlomethyl]pyrid-3-ylthio}-3-cephem-4-carboxylatc, diphenylmethyl ester (200 mg) in methylene chloride (20 mL) were added triethyl silane (10 mL) and trifuloroacetic acid (0.5 mL) room temperature. After 40 min. reaction, the mixture was concentrated under reduced pressure and the oily residue was triturated with diisopropyl ether and filtered to produce precipitate of crude product. The resulting solid was dissolved in small amount of water and after filrering off insoluble material product was isolated from the filtrate by reverse phase MPLC chromatography (Amberchrom CG-161, gradient of acetonitrile in 0.1% trifluoroacetic acid). Fraction containing pure product was concentrated under reduced pressure and lyophilized to produce pure title product (25 mg). ¹H NMR (D₂O) δ 2.80 (t, J=6, 2H), 3.30 (d, J=16, 1H), 3.38 (t, J=6, 2H), 3.75 (d, J=16, 1H), 4.21 (s, 2H), 5.35 (d, J=6, 1H), 5.89 (d, J=b, 1H), 7.88 (dd, J=4, J=6, 1H), 8.46 (d, J=6, 1H), 8.55 (d, J=4, 1H).

### Example 12:

### (7R)-7-[(Z)-2-(2-amino-5-chlorothiazol-4-yl)-2-(triphenylmethoxyimino)acetamido]-3-{2-[2-N,N'-bis(t-butoxycarbonyl)guanidinoethylthiomethyl]pyrid-3-ylthio}-3-cephem-4-carboxylate, diphenylmethyl ester

Bis{2-[2-N,N'-bis(t-butoxycarbonyl)guanidinoethylthiomethyl]pyrid-3-yl} disulfide was reacted with (7*R*)-7-[(Z)-2-(2-amino-5-chlorothiazol-4-yl)-2-(triphenylmethoxyimino)acetamido]-3-chloro-3-cephem-4-carboxylate, diphenylmethyl ester in a manner similar to that described for preparation of 67 to produce title compound. ¹H NMR (CDCl₃) δ 1.57 (s, 18H), 2.83 (m, 2H), 3.22 (d, J=16, 1H), 3.37 (d, J=16, 1H), 4.05 (d, J=10, 1H), 4.10 (d, J=10, 1H), 5.22 (d, J=6, 1H), 6.10 (m, 1H), 7.10 (s, 1H), 7.30-7.50 (m, 27H), 7.80 (d, J=6, 1H), 8.58 (d, J=4, 1H).

### Example 13:

### (7R)-7-[(Z)-2-(2-amino-5-chlorothiazol-4-yl)-2-(hydroxyimino)acetamido]-3-[2-(guanidinoethylthiomethyl)pyrid-3-ylthio]-3-cephem-4-carboxylate, trifluoroacetic acid salt

To a solution of (7R)-7-[(Z)-2-(2-amino-5-chlorothiazol-4-yl)-2-(triphenylmethoxyimino)acetamido]-3-{2-[2-N,N'-bis(t-butoxycarbonyl)guanidinoethylthiomethyl]pyrid-3-ylthio}-3-cephem-4-carboxylate, diphenylmethyl ester (160 mg, 0.128 mmol) in methylene chloride (5.0 mL) and triethylsilane (1.5 mL) colled to 0 °C was added trifluoroacetic acid (8.0 mL) and reaction was stirred at 10 °C for 7 hours. Reaction mixture was poured into ethyl ether cooled to -10°C (80 mL) and the resulting precipitate was washed thoroughly with ether to produce solid of title product (90 mg). ¹H NMR (D₂O) δ 2.80 (m, 2H), 3.05-3.2 (m, 3H), 3.78 (d, J=16, 1H), 4.12 (s, 1H), 5.38 (d, J=6, 1H), 5.92 (d, J=6, 1H), 7.85 (dd, J=4, J=6, 1H), 8.32 (d, J=6, 1H), 8.56 (d, J=4, 1H).

### Example 14:

### (7R)-7-[(Z)-2-(5-amino-1,2,4-thiadiazol-3-yl)-2-(triphenylmethoxyimino)acetamido]-3-[2-(t-bntoxycarbonylaminoethylthiomethyl)pyrid-3-ylthio]-3-cephem-4-carboxylate, diphenylmethyl ester

(7*R*)-7-[(Z)-2-(5-amino-1,2,4-thiadiazol-3-yl)-2-(triphenylmethoxyimino)acetamido]-3-methanesulfonyloxy-3-cephem-4-carboxylate, diphenylmethyl ester was reacted with di[2-(2-t-butoxycarbonylaminocthylthlomethyl)pyrid-3-yl] disulfide in a manner similar to that described for preparation of 67 to produce title compound. ¹H NMR (CDCl₃/CD₃OD) δ 1.40 (s, 9H), 2.59 (t, J=6, 2H), 3.12 (d, J=16, 1H), 3.22 (m, 3H), 3.90 (s, 2H), 5.18 (d, J=6, 1H), 6.02 (d, J=6, 1H), 7.00 (s, 1H), 7.20-7.50 (m, 26H), 7.72 (d, J=6, 1H), 8.42 (d, J=6, 1H).

### Example 15:

### (7R)-7-[(Z)-2-(5-amino-1,2,4-thiadiazol-3-yl)-2-(hydroxyimino)acetatnido]-3-[2-(aminoethylthiomethyl)pyrid-3-ylthio]-3-cephem-4-carboxylate, methanesulfonic acid salt

(7*R*)-7-[(Z)-2-(5-amino-1,2,4-thiadiazol-3-yl)-2-(triphenylmethoxyimino)acetamido]-3-[2-(t-butoxycarbonylaminoethylthiomethyl)pyrid-3-ylthio]-3-cephem-4-carboxylate, diphenylmethyl ester was subjected to standard deprotection. Crude product was dissolved in small volume of water and free zwitterion was isolated by loading the solution on HP20 reverse phase column, eluting with water until eluate become neutral and elution of the product with water/acetonitrile mixture (60/40). The fractions containing pure product were evaporated to dryness under reduced pressure. The solid residue of zwitterionic product was converted into soluble, methanesulfonate salt by stirring suspension in small volume of water and addinq aqueous methanesulfonic acid until all material solubilized. Lyophilization of resulting solution produced methanesulfonate salt of title product. ¹H NMR (D₂O) δ 2.70 (s, 2.5H), 2.80 (t, J=6, 2H), 3.15 (t, J=6, 2H), 3.21 (d, J=16, 1H), 3.68 (d, J=16, 1H), 4.10 (s, 2 H), 5.29 (d, J=6, 2H), 5.83 (d, J=6, 1H), 7.65 (dd, J=4, J=6, 1H), 8.20 (d, J=6, 1H), 8.41 (d, J=4, 1H).

### Example 16:

### 2-chloro-4-chloromethyl-5-(2-phenylsulfonylethyl)thio-1,3-thiazole

To a solution of 4-chloromethyl-5-(2-phenylsulfonylechyl)thio-1,3-thiazole (400 mg, 1.2 mmol) in N,N-dimethylformamide (3 mL) was added N-chlorosuccinimide (160 mg, 1.2 mmol), and the mixture was stirred at room temperature for 16 h. The resulting mixture was poured into water and extracted with ethyl acetate. The organic solution was washed with water, dried over sodium sulfate, and concentrated. The residue was chromatographed (silica gel, 20% ethyl acetate-hexane), affording 260 mg of the title compound. ¹H NMR (CDCl₃) δ 3.09 (2H), 3.36 (2H), 4.60 (2H), 7.5-7.9 (5H).

### Example 17:

### Methyl 6-tert-butoxycarbonylamino-2-pyridinecarboxylate

To a suspension of 2,6-pyridinedicarboxylic acid (8.35 g, 50 mmol) and triethylamine (5.05 g, 50 mmol) in *tert*-butyl alcohol (ca. 60 mL) was dropwise added diphenylphosphoryl azide (13.8 g, 50 mmol), and the mixture was stirred at room temperature until the reaction mixture became a clear solution. The solution was then slowly heated and refluxed for 2.5 hour. After cooling, the mixture was partitioned between ethyl acetate-hexane (1:1, v/v) and 1% aqueous sodium hydroxide. The organic layer was washed again with 1% aqueous sodium hydroxide. All the aqueous solutions were combined and neutralized to pH = 5 with concentrated hydrochloric acid in the presence of ethyl acetate. The ethyl acetate layer was washed with brine, dried over sodium sulfate and concentrated to obtain 5.0 g of 6-*tert*-butoxycarbonylamino-2-pyridinecarboxylic acid.

This acid (1.38 g, 5.8 mmol) was added to an ethyl acetate solution (20 mL) of Vilsmeier reagent (prepared from phosphorus oxychloride (1.06 g, 6.9 mmol) and N,N-dimethylformamide (0.51 g, 6.9 mmol)) and stirred at 0 °C for 1 h. Anhydrous methanol (10 mL) was added, and the mixture was stirred for an additional 15 min. The mixture was then concentrated and partitioned between ethyl acetate and saturated aqueous sodium bicarbonate. The organic phase was washed with 1% aqueous lithium chloride and then brine, dried over sodium sulfate, and concentrated to afford 1.4 g of the title compound as a white crystalline solid. ¹H NMR (CDCl₃) δ 1.51 (9H), 3.98 (3H), 7.54 (br s, 1H), 7.80 (2H), 8.16 (1H).

### Example 18:

### Methyl 6-tert-butoxycarbonylamino-2-pyridineglyoxylate

To a solution of methyl 6-*tert*-butoxycarbonylamino-2-pyridinecarboxylate (5.3 g, 21 mmol) and methyl methylsulfinylmethyl sulfide (3.7 g, 29 mmol) in N,N-dimethylformamide (15 mL) was added sodium hydride (60% in oil, 1.3 g, 34 mmol) at 0 °C, and the mixture was stirred at room temperature for 2 h. Diethyl ether (100 mL) was added and the mixture was stirred for 30 min. The resulting precipitate was collected, dissolved in water, neutralized to pH = 5 with 6% hydrochloric acid, and extracted with diethyl ether. The ether extract was dried and concentrated to afford 3.5 g of the adduct.

This adduct was dissolved in dichloromethane (10 mL), and pyridine (3.1 g, 40 mmol) and trifluoroacetic anhydride (4.2 g, 20 mmol) were added at 0 °C. After stirring at room temperature for 30 min, 0.5 M sodium methoxide in methanol (15 mL) was added. The mixture was then stirred for 1h and concentrated. Extraction with ethyl acetate from aqueous sodium bicarbonate followed by evaporation afforded 3.7 g of the crude dithioorthoester.

The above crude (10 g) was dissolved in acetic acid (30 mL) and sodium perborate monohydrate (8.4 g, 84 mmol) was added. The resulting suspension was stirred for 2.5 h. The solvent was evaporated and ethyl acetate was added. The resulting solution was then washed with aqueous sodium bicarbonate, concentrated, and chromatographed (silica gel, dichloromethane) to afford 3.2 g of the title compound. ¹H NMR (CDCl₃) δ 1.53 (9H), 3.98 (3H), 7.32 (br s, 1H), 7.77 (d, 1H), 7.86 (t, 1H), 8.24 (d, 1H).

### Example 19:

### 2-(2-tert-butoxycarbonylaminopyrid-6-yl)-2-(triphenylmethoxyimino)acetic acid

To a solution of methyl 6-*tert*-butoxycarbonylamino-2-pyridineglyoxylate (1.6 g, 5.7 mmol) in 95% ethanol were added hydroxylamine hydrochloride (0.6 g, 8.6 mmol) and pyridine (0.96 mL, 9.7 mmol), and the mixture was stirred at room temperature for 1.5 h. The solvent was removed *in vacuo* and the oily residue was partitioned between ethyl acetate and water. The organic solution was washed with 3% hydrochloric acid and then water, dried over sodium sulfate, and concentrated to give 1.63 g of the crude hydroxyimino ester.

The crude ester was dissolved in dichloromethane and cooled to 0 °C. Trityl chloride (1.87 g, 6.7 mmol) and triethylamine (0.84 mL, 6.7 mmol) were added and stirred for 2 h. The mixture was diluted with dichloromethane, washed with 1% hydrochloric acid and concentrated to afford 3.1 g of the tritylated ester. Crystallization from hexane-ethyl acetate provided the *syn*-oxyimino product that was free from the *anti*-isomer.

The above ester (4.25 g, 7.91 mmol) was dissolved in a mixed solvent of isopropyl alcohol, tetrahydrofuran and water (10:5:1, v/v/v) containing sodium hydroxide (0.64 g, 16 mmol) and was stirred at 65 °C for 1 h. The reaction mixture was concentrated to dryness, triturated with hexane, and partitioned between dichloromethane and diluted hydrochloric acid. The organic layer was concentrated, affording 3.7 g of the title compound. ¹H NMR (CDCl₃) δ 1.53 (9H), 7.05-7.50 (16H), 7.55 (1H), 7.64 (1H).

### Example 20:

### 4-Hydroxymethyl-3-mercaptopyridine

To a suspension of 3-mercaptoisonicotinic acid (1.80 g, 11.6 mmol) in dry THF (70 mL) was added slowly borane in THF (52 mL, 1 M, 52 mmol) and the reaction mixture was stirred for 30 min. The solvent was evaporated under reduced pressure, methanol (40 mL) was added, and after gas evolution stopped, concentrated hydrochloric acid (3.6 mL) was added. The solution was filtered and the filtrate was evaporated to dryness. The residue was redissolved in a small volume of water, concentrated aqueous ammonia (3.6 mL) was added and the reaction mixture was evaporated to dryness. After overnight drying under vacuum, a quantitative yield of the title product was obtained, which was used for the next step without purification. ¹H NMR (CD₃OD) δ 4.78 (s, 2H), 8.13 (d, 2H, J=6), 8.60 (d, 2H, J=6), 8.70 (s, 1H).

### Example 21:

### 3-(Triphenylmethylthio)-4-hydroxymethylpyridine

4-hydroxymethyl-3-mercaptopyridine (100 mg, 0.71 mmol) was dissolved in DMF (5 mL) and diisipropylethylamine (0.12 mL, 0.71 mmol) was added followed by triphenylmethylchloride (197 mg, 0.71 mmol). After 30 min. the reaction mixture was partitioned between water and ethyl acetate, and the organic layer was thoroughly washed with water and dried with anhydrous sodium sulfate. Purification by radial chromatography on silica gel produced pure title material (67 mg, 25% yield). ¹H NMR (CDCl₃) δ 4.17 (s, 2H), 7.20-7.40 (m, 15H), 7.42 (d, 1H, J=6), 8.22 (s, 1H), 8.40 (d, 1H, J=6).

### Example 22:

### 4-{[(2-N-tert-butoxycarbonyleminoethyl)thio]methyl}-3-(triphenylmethylthio)pyridine

A solution of thionyl chloride (126 mg, 1.05 mmol) in dry DMF (2 mL) was stirred for 30 min. at room temperature. This solution was then cannulated into a solution of 3-(triphenylmethylthio)-4-hydroxymethylpyridine (270 mg, 0.70 mmol) in DMF (2 mL) at room temperature and the reaction was stirred for 30 min. 2-(N-t-butoxycarbonylaminoethyl)thiol (187 mg, 1.05 mmol) and powdered potassium carbonate (486 mg, 3.52 mmol) were added to the reaction mixture and stirring was continued for an additional 30 min. The reaction was partitioned between water and ethyl acetate and the organic layer was thoroughly washed with water and dried. After removing the solvent under reduced pressure the residue was purified by radial chromatography on silica gel (hexane/ethyl acetate - 4/1) to yield the title material as an off-white solid (220 mg, 58%). ¹H NMR (CDCl₃) δ 1.46 (s, 9H), 2.42 (t, 2H, J=6), 3.18 (s, 2H), 3.23 (m, 2H), 4.80 (br s, 1H), 7.10 (d, 1H, J=6), 7.20 - 7.45 (m, 15H), 8.28 (m, 2H).

### Example 23:

### 4-[(2-N-tert-butoxycarbonylaminoethylthio)methyl]-3-(tert-butoxycarbonylthio)pyridine

To 4-(2-N-t-butoxycarbonylaminoethylthiomethyl)-3-(triphenylmethylthio)pyridine (790 mg, 1.45 mmol) and triethylsilane (2 mL, 12.5 mmol) dissolved in methylene chloride (15 mL) was added trifluoroacetic acid (15 mL). After 1 hour stirring at room temperature, the reaction mixture was evaporated to dryness under reduced pressure and the residue was dissolved in dry tetrahydrofuran (10 mL). To this solution diisopropylethylamine (1.40 mL, 7.84 mmol) was added, followed by di-t-butyldicarbonate (1280 mg, 5.88 mmol), and after 3 hr. reaction at room temperature the solvent was removed at reduced pressure. Purification on silica gel by radial chromatography (methylene chloride/methanol - 50/1) produced pure title product as a yellow foam (460 mg, 79%). ¹H NMR (CDCl₃) δ 1.42 (s, 9H), 1.50 (s, 9H), 2.58 (t, 2H, J=6), 3.23 (t, 2H, J=6), 3.84 (s, 2H), 4.95 (br s, 1H), 7.42 (d, 1H, J=6), 8.58 (d, 1H, J=6), 8.70 (s, 1H).

### Example 24:

### 4-(2-N-t-butoxycarbonylaminoethylthiomethyl)-3- mercaptopyridine

To a solution of 4-(2-N-t-butoxycarbonylaminoethylthiomethyl)-3-(tert-butoxycarbonylthio)pyridine (415 mg, 1.04 mmol) in methanol (2 mL) was added under nitrogen a methanolic solution of sodium methoxide (1.04 mL, 1.0 M), and the reaction mixture was heated at 50 °for 30 min. After evaporating the solvent in vacuum the reaction was partitioned between water and ethyl acetate with addition of acetic acid (125 mg, 2.08 mmol). Evaporation of the solvent under reduced pressure yielded the title material (298 mg, 96% yield) which was used in the next step without further purification. ¹H NMR (CD₃OD) δ 1.42 (s, 9H), 2.60 (m, 2H), 3.20 (m, 2H), 4.0 (s, 2H), 4.95 (br s, 1H), 7.52 (d, 1H, J=6), 8.00 (d, 1H, J=6), 8.40 (s, 1H).

### Example 25:

### (7R)-7-[(Z)-2-(2-amino-5-chloroihiazol-4-yl)-2-(triphenylmethoxyimino)acetamido]-3-[4-(N-tert-butoxycarbonylaminoethylthio)pyrid-3-ylthio]-3-cephem-4-carboxylate, diphenylmethyl ester

To a solution of 4-(2-N-t-butoxycarbonylaminoethylthiomethyl)-3-mercaptopyridine (298 mg, 0.99 mmol) in ethyl acetate (5 mL) was added (7R)-7-[(Z)-2-(2-amino-5-chlorothiazol-4-yl)-2-(triphenylmethoxyimino)acetamido]-3-methanesulfonyloxy-3-cephem-4-carboxylate diphenylmethyl ester. After 1 hour stirring at room temperature the reaction was partitioned between ethyl acetate and dilute sodium bicarbonate solution (30 mL, 1%). The organic layer was dried over anhydrous sodium sulfate and was evaporated under reduced pressure. The residue was purified by radial chromatography on silica gel to yield pure title product (366 mg, 33%). ¹H NMR (CD₃OD) δ 1.40 (s, 9H), 2.50 (m, 2H), 3.20 (m, 2H), 3.12 (d, 1H, J=16) 3.80 (s, 2H), 3.38 (d, 1H, J=16), 5.25 (d, 1H, J=6), 6.00 (d, 1H, J=6), 7.00 (s, 1H), 7.20-7.40 (m, 25H), 7.42 (d, 1H, J=6) 8.40 (d, 1H, J=6), 8.50 (s, 1H).

### Example 26:

### (7R)-7-[(Z)-2-(2-amino-5-chlorothiazol-4-yl)-2-(hydroxyimino)acetamido]-3-(4-aminoethylthiopyrid-3-ylthio)-3-cephem-4-carboxylate, trifluoroacetic acid salt

To a suspension of (7R)-7-[(Z)-2-(2-amino-5-chlorothiazol-4-yl)-2-(triphenylmethoxyimino)acetamido]-3-[4-(N-*tert*-butoxycarbonylaminoethylthio)pyrid-3-ylthio]-3-cephem-4-carboxylate, diphenylmethyl ester (342 mg, 0.31 mmol) in methylene chloride (3.5 mL) was added triethylsilane (1.7 mL, 4.06 mmol) followed by addition of trifluoroacetic acid (4.5 mL). After stirring at room temperature for 1 hr. the reaction was cooled to 0 °C and isopropyl ether (30 mL) was added. Stirring was continued at 0 °C for 10 min and the resulting precipitate was filtered and washed thoroughly with diisopropyl ether and dried in vacuum, yielding the title deprotected cephem bis-trifluoroacetate salt (222 mg, 85%). ¹H NMR (D₂O) δ 2.90 (t, 2H, J=6), 3.31 (t, 2H, J=6), 3.43 (d, 1H, J=17), 3.85 (d, 1H, J=17), 4.18 (s, 2H), 5.43 (d, 1H, J=4.5), 5.97 (d, 1H, J=4.5), 8.10 (d, 1H, J=6), 8.66-8.68 (m, 2H).

The following compounds were also prepared by methods essentially analogous to those used for preparation of the compounds described above:

### Example 27:

### (7R)-7-[(Z)-2-(5-amino-1,2,4-thiadiazol-3-yl)-2-(hydrogylmino)acetamido]-3-[3-(2-aminoethylthiomethyl)pyrid-4-ylthio]-3-cephem-4-carboxylate, trifluoroacetic acid salt

¹H NMR (CD₃OD) δ 2.90 (t, 2H, J=6), 3.27 (t, 2H, J=6), 3.47 (d, 1H, J=18), 4.08 (s, 2H), 5.49 (d, 1H, J=5), 6.11 (d, 1H, J=5), 7.66 (d, 1H, J=6), 8.55 (d, 1H, J=6), 8.69 (s, 1H).

### Example 28:

### (7R)-7-[(Z)-2-(2-aminopyrid-6-yl)-2-(hydroxyimino)acetamido]-3-[3-(2-aminoethylthiomethyl)pyrid-4-ylthio]-3-cephem-4-carboxylate, trifluoroacetic acid salt

### Example 29:

### (7R)-7-[(Z)-2-(5-amino-1,2,4-thiadiazol-3-yl)-2-(hydroxyimino)acetamido]-3-[3-(2-guanidinoethylthiomethyl)pyrid-4-ylthio]-3-cephem-4-carboxylate, trifluoroacetic acid salt

¹H NMR (CD,OD) δ 2.93 (s, 3H), 2.95 (t, 2H, J=6), 3.57 (d, 1H, J=18), 3.59 (t, 2H, J=6), 4.13 (d, 1H, J=18), 5.58 (d, 1H, J=5), 6.21 (d, 1H, J=5), 7.83 (d, 1H, J=6), 8.68 (d, 1H, J=6), 8.84 (s, 1H).

### Example 30:

### (7R)-7-[(Z)-2-(5-amino-1,2,4-thiadiazol-3-yl)-2-(hydroxyimino)acetamido]-3-[4-(2-aminoethylthiomethyl)pyrid-3-ylthio]-3-cephem-carboxylate. trifluoroacetic acid salt

¹H NMR (CD₃OD) δ 2.85 (t, 2H, J=9), 3.33 (t, 2H, J=9), 3.43 (d, 1H, J=24), 3.78 (d, 1H, J=24), 4.18 (d, 2H, J=5), 5.32 (d, 1H, J=7), 5.94 (d, 1H, J=7), 8.11 (d, 1H, J=7), 8.72 (d, 1H, J=8), 8.77 (s, 1H).

### Example 31:

### (7R)-7-[(Z)-2-(2-aminopyrid-6-yl)-2-(hydroxyimino)acetamido]-3-[4-(2-aminoethylthiomethyl)pyrid-3-ylthio]-3-cephem-4-carboxylate, trifluoroacetic acid salt

¹H NMR (CD₃OD) δ 2.68 (t, 2H, J=9), 3.09 (t, 2H, J=9), 2.41 (2H, J=23), 3.90 (s, 2H), 5.22 (d, 1H, J=6), 5.81 (d, 1H, J=6), 6.88 (d, 1H, J=9), 6.98 (d, 1H, J=10), 7.70 (s, 1H), 7.82 (t, 1H, J=9), 8.41 (d, 1H, J=7), 8.55 (d, 1H, J=7).

### Example 32:

### 3-triphenylmethylthio-2-hydroxymethylpyridine

Into a 3 L round bottom flask equipped with mechanical stirrer and nitrogen purge was charged 3-mercapto-2-hydroxymethylpyridine hydrochloride (25.0 g, 0.141 mol, 1 eq.) followed by DMF (1.2 L) and to the cloudy suspension was charged finely powdered potassium carbonate (58.5 g, 0.423 mol, 3.0 eq.). To the resulting solution was charged triphenylmethylchloride (38.1 g, 0.137 mol, 0.97 eq) and the reaction mixture was allowed to stir at room temperature for three hours until TLC analysis showed reaction completion (ethyl acetate-hexanes). The DMF solution was poured into 3 L of 4:1 water/ethyl acetate. The aqueous layer was separated and extracted an additional time with ethyl acetate (200 mL). The organics were combined and hexane (150 mL) was added. The organic layer was washed with water (2 x 200 mL) then brine (2 x 200 mL). The organic layer was dried with sodium sulfate, filtered and evaporated in vacuo to give 48.63 g of the title compound as a brown oil which was taken directly on to the next reaction. ¹H NMR (CDCl₃) δ 5.05 (s, 2H); 7.1-7.4 (m, 16H); 8.20 (d, 1H); 8.58 (d, 1H)

### Example 33:

### 3-triphenylmethylthio-2-(N-t-butoxycarbonylaminoethylthiomethyl)pyridine

Into a 3 L round bottom flask equipped with a mechanical stirrer, thermometer and nitrogen purge was charged 3-triphenylmethylthio-2-hydroxymethylpyridine (40.0 g, 0.104 mol, 1eq.) followed by DMF (1.2 L). The solution was cooled to 0 °C and a pre-formed solution of thionyl chloride (12.4 g, 0.104 mol, 1.0 eq.) in DMF (200 mL) was charged dropwise over approximately 5 minutes. To this orange coloured solution was charged BOC-cysteamine (18.4 g, 0.104 mol, 1 eq.) followed by finely powdered potassium carbonate (43.1 g, 0.104 mol, 1 eq) and the resulting suspension was vigorously stirred for two hours until TLC analysis showed reaction completion (1:1 EtOAc/hexanes). The solution was poured into a 3 L of a 4:1 mixture of ice water/4:1 ethyl acetate-hexane. The aqueous was separated and extracted with an additional 200 mL of 4:1 ethyl acetate-hexane. The organics were combined and washed with water (2 x 200 mL) then brine (2 x 200 mL). The organic layer was dried with sodium sulfate, filtered and the solvent was removed in vacuo to give 54 grams of a brown oil. This oil was purified by column chromatography on silica gel to give 41 grams of the title compound as a light yellow oil. ¹H NMR (CDCl₃) δ 1.41 (s, 9H); 2.77 (t, 2H); 3.42 (t, 3H); 4.11 (s, 2H); 7.05-7.45 (m, 16H); 8.22 (d, 1H); 8.65 (d, 1H)

### Example 34:

### 3-mercapto-2-(aminoethylthiomethyl)pyridine trifluoroacetate

Into a 1 L round bottom flask equipped with a mechanical stirrer, thermometer and nitrogen purge was charged 3-triphenylmethylthio-2-(t-butoxycarbonylaminoethylthiomethyl)pyridine (40.0 g, 0.074 mol, 1 eq) followed by dichloromethane (200 mL). The light yellow solution was cooled to 0 °C and triethylsilane (60 mL) was added followed by dropwise addition of trifluoroacetic acid (200 mL) over approximately five minutes. The cold bath was removed and the solution was allowed to warm to room temperature (over 30 minutes) and was then allowed to stir at 20 °C for 30 minutes. The solvent was removed by rotary evaporation to give 27.75 g of the title compound as an orange waxy solid. ¹H NMR (D₂O) δ 2.88 (t, 2H); 3.28 (t, 3H); 4.18 (s, 2H); 7.45 (dd, 1H); 8.22 (d, 1H); 8.55 (d, 1H).

### Example 35:

### Bis(2-aminoethylthiomethylpyrid-3-yl) disulfide

Into a 1 L round bottom flask equipped with magnetic stirrer and thermometer was placed a 18 gauge stainless steel needle subsurface (to bubble in house air). To this apparatus was charged 3-mercapto-2-(aminoethylthiomethyl)pyridine trifluoroacetate (25 g, 0.125 mol, 1 eq) followed by methanol (300 mL) and the solution was cooled to 0 °C. Concentrated ammonium hydroxide was added until a pH of 8 was reached. Solid ferric chloride (2.03 g, 0.0125 mol, 0.1 eq.) was added and air was vigorously bubbled in over a period of six hours during which time the black suspension of iron chloride became a light brown chunky solid. Mass spectral analysis confirmed the conversion to the di-sulfide. The methanolic solution was filtered over a small pad of celite to give a green colored filtrate. The methanol was removed in vacuo to give a brownish-green waxy solid. Removal of water by coevaporation with toluene (4 x 50 mL) gave 23.1 g of the title compound as a green waxy solid. ¹H NMR (D₂O) δ 2.88 (t, 2H); 3.28 (t, 3H); 4.18 (s, 2H); 7.45 (dd, 1H); 8.22 (d, 1H); 8.55 (d, 1H). Mass spectrum: M+1 peak = 399

### Example 36:

### Bis(2-N-tert-butoxycarboaylaminoethylthiomethylpyrid-3-yl) disulfide

Into a 1 L round bottom flask equipped with mechanical stirrer was charged XII (15 g, 0.038 mol, 1 eq.) followed by 150 mL of 5:1 water/dioxane. To this homogenous solution was charged finely powdered potassium carbonate (10.5 g, 0.076 mol, 2.0 eq.) and BOC-anhydride (9.2 g, 0.042 mol, 1.1 eq.). The solution was allowed to stir at 20 °C for three hours until reaction was determined to be complete by TLC analysis (1:1 hexane/EtOAc). The dioxane was removed by rotary evaporation at which time a white powder began to precipitate out of solution. The solution was allowed to cool to room temperature and the white solid was collected by vacuum filtration and the solid was washed twice with cold water to give 17.88 g of the title compound. ¹H NMR (CDCl₃) δ 1.55 (s, 9H); 2.80 (t, 2H); 3.42 (t, 2H); 4.12 (s, 2H); 7.30 (dd, 1H); 8.02 (d, 1H); 8.48 (d, 1H)

### Example 37:

### (7R)-7-[(Z)-2-(5-amino-1,2,4-thiadiazol-3-yl)-2-(triphenylmethoxyimino)acetamido]-3-[2-(N-tert-butoxycarbonylaminoethylthiomethyl)pyrid-3-ylthio]-3-cephem-4-carboxylate, t-butyl ester

Into a 250 mL round bottom flask equipped with magnetic stirrer and nitrogen purge was charged (7R)-7-[(Z)-2-(5-amino-1,2,4-thiadiazol-3-yl)-2-(triphenylmethoxyimino)acetamido]-3-chloro-3-cephem-4-carboxylate, t-butyl ester (5 g, 0.007 mol, 1 eq.) followed by DMF (80 mL) and water (0.8 mL) and to the resulting clear yellow solution was charged bis(2-N-tert-butoxycarbonylaminoethylthiomethylpyrid-3-yl) disulfide (3.29 g, 0.0035 mol., 0.5 eq) followed by triphenylphosphine (1.83 g, 0.007 mol, 1.0 eq). The yellow solution was allowed to stir at 20 °C for 3 hours. The reaction solution was poured into ice/water and extracted with 3:1 ethyl acetate-hexane (3 x 100 mL). The organics were combined and washed with water (2 x 100 mL) and then brine (2 x 50 mL). The organic layer was dried with sodium sulfate, filtered and the solvent removed in vacuo to give 6.55 g of the title compound as a light yellow foam. ¹H NMR (CDCI,) δ 1.42 (s, 18H); 1.61 (s, 9H); 2.87 (m, 3H); 2.95 (dd, 1H); 3.20 (d, 1H); 3.53 (d, 1H); 3.60 (t, 2H); 4.09 (s, 2H); 4.55 (bs, 1H); 5.20 (d, 1H); 5.80 (bs, 1H); 6.17 (q, 1H); 6.99 (bs, 1H); 7.2-7.8 (m, 17H), 8.60 (d, 1H)

### Example 38:

### (7R)-7-[(Z)-2-(5-amino-1,2,4-thiadiazol-3-yl)-2-(hydroxyimino)-3-[2-aminoethylthiomethyl)pyrid-3-ylthio]-3-cephem-4-carboxylic acid bis-TFA salt

Into a 250 mL round bottom flask equipped with magnetic stirrer and nitrogen purge was charged (7R)-7-[(Z)-2-(5-amino-1,2,4-thiadiazol-3-yl)-2-(triphenylmethoxyimino)acetamido]-3-[2-(N-*tert*-butoxycarbonylaminoethylthiomethyl)pyrid-3-ylthio]-3-cephem-4-carboxylate, t-butyl ester (3.5 g, 0.003 mol, 1.0 eq.) followed by dichloromethane (20 mL). To the clear light yellow solution was charged triethylsilane(10.5 mL) followed by trifluroacetic acid (35 mL) dropwise over 2 minutes. The resulting yellow solution was allowed to stir at 20 °C for 8 hours until complete de-protection was confirmed by HPLC analysis. The acidic solution was poured into rapidly stirring 0 °C diethyl ether (300 mL) and the resulting white solid was collected by vacuum filtration and the filter cake was washed with additional diethyl ether (2 x 20 mL). The material was collected and was allowed to dry under high vacuum at 20 °C for one hour to give the title compound as an off-white solid. ¹H NMR (CD₃OD) δ 2.80 (t, 2H); 3.02 (m, 2H); 3.61 (m, 3H); 4.17 (s, 2H); 4.28 (q, 1H); 5.38 (d, 1H); 6.05 (d, 1H); 7.51 (q, 1H); 8.05 (d, 1H); 8.57 (d, 1H)

### Example 39:

### (7R)-7-[(Z)-2-(5-amino-1,2,4-thiadiazol-3-yl)-2-(hydroxyimino)-3-[2-aminoethylthiomethyl)pyrid-3-ylthio]-3-cephem-4-carboxylate zwitterion

A 1 inch diameter glass column was packed with 30 grams of HP-20 resin and the acidic solution of (7R)-7-[(Z)-2-(5-amino-1,2,4-thiadiazol-3-yl)-2-(hydroxyimino)-3-[2-aminoethylthiomethyl)pyrid-3-ylthio]-3-cephem-4-carboxylic acid bis-TFA salt (1.2 g, 0.0013 mol) in water (20 mL) was charged on top of the resin and distilled water was eluted through until the pH of the eluent reached 5-6 (from < 2). The solvent system was changed immediately to 4:1 water/acetonitrile and the zwitterionic compound was eluted in approximately 100 mL of eluent. The acetonitrile was removed in vacuo at < 40 °C and the remaining water was lyophilized to give 0.79 g of the title compound as a white fluffy solid.

### Example 40: Synthesis of Prodrugs:

### Bis [2-[2-(bis-N_{α}, N_{δ}-t-butoxycarbonyl)-N-(L)-ornithylaminoethylthiomethyl]-pyrid-3-yl] disulfide

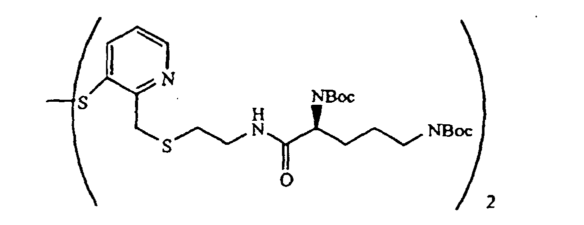

To DMF (7 mL) was added N-Boc-Orn-(Boc)-OH (0.504 g, 1.5 mmol), bis (2-(2-aminoethylthiomethyl)pyrid-3-yl) disulfide tetra HCl salt (0.389 g, 0.71 mmol), diisopropylethyl amine (0.641 g, 5.0 mmol, 0.86 mL), and PyBOP (0.780 g, 1.5 mmol), respectively. The reaction was stirred at room temperature for 2 hr, and then diluted with ethyl acetate (50 mL). The organics were washed with water (2 x 20 mL), brine (2 x 20 mL), separated dried over sodium sulfate, filtered and concentrated in vacuo. Purification of the title compound was achieved via SiO₂ column chromatography eluting with ethyl acetate affording 0.412 g (56%) of a white foamy solid.

### Compound 2-A

### (7R)-7-[(Z)-2-(5-amino-1,2,4-thiadiazol-3-yl)-2-(hydroxyimino)acetamido]-3-{2-[2-N-(L)-ornithylaminoethylthiomethyl]pyrid-3-ylthio}-3-cephem-4-carboxylic acid, methanesulfonic acid salt

To a predried 25 mL round-bottomed flask was added DMF (1.5 mL), (7R)-7-[(Z)-2-(5-amino-1,2,4-thiadiazol-3-yl)-2-(triphenylmethoxyimino)acetamido]-3-methanesulfonyl-3-cephem-4-diphenylmethyl carboxylate (0.69, 0.8 mmol, bis [2-[2-(bis-N_{α}, N_{δ}-t-butoxycarbonyl)-N-(L)-ornithylaminoethylthiomethyl]pyrid-3-yl] disulfide (0.41 g, 0.4 mmol), water (0.036 mL), and triphenylphosphine (0.209 g, 0.8 mmol), respectively. The reaction mixture was stirred for 2 hr and then diluted with ethyl acetate (50 mL). The organics were washed with water (2 x 25 mL) and brine (2 x 25 mL). The organics were separated, dried over sodium sulfate, filtered, and concentrated in vacuo. Purification was achieved via SiO₂ column chromatography eluting with hexanes/ethyl acetate ramping from 1/1 to ethyl acetate (100%) affording 0.45 g (45%) of a white foamy solid. The white foamy solid was dissolved in methylene chloride/triethylsilane (2 mL; 1/1) and then cooled to 0 °C, then trifluoroacetic acid (5 mL) was added via syringe. The reaction was stirred for 30 min and then triturated with cold diethyl ether (30 mL) resulting in the precipitation of the desired TFA salt. The TFA salt was dissolved in water (5 mL) and placed atop a column of HP20 resin in water. The column was washed with water until an effluent pH of 6.5. The column was then eluted with acetonitrile/water (1/4; v/v). The UV active fractions were combined and tritiated with methanesulfonic acid to a pH = 3.0. The organics were removed in vacuo and the product was obtained via lyophilization affording 0.095 g as a white amorphous solid. ¹H NMR (D₂O) δ 2.00 (m, 2H), 2.18 (m, 2H), 3.00 (t, 2H, J = 7), 3.26 (t, 2H, J = 7), 3.51 (d, 1H, J = 18), 3.66 (m, 2H), 4.00 (d, 1H, J = 18), 4.24 (t, 1H, J = 7), 4.43 (s, 2H), 5.59 (d, 1H, J = 5), 6.13 (d, 1H, J = 5), 8.05 (dd, 1H, J = 7, 5), 8.51 (d, 1H, J = 8), 8.77 (d, 1H, J = 5).

The following compounds were synthesized in a similar manner as described above. The acylation of bis (2-(2-aminoethylthiomethyl)pyrid-3-yl) disulfide tetra HCl salt could also be performed utilizing activated esters (pentafluorophenol or N-hydroxysuccinimide) of the respective amino acids with similar results.

### Compound 2-B

### (7R)-7-[(Z)-2-(5-amino-1,2,4-thiadiazol-3-yl)-2-(hydroxyimino)acetamido]-3-{2-[2-N-(L)-prolylaminoethylthiomethyl]pyrid-3-ylthio)-3-cephem-4-carboxylate, methane sulfonic acid salt

¹H NMR (D₂O) δ 2.12 (m, 3H), 2.52 (m, 1H), 2.87 (m, 2H), 2.92 (s, 3H), 3.58 (d, AB, 1H, J = 17), 3.51 (m, 2H), 3.75 (d, AB, 1H, 17), 4.18 (m, 1H), 5.43 (d, 1H, J = 5), 6.01 (d, 1H, J = 5), 7.49 (dd, 1H, J = 7, 5), 8.45 (d, 1H, J = 5).

### Compound 2-C

### (7R)-7-[(Z)-2-(5-N-(L)-alanylamino-1,2,4-thiadiazol-3-yl)-2-(hydroxyimino)acetamido]-3-{2-[2-aminoethylthiomethyl]pyrid-3-ylthio}-3-cephem-4-carboxylate, trifluoroacetic acid salt

¹H NMR (CD₃OD) δ 1.74 (d, 3H, J = 7), 2.94 (t, 2H, J = 7), 3.38 (t, 2H, J = 7), 3.41 (d, I H, J = 17), 3.70 (d, 1H, J = 17), 4.18 (s, 2H), 4.41 (s, 2H), 5.33 (d, 1H, J = 5), 6.04 (d, 1H, J = 5), 7.45 (dd, 1H, J = 7, 5), 8.01 (d, 1H, J = 8), 8.53 (d, 1H, J = 5).

### Compound 2-D

### (7R)-7-[(Z)-2-(5-amino-1,2,4-thiadiazol-3-yl)-2-(hydroxyimino)acetamido]-3-{2-[2-N-(L,L)-alanylalanylaminoethylthiomethyl]pyrid-3-ylthio}-3-cephem-4-carboxylate, trifluoroacetic acid salt

¹H NMR (D₂O) δ 1.59 (d, 3H, J = 8), 1.75 (d, 3H, J = 8), 2.98 (t, 2H, J = 7), 3.54 (d, 1H, J = 17), 3.68 (m, 2H), 4.01 (d, 1H, J = 17), 4.30 (q, 1H, J = 8), 4.46 (s, 2H), 4.50 (q, 1H, J = 8), 5.59 (d, 1H, J = 5), 6.14 (d, 1H, J = 5), 8.09 (dd, 1H, J = 7, 5), 8.59 (d, 1H, J = 8), 8.77 (d, 1H, J = 5).

### Compound 2-E

### (7R)-7-[(Z)-2-(5-amino-1,2,4-thiadiazol-3-yl)-2-(hydroxyimino)acetamido]-3-{2-[2-N-glycylaminoethylthiomethyl]pyrid-3-ylthio}-3-cephem-4-carboxylate, methanesulfonic acid salt

¹H NMR (D₂O) δ 2.88 (t, 2H, J = 7), 2.92 (s, 3H), 3.41 (d, 1H, J = 16), 3.54 (m, 2H), 3.88 (d, 1H, J = 16), 3.91 (s, 2H), 4.30 (s, 2H), 5.48 (d, 1H, J = 5), 5.94 (d, 1H, J = 5), 7.89 (dd, 1H, J = 8, 5), 8.34 (d, 1H, J = 8), 8.59 (d, 1H, J = 5).

### Compound 2-F

### (7R)-7-[(Z)-2-(5-amino-1,2,4-thiadiazol-3-yl)-2-(hydroxyimino)acetamido]-3-{2-[2-N-(L)-aspartylaminoethylthiomethyl]pyrid-3-ylthio}-3-cephem-4-carboxylate, trifluoroacetic acid salt

¹H NMR (CD₃OD) δ 2.93 (m, 2H), 3.07 (dd, 1H, J = 24, 9), 3.17 (dd, 1H, J = 24, 5), 3.47 (d, 1H, J = 18), 3.58 (m, 2H), 3.76 (d, 1H, J = 18), 4.28 (s, 2H), 4.36 (m, 1H), 5.45 (d, 1H, J = 5), 6.11 (d, 1H, J = 5), 7.52 (dd, 1H, J = 7, 5), 8.22 (d, 1H, J = 8), 8.67 (d, 1H, J = 5).

### Compound 2-G

### (7R)-7-[(Z)-2-(5-amino-1,2,4-thiadiazol-3-yl)-2-(hydroxyimino)acetamido]-3-{2-[2-N-(L)-alanylaminoethylthiomethyl]pyrid-3-ylthio}-3-cephem-4-carboxylate, methanesulfonic acid salt

¹H NMR (CD₃OD) δ 1.69 (d, 3H, J = 8), 2.89 (s, 3H), 2.91 (t, 2H, J = 7), 3.43 (t, 2H, J = 17), 3.63 (t, 2H, J = 7), 3.73 (d, 1H, J = 17), 4.09 (q, 1H, J = 8), 4.25 (s, 2H), 5.44 (d, 1H, J = 5), 6.09 (d, 1H, J = 5), 7.54 (dd, 1H, J = 7, 5), 8.02 (d, 1H, J = 7), 8.62 (d, 1H, J = 5).

### Compound 2-H

### (7R)-7-[(Z)-2-(5-amino-1,2,4-thiadiazol-3-yl)-2-(hydroxyimino)acetamido]-3-{2-[2-N-(L)-(N_{α}-methyl)alanylaminoethylthiomethyl)pyrid-3-ylthio}-3-cephem-4-carboxylate, methanesulfonic acid salt

¹H NMR (CD₃OD) δ 1.70 (d, 3H, J = 10), 2.87 (s, 3H), 2.88 (s, 3H), 2.95 (t, 2H, J = 7), 3.53 (d, 1H, J = 18), 3.67 (t, 2H, J = 7), 3.83 (d, 1H, J = 18), 4.15 (q, 1H, J = 8), 4.34 (s, 2H), 5.48 (d, 1H, J = 5), 6.12 (d, 1H, J = 5), 7.58 (dd, 1H, J = 7, 5), 8.36 (d, 1H, J = 7), 8.73 (d, 1H, J = 5).

### Compound 2-I

### (7R)-7-[(Z)-2-(5-amino-1,2,4-thiadiazol-3-yl)-2-(hydroxyimino)acetamido}-3-{2-[2-N-(L)-histidylaminoethylthiomethyl]pyrid-3-ylthio]-3-cephem-4-carboxylate, methanesulfonic acid salt

¹H NMR (CD₃OD) δ 2.88 (t, 2H, J = 7), 2.90 (s, 3H), 3.50 (d, 1H, J = 17), 3.57 (t, 2H, J = 6), 3.76 (d, 1H, J = 17), 4.26 (s, 2H). 4.38 (t, 2H, J = 7), 5.46 (d, 1H, J = 5), 6.07 (d, 1H, J = 5), 7.65 (m, 1H), 8.21 (d, 1H, J = 6), 8.66 (m, 1H), 9.08 (s, 1H).

### Compound 2-J

### (7R)-7-[(Z)-2-(5-amino-1,2,4-thiadiazol-3-yl)-2-(hydroxyimino)acetamido]-3-{2-[2-N-(L)-valylaminoethylthiomethyl]pyrid-3-ylthio}-3-cephem-4-carboxylate, methanesulfonic acid salt

¹H NMR (D₂O) δ 1.12 (d, 3H, J = 6), 1.13 (d, 3H, J = 6), 2.30 (septet, 1H, J = 6), 2.91 (t, 2H, J = 6), 2.92 (s, 3H), 3.43 (d, AB, J = 18), 3.54 (m, 1H), 3.67 (m, 1H), 3.88 (d, 1H, J = 6), 3.91 (d, AB, J = 18), 4.33 (s, 2H), 5.50 (d, 1H, J = 5), 6.05 (d, 1H, J = 5), 7.93 (dd, 1H, J = 7, 5), 8.40 (d, 1H, J = 7), 8.64 (d, 1H, J = 5).

### Compound 2-K

### (7R)-7-[(Z)-2-(5-amino-1,2,4-thiadiazol-3-yl)-2-(hydroxyimino)acetamido]-3-{2-[2-N-(L)-asparagylaminoethylthiomethyl]pyrid-3-ylthio}-3-cephem-4-carboxylate, trifluoroacetic acid salt

¹H NMR (D₂O) δ 2.74-2.79 (m, 4H), 2.93 (t, 2H, J = 6), 3.27 (dd, 1H, J =18, 13), 3.32-3.50 (m, 3H), 3.75 (d, 1H, J = 18), 4.17 (s, 2H), 4.29 (t, 1H, J = 14), 5.35 (d, 2H, J = 5), 5.90 (d, 1H, J = 5), 7.75 (dd, 1H, J = 8,5), 8.22 (d, 1H, J = 8), 8.45 (d, 1H, J= 5).

### Compound 2-L

### (7R)-7-[(Z)-2-(5-amino-1,2,4-thiadiazol-3-yl)-2-(hydroxyimino)acetamido]-3-{2-[2-N-(L)-lysylaminoethylthiomethyl]pyrid-3-ylthio}-3-cephem-4-carboxylate, methanesulfonic acid salt

¹H NMR (CD₃OD) δ 1.73 (m, 1H), 1.92 (m, 1H), 2.09 (m, 1H), 2.91 (s, 3H), 2.98 (m, 3H), 3.18 (t, 1H, J = 6), 3.49 (d, AB, 1H, J = 17), 3.65 (m, 2H), 3.75 (d, AB, 1H, J = 17), 4.09 (t, 1H, J = 6), 4.31 (s, 2H), 5.48 (d, 1H, J = 5), 6.09 (d, 1H, J = 5), 7.61 (dd, 1H, J = 7, 5), 8.19 (d, 1H, J = 7), 8.69 (d, 1H, J = 5).

### Compound 2-M

### (7R)-7-[(Z)-2-(5-amino-1,2,4-thiadiazol-3-yl)-2-(hydroxyimino)acetamido]-3-{2-[2-N-(L)-serylaminoethylthiomethyl]pyrid-3-ylthio}-3-cephem-4-carboxylate, methanesulfonic acid salt

¹H NMR (D₂O) δ 2.85 (m, 3H), 3.35 (d, 1H, J = 17), 3.45 (t, 2H, J = 6), 3.84 (d, 1H, J = 17), 3.99 (ddd, 2H, J = 24, 12, 5), 4.17 (t, 2H, J = 5), 4.26 (s, 2H), 5.42 (d, 1H, J = 5), 5.97 (d, 1H, J = 5), 7.80 (dd, 1H, J = 8, 5) 8.24 (d, 1H, J = 5), 8.49 (d, 1H, J = 5).

### Compound 2-N

### (7R)-7-[(Z)-2-(5-amino-1,2,4-thiadiazol-3-yl)-2-(hydroxyimino)acetamido]-3-{2-[2-N-(L)-glutaminylaminoethylthiomethyl]pyrid-3-ylthio}-3-cephem-4-carboxylate, methanesulfonic acid salt

¹H NMR (D₂O) δ 2.19 (q, 2H, J = 7), 2.50 (t, 2H, J = 8), 2.85 (m, 5H), 3.34 (d, 1H, J = 18), 3.51 (m, 2H), 3.84 (d, 1H, J = 18), 4.08 (t, 1H, J = 7), 4.28 (s, 2H), 5.43 (d, 1H, J = 5), 5.97 (d, 1H, J = 5), 7.90 (dd, 1H, J = 8), 8.37 (d, 1H, J = 8), 8.58 (d, 1H, J = 8).

### Bis-{2-[2-(5-methyl-1,3-dioxolan-4-en-2-on-4-yl)methoxycarbonyl)aminoethylthiomethyl]pyrid-3-yl} disulfide

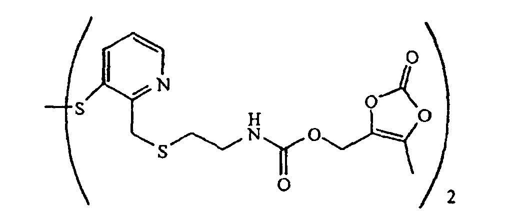

To a predried 25 mL round-bottomed flask charged with DMF (15 mL) was added bis (2-(2-aminoethylthiomethyl)pyrid-3-yl) disulfide tetra HCl salt (1.0 g, 3.39 mmol) and (5-methyl-2-oxo-1,3-dioxolan-4-enyl)methyl p-nitrophenyl carbonate (0.922 g, 1.69 mmol). With stirring, triethylamine (3.6 mL, 21 mmol) was added dropwise via syringe. Following complete consumption of the starting materials, as monitored by thin layer chromatography (ethyl acetate), the reaction was diluted with 20 mL of water, and extracted with ethyl acetate (2 x 20 mL). The organic layer was separated, washed with 1 N HCl (1 x 10 mL), 2% sodium bicarbonate (1 x 10 mL), water (1 x 10 mL), and brine (1 x 10 mL), respectively. The organics were separated, dried over sodium sulfate, filtered, and concentrated in vacuo. Purification of the title compound was achieved via SiO₂ column chromatography eluting with ethyl acetate (100%) affording 0.252 g (10%) as a yellow solid.

### Compound 2-O

### (7R)-7-[(Z)-2-(5-amino-1,2,4-thiadiazol-3-yl)-2-(hydroxyimino)acetamido]-3-{2-[2-(5-methyl-1,3-dioxolan-4-en-2-on-4-yl)methoxycarbonyl)aminoethylthiomethyl]pyrid-3-ylthio}-3-cephem-4-carboxylate, sodium salt

To a predried 25 mL round-bottomed flask charged with DMF (3 mL) were added (7R)-7-[(Z)-2-(5-amino-1,2,4-thiadiazol-3-yl)-2-(triphenylmethoxyimino)acetamido]-3-methanesulfonyl-3-cephem-4-diphenylmethyl carboxylate (0.200 g, 0.23 mmol), bis-{2-[2-(5-methyl-1,3-dioxolan-4-en-2-on-4-yl)methoxycarbonyl)aminoethylthiomethyl]pyrid-3-yl} disulfide (0.080 g, 0.12 mmol), water (0.010 mL), and triphenylphosphine (0.060 g, 0.24 mmol), respectively. Upon stirring for 2.5 hr, the reaction was diluted with ethyl acetate (10 mL) and the organics were washed with water (1 x 10 mL) and brine (1 x 10 mL). The organics were separated and dried over anhydrous sodium sulfate, filtered and concentrated in vacuo. Purification was achieved via SiO₂ column chromatography eluting with ethyl acetate (100%) affording 0.212 g (82%) as a yellow foamy solid. The yellow solid was dissolved in methylene chloride/triethylsilane (2 mL; 1/1, v/v) and cooled to 0 °C. To the reaction flask was added dichloroacetic acid (4.2 mL) and the mixture was stirred for 1 hr. The reaction was triturated with diisopropyl ether (15 mL) resulting in the precipitation of the desired product. The solids were collected, dissolved in water (5 mL), and tritiated with 0.5 M sodium bicarbonate until the solution became homogenous. The solution was loaded atop a column of HP20 resin packed with water and the column was washed with water until the pH of the effluent became 7. The column was then eluted with acetonitrile/water (7/1). The UV active fractions were combined, reduced in vacuo to remove the acetonitrile, and the title compound was isolated via lyophilization affording 0.070 g (50%) of the title compound as a white amorphous solid. ¹H NMR (D₂O) δ 2.21 (s, 3H), 2.90 (bt, 2H, J = 7), 3.47 (m, 2H), 3.80 (d, 1H, J = 18), 4.20 (s, 2H), 4.95 (s, 2H), 5.56 (d, 1H, J = 5), 6.02 (d, 1H, J = 5), 7.53 (dd, 1H, J = 7, 5), 8.03 (d, 1H, J = 7), 8.51 (d, 1H, J = 5).

The following compound was converted to its respective sodium salt in a similar manner as described above.

### Compound 2-P

### (7R)-7-[(Z)-2-(5-amino-1,2,4-thiadiazol-3-yl)-2-(hydroxyimino)acetamido]-3-{2-(2-N-(L)-pyroglutaminylaminoethylthiomethyl]pyrid-3-ylthio}-3-cephem-4-carboxylate, sodium salt

¹H NMR (CD₃OD) δ 2.50 (m, 4H), 2.82 (t, 2H, J = 7), 3.30 (s, 3H), 3.35 (d, 1H, J = 17), 3.52 (m, 2H), 3.71 (d, 1H, J = 17), 4.17 (s, 2H), 4.35 (m, I H), 5.38 (d, 1H, J = 5), 7.41 (dd, 1H, J = 7, 5), 8.42 (m, 1H).

### Example 41:

### 2-chloromethyl-3-triphenylmethylthiopyridine hydrochloride

A thick suspension of 2-hydroxymethyl-3-triphenylmethylthiopyridine (90.3g, 235 mmol) in *N*,*N*-dimethylformamide (270 mL) in a flask equipped with efficient mechanical stirring was chilled to 2 °C. Thionyl chloride (25.8 mL, 354 mmol) was added in 5 mL portions over the period of 1 h while maintaining a temperature below 10 °C. After completing the addition the reaction mixture was allowed to reach room temperature and was stirred for 30 min. The precipitate was filtered, washed with ethyl acetate (500 mL) and dried to give 86.7g (84%) 2-chloromethyl-3-triphenylmethylthiopyridine hydrochloride as colorless crystals.

### Example 42:

### 2-(2-aminoethylthiomethyl)-3-triphenylmethylthiopyridine

To a stirred solution of sodium methoxide (0.787 mol) in methanol (425 mL) were added at room temperature under nitrogen 2-chloromethyl-3-triphenylmethylthiopyridine hydrochloride (86 g, 0.196 mol) and cysteamine hydrochloride (33.0 g, 0.290 mol). After 2.5 h, the reaction mixture was partitioned between ethyl acetate (500 mL), water (500 mL) and brine (250 mL). The organic layer was separated and the aqueous layer was extracted with ethyl acetate (250 mL). The combined organic extracts were washed with brine (3 X 500 mL) and dried over anhydrous sodium sulfate. Following filtration, evaporation of the filtrate under reduced pressure produced 2-(2-aminoethylthiomethyl)-3-triphenylmethylthiopyridine as a yellowish oil (92.7 g, after correcting for residual solvent by NMR, 87.0 g, quantitative yield).

### Example 43:

### 2-(2-aminoethylthiomethyl)3-triphenylmethylthiopyridine

Into a 20 mL flask was charged 2-(2-*N-tert*-butoxycarbonylaminoethylthiomethyl)3-triphenylmethylthiopyridine (0.20 g, 0.37 mmol, 1.0 eq.) and 4.5 M HCl (dry) in methanol (2.0 mL, 9.0 mmol, 24 eq). The solution was placed in the refrigerator (4 °C) for 2 h. At the end of two hours, the reaction solution was pipetted into 20 mL water with ice chips and a suspension immediately formed. The bis-HCl salt was next neutralized with saturated sodium bicarbonate (1 mL) and extracted with ethyl acetate (15 mL). The phases were separated and the aqueous phase was extracted with additional ethyl acetate (10 mL). The combined organic extracts were dried with sodium sulfate, filtered, and the solvent removed in vacuo to give a white powder (80 mg, 50%) containing predominantly 3-triphenylmethylthio-2-(2-aminoethylthiomethyl)pyridine (HPLC, NMR). ¹H NMR (4:1 CDCl₃:CD₃OD) δ 2.65 ppm (t, 2H), 3.00 (t, 2H), 3.52 (s, 2H), 6.82 (d, 1H), 7.00-7.40 (m, 15H), 8.20 (d, 1H).

### Example 44:

### 2-[2-L-(N-tert-butoxycarbonyl-4-tert-butylaspartyl)aminoethylthiomethyl]-3-triphenylmethylthiopyridine

To a stirred solution of 2-(2-aminoethylthiomethyl)3-triphenylmethylthiopyridine (57.1 g, 129 mmol) in ethyl acetate (285 mL) was added a solution of *N*-(*tert*-butoxycarbonyl)-4-*tert*-butyl-L-aspartic acid pentafluorophenol ester (52.8 g, 116 mmol) and the mixture was stirred at room temperature for 4 h. The reaction mixture was washed with an aqueous solution of sodium carbonate (0.9 M, 1 L). The organic layer was separated, washed twice with brine (500 mL) and dried over anhydrous sodium sulfate. Hexane (400 mL) was added with stirring until a slight cloudiness persisted, and then ethyl acetate was added to give a clear solution. The resulting solution was filtered through a plug of silica gel (300 mL; 2 inch layer) followed by washing with ethyl acetate -hexane solution (1 L). The combined filtrates were concentrated to an oily residue and purified by chromatography over silica gel using a step-gradient elution with ethyl acetate-hexane solution (1:2, then 1:1 and finally, 2:1). The filtrates were evaporated to an oily residue of 2-[2-L-(*N-tert*-butoxycarbonyl-4-*tert*-butylaspartyl)aminoethylthiomethyl]-3-triphenylmethylthiopyridine (65 g, 75%) which was used immediately in the next step.

### Example 45:

### 2-(2-L-aspartylaminoethylthiomethyl)-3-mercaptopyridine dihydrochloride

To a stirred solution of 2-[2-L-(*N-tert*-butoxycarbonyl-4-*tert*-butylaspartyl)aminoethylthiomethyl]-3-triphenylmethylthiopyridine (65 g, 87 mmol) in dichloromethane (130 mL) under nitrogen was added triethylsilane (130 mL, 810 mmol) and the solution was cooled to 0 °C. Trifluoroacetic acid (650 mL) was added and the mixture was allowed to return to room temperature and stirred for 3.5 h. The mixture was concentrated under reduced pressure to a syrupy residue (~30 °C bath) which was then dissolved in ethyl acetate (300 mL). To this solution was added with stirring a solution of dry HCl in ethyl acetate (150 mL, 1.75 M, 262 mmol) followed by addition of more ethyl acetate (300 mL) to facilitate stirring. After stirring for an additional 30 min solids were collected by filtration under nitrogen, washed with ethyl acetate and dried in vacuo to yield 2-(2-L-aspartylaminoethylthiomethyl)-3-mercaptopyridine dihydrochloride as a white powder (33.7 g, 99%).

### Example 46:

### Bis(2-[2-L-(N-tert-butoxycarbonyl-4-tert-butylaspartyl)aminoethylthiomethyl]pyrid-3-yl) disulfide

A flask was charged with bis(2-aminoethylthiomethyl)pyrid-3-yl) disulfide (80 mg, 0.20 mmol, 1.0 eq.), aqueous 1M sodium bicarbonate (2.0 mL, 2.0 mmol, 10 eq.), and water (5 mL). To this was added *N*-(*tert*-butoxycarbonyl)-4-*tert*-butyl-L-aspartic acid pentafluorophenol ester (0.18 g, 0.40 mmol, 2.0 eq.) dissolved in ethyl acetate (2.5 mL), followed by sodium chloride (50 mg, 10% solution). The biphasic mixture was stirred vigorously at room temperature and after 24 h. The layers were separated and the aqueous phase was washed with additional ethyl acetate (2.5 mL). The organic extracts were combined and washed with brine (2 x 5 mL), dried with sodium sulfate, filtered and the solvent was removed in vacuo. The resulting oily product was purified by radial chromatography first with 1:5 (v/v) hexane-ethyl acetate to remove less polar materials, followed by neat ethyl acetate to afford pure bis(2-[2-L-(*N-tert*-butoxycarbonyl-4-*tert*-butylaspartyl)aminoethylthiomethyl]pyrid-3-yl) disulfide (100 mg, 54% yield). ¹H NMR (CDCl₃) δ 1.50 ppm (s, 18H), 1.55 (s, 18H), 2.72 (m, 6H), 2.92 (d, 2H), 2.98 (q, 2H), 3.55 (q, 4H), 4.55 (d, 2H), 5.80 (d, 2H), 7.35 (m, 4H), 8.10 (d, 2H), 8.50 (d, 2H).

### Example 47:

### 2-[2-L-(N-tert-butoxycarbonyl-4-tert-butylaspartyl)aminoethylthiomethyl]-3-mercaptopyridine

To a stirred solution of bis(2-[2-L-(*N-tert*-butoxycarbonyl-4-*tert*-butylaspartyl)aminoethylthiomethyl]pyrid-3-yl) disulfide (19.3 g, 41.0 mmol) in *N*,*N*-dimethylformamide (170 mL) was added water (1.7 mL, 94 mmol) followed by triphenylphosphine (11.8 g, 45 mmol). After 4 h at room temperature, addtional triphenylphosphine (5.38 g, 20.5 mmol) was added and the reaction was continued at room temperature overnight. The reaction mixture was partitioned between water (500 mL) and 3:1 (v/v) ethyl acetate-hexane. The organic extract was washed with brine (3 X 250 mL), dried over sodium sulfate and concentrated uder reduced pressure to produce 2-[2-L-(*N*-*tert*-butoxycarbonyl-4-*tert*-butylaspartyl)aminoethylthiomethyl]-3-mercaptopyridine, which was used immediately in the next reaction.

### Example 48:

### 2-[2-L-(N-tert-butoxycarbonyl-4-tert-butylaspartyl)aminoethylthiomethyl]-3-mercaptopyridine

A flask equipped with a mechanical stirrer was charged with 2-(2-aminoethylthiomethyl)-3-mercaptopyridine dihydrochloride (2.1 g, 7.7 mmol, 1.07 eq.), *N*-(*tert*-butoxycarbonyl)-4-*tert*-butyl-L-aspartic acid pentafluorophenol ester (3.3 g, 7.2 mmol, 1.0 eq.), triethylamine (2.1 mL, 15 mmol, 2.1 eq.) and *N,N*-dimethylformamide (55 mL). The solution was allowed to stir at 20 °C for 3 h. The solution was diluted to 200 mL with water and washed with 3:1 (v/v) ethyl acetate-hexanes (2 x 50 mL). The combined organic extracts were washed with brine (3 x 100 mL), dried with sodium sulfate, filtered and the solvent removed in vacuo to give 2-[2-L-(*N-tert*-butoxycarbonyl-4-*tert*-butylaspartyl)aminoethylthiomethyl]-3-mercaptopyridine which was used in the next step without further purification.

### Example 49:

### 2-(2-L-aspartylaminoethylthiomethyl)-3-mercaptopyridine dihydrochloride

A flask equipped with a magnetic stirrer was charged with 2-[2-L-(*N*-*tert*-butoxycarbonyl-4-*tert*-butylaspartyl)aminoethylthiomethyl]-3-mercaptopyridine (0.24 g, 0.51 mmol, 1.0 eq.), followed by CH₂Cl₂ (1.2 mL) and triethylsilane (1.2 mL). Dropwise, trifluoroacetic acid (4.8 mL) was added to the solution, which was allowed to stir at 20 °C for 3 h. The solution was concentrated in vacuo, and the residue was dissolved in ethyl acetate (25 mL). Into this solution was bubbled anhydrous HCl. The white solid, which precipitated almost immediately, was carefully filtered and washed with ethyl acetate, white avoiding exposure to moisture, and dried in vacuo to yield **0.18** g (90%) 2-(2-L-aspartylaminoethylthiomethyl)-3-mercaptopyridine dihydrochloride as a white powder.

### Example 50:

### Sodium (Z)-2-(5-amino-1,2,4-thiadiazol-3-yl)-2-(triphenylmethyl)oxyiminoacetate

A suspension of methyl (Z)-2-(5-ethoxycarbonylamino-1,2,4-thiadiazol-3-yl)-2-(triphenylmethyl)oxyiminoacetate (7.3 g, 14.1 mmol) was heated at 80 °C under reflux in 55 mL of 2:1 (v/v) aqueous NaOH (2.5 M) and ethanol. After 24 h the reaction mixture was allowed to cool to room temperature and the precipitate was collected by filtration, washed with water (washes were not combined with the original filtrate) and dried in vacuo to produce crystalline sodium (Z)-2-(5-amino-1,2,4-thiadiazol-3-yl)-2-(triphenylmethyl)oxyiminoacetate (3.08 g, 48% yield). The filtrate was resubjected to an additional 24h of heating at 80°C and the above described workup procedure was repeated to produce a second crop of sodium (Z)-2-(5-amino-1,2,4-thiadiazol-3-yl)-2-(triphenylmethyl)oxyiminoacetate (1.43 g, 22% yield). A third crop of sodium (Z)-2-(5-amino-1,2,4-thiadiazol-3-yl)-2-(triphenylmethyl)oxyiminoacetate (0.49 g, 8% yield) was obtained after subjecting the filtrate to another 24 h period of heating at 80 °C.

### Example 51:

### (7R)-7-amino-3-chloroceph-3-em-4-carboxylate, tert-butyl ester

To a suspension of 7-amino-3-chloroceph-3-em-4-carboxylate (23.7 g, 101.2 mmol) in *t*-butyl acetate (510 mL), under nitrogen, was added BF, etherate (80 mL) and the mixture was stirred vigorously at room temperature until complete dissolution was observed. After 2.5 h the reaction mixture was poured into stirred ice water (1 L) and the organic layer was discarded. The aqueous layer was washed with 1:1 (v/v) ethyl acetate-hexane (200 mL) and separated. To this aqueous solution with ice cooling and stirring was added ethyl acetate (500 mL) followed by portionwise addition of sodium carbonate (216 g) until a pH of 8-8.5 was reached. The organic layer was separated and the aqueous layer was extracted with ethyl acetate (100 mL). The combined organic extracts were dried over anhydrous sodium sulfate and concentrated under reduced pressure to give an oily residue (28 g) which solidified after removing the flask from the bath. To this residue was added toluene (100 mL; partial dissolution) followed by hexane (100 mL). After 10 min of additional stirring crystalline (7R)-7-amino-3-chloroceph-3-em-4-carboxylate, *tert*-butyl ester was filtered, washed with hexane and dried under reduced pressure (15.4 g; 52%).

### Example 52:

### (7R)-7-[(Z)-2-(5-amino-1,2,4-thiadiazol-3-yl)-2-(triphenylmethyloxyimino)acetamido-3-chloroceph-3-em-4-carboxylate, tert-butyl ester

To a stirred suspension of sodium (Z)-2-(5-amino-1,2,4-thiadiazol-3-yl)-2-triphenylmethyloxyiminoacetate (14.38 g, 31.7 mmol) in dry THF (170 g) was added diphenyl chlorophosphate (9.9 mL, 47.8 mmol, 1.5 eq.). After a few minutes of stirring most of the starting material dissolved. Stirring was continued for 1 h at ambient temperature and *tert*-butyl (7R)-7-amino-3-chloroceph-3-em-4-carboxylate (9.19 g, 31.7 mmol, 1.0 eq.) was added followed by addition of 2,6-lutidine (3.7 mL, 31.7 mmol, 1.0 eq.). After 3 h at room temperature the reaction mixture was partitioned between ethyl acetate (200 mL) and 0.5 M HCl (100 mL). Following phase separation, the organic layer was washed again with 0.5 M HCl (100 mL), then with 0.5 M NaHCO₃ solution (2 X 100 mL). The organic phase was dried over anhydrous sodium sulfate and concentrated to a volume of about 60 mL. To this solution was added 1:2 ethyl acetate-hexane (80 mL) and after seeding, the product, (7R)-7-[(Z)-2-(5-amino-1,2,4-thiadiazol-3-yl)-2-(triphenylmethyloxyimino)acetamido-3-chloroceph-3-em-4-carboxylate, *tert*-butyl ester was allowed to crystallize overnight. Solids were collected by filtration and dried in vacuum to afford 14.1 g (63%) of product.

### Example 53:

### (7R)-7-[(Z)-2-(5-amino-1,2,4-thiadiazol-3-yl)-2-(triphenylmethyloxyimino)acetamido-3-[2-(2-L-(N-tert-butoxycarbonyl-4-tert-butylaspartyl)aminoethylthiomethyl)pyrid-3-ylthio]ceph-3-em-4-carboxylate, tert-butyl ester

To a nitrogen purged flask equipped with a magnetic stirrer was added at room temperature *N,N*-dimethylformamide (335 mL, deoxygenated by prolonged purging with a stream of dry nitrogen), (7R)-7-[(Z)-2-(5-amino-1,2,4-thiadiazol-3-yl)-2-(triphenylmethyloxyimino)acetamido-3-chloroceph-3-em-4-carboxylate, *tert*-butyl ester (16.0 g, 22.8 mmol), bis(2-[2-L-(*N*-*tert*-butoxycarbanyl-4-*tert*-butylaspartyl)antinoethylthiomethyl]pyrid-3-yl) disulfide (10.8 g, 11.4 mmol), water (3.4 mL) and triphenylphosphine (6.6 g, 25.1 mmol). After overnight reaction the reaction mixture was partitioned between etyl acetate/hexane 3:1 (v/v) mixture (900 mL) and water (1.5 L). Organic layer was thoroughly washed with water and concentrated under reduced pressure to produce oliy residue. Chromatography on silica gel column (gradient elution with hexane-ethyl acetate) provided (7R)-7-[(Z)-2-(5-amino-1,2,4-thiadiazol-3-yl)-2-(triphenylmethyloxyimino) acetamido-3-[2-(2-L-(*N*-*tert*-butoxycarbonyl-4-*tert*-butylaspartyl)aminoethylthiomethyl) pyrid-3-ylthio]ceph-3-em-4-carboxylate, *tert*-butyl ester (18.4 g, 84%) contaminated with small amount of triphenylphosphne oxide.

### Example 54:

### (7R)-7-[(Z)-2-(5-amino-1,2,4-thiadiazol-3-yl)-2-(triphenylmethyloxyimino)acetamido-3-(2-(2-L-aspartylaminoethylthiomethyl)pyrid-3-ylthio)ceph-3-em-4-carboxylate, tert-butyl ester

To a nitrogen purged flask equipped with a magnetic stirrer was added *N,N*-dimethylformamide (45 mL, deoxygenated by prolonged purging with a stream of dry nitrogen), (7R)-7-[(Z)-2-(5-amino-1,2,4-thiadiazol-3-yl)-2-(triphenylmethyloxyimino)acetamido-3-chloroceph-3-em-4-carboxylate, *tert*-butyl ester (10.5 g, 14.9 mmol) and 2-(2-L-aspartylaminoethylthiomethyl)-3-mercaptopyridine dihydrochloride (7.5 g, 19.3 mmol). The mixture was stirred until complete dissolution of starting materials and was then cooled to 0 °C (ice water bath). Triethanolamine (5.8 g, 38.9 mmol) solution in *N,N*-dimethylformamide (6 mL) was added in one portion. After 4.5 h additional amounts of 2-(2-L-aspartylaminoethylthiomethyl)-3-mercaptopyridine dihydrochloride (0.7 g, 1.8 mmol) in in *N,N*-dimethylformamide (5 mL) and triethanolamine (0.54 g, 3.6 mmol) in *N,N*-dimethylformamide (1 mL) were added. After an additional 1 h, the reaction mixture was poured into a flask containing a vigorously stirred mixture of water (100 mL) and crushed ice (200 mL). Stirring was continued until all of the ice melted. The precipitated crude product was filtered, washed thoroughly with water and dried overnight in vacuo. The crude product was washed three times with ethyl acetate (3 x 200 mL) with repeated thorough trituration with the spatula. The resulting yellow precipitate was dried in vacuo to produce (7R)-7-[(Z)-2-(S-amino- 1,2,4-thiadiazol-3-yl)-2-(triphenylmethyloxyimino)acelamido-3-(2-(2-L-aspartylaminoethylthiomethyl)pyrid-3-ylthio)ceph-3-em-4-carboxylate, *tert*-butyl ester (13.2 g, 90% yield).

### Example 55:

### (7R)-7-[(Z)-2-(5-amino-1,2,4-thiadiazol-3-yl)-2-(triphenylmethyloxyimino)acetamido-3-(2-(2-L-aspartylaminoethylthiomethyl)pyrid-3-ylthio)ceph-3-em-4-carboxylate, tert-butyl ester

To a nitrogen purged flask equipped with a magnetic stirrer was added *N,N*-dimethylformamide (2.4 mL), deoxygenated by prolonged purging with a stream of dry nitrogen 2-(2-L-aspartylaminoethylthiomethyl)-3-mercaptopyridine dihydrochloride (0.22 g, 0.57 mmol) was added. The mixture was stirred until complete dissolution of starting materials and was then cooled to 0 °C (ice water bath). A solution of sodium hydroxide in water (0. 38 mL, 3.0 M, 1.14 mmol) was added followed by addition of (7R)-7-[(Z)-2-(5-amino-1,2,4-thiadiazol-3-yl)-2-(triphenylmethyloxyimino)acetamido-3-chloroceph-3-em-4-carboxylate, *tert*-butyl ester (0.40 g, 0.57 mmol). After 4 h the reaction mixture was poured into a flask containing a vigorously stirred ice water. The precipitated crude product was filtered, washed thoroughly with water and dried overnight in vacuum. The crude product was washed three times with ethyl acetate with repeated thorough trituration with the spatula. The resulting yellow precipitate was dried in vacuo to produce (7R)-7-[(Z)-2-(5-amino-1,2,4-tniadiazol-3-yl)-2-(triphenylmethyloxyimino)acetamido-3-(2-(2-L-aspartylaminoethylthiomethyl)pyrid-3-ylthio)ceph-3-em-4-carboxylate, *tert*-butyl ester (0.46 g, 90% yield).

### Example 56:

### (7R)-7-[(Z)-2-(5-amino-1,2,4-thiadiazol-3-yl)-2-(hydroxyimino)acetamido-3-[2-(2-L-aspartyl)aminoethylthiomethyl)pyrid-3-ylthio]ceph-3-em-4-carborylate, bis-trifluoroacetic acid salt

Into a flask equipped with a magnetic stirring bar and nitrogen purge was charged (7R)-7-[(Z)-2-(5-amino-1,2,4-thiadiazol-3-yl)-2-(triphenylmethyloxyimino)acetamido-3-[2-(2-L-(*N-tert*-butoxycarbonyl-4-*tert*-butylaspartyl)aminoethylthiomethyl)pyrid-3-ylthio]ceph-3-em-4-carboxylate, *tert*-butyl ester (3.5 g, 3 mmol) followed by dichloromethane (20 mL). To the clear light yellow solution was charged triethylsilane (10.5 mL) followed by trifluoroacetic acid (35 mL) dropwise over 2 min. The resulting yellow solution was allowed to stir at 20 °C for 8 h. The acidic solution was poured into rapidly stirring diethyl ether (300 mL) cooled to 0 °C and the resulting white solid was collected by filtration. The filter cake was washed with fresh diethyl ether (2 X 20 mL) and dried in vacuum at to yield 2.41 g (86%) (7R)-7-[(Z)-2-(5-amino-1,2,4-thiadiazol-3-yl)-2-(hydroxyimino)acetamido-3-[2-(2-L-aspartyl)aminoethylthiomethyl)pyrid-3-ylthio]ceph-3-em-4-carboxylate, bis-trifluoroacetic acid salt. NMR: example 120
This salt was processed to the zwitterion form as described previously in example 121.

### Example 57:

### (7R)-7-[(Z)-2-(5-amino-1,2,4-thiadiazol-3-yl)-2-(hydroxyimino)acetamido-3-[2-(2-L-aspartyl)aminoethylthiomethyl)pyrid-3-ylthio]ceph-3-em-4-carboxylate, bis-trifluoroacetic acid salt

Into a flask equipped with a magnetic stirring bar and nitrogen purge was charged *tert*-butyl (7R)-7-[(Z)-2-(5-amino-1,2,4-thiadiazol-3-yl)-2-(triphenylmethyloxyimino)acetamido-3-[2-(2-L-aspartyl)aminoethylthiomethyl)pyrid-3-ylthio]ceph-3-em-4-carboxylate (12.2 g, 12.0 mmol), dichloromethane (61 mL) and triethylsilane (61 mL, 380 mmol). The mixture was cooled to 0°C followed by dropwise addition of trifluoroacetic acid (244 mL, 3.20 mol). Following the addition, the cooling bath was removed, and the reaction was allowed to warm to room temperature. After 3h the mixture was concentrated under reduced pressure to an oily residue. Diisopropyl ether (400 mL) was added with stirring, and the precipitate was stored overnight at 4 °C. The granular precipitate was filtered, thoroughly washed with diisopropyl ether, and dried in vacuum to yield 12.8 g (7R)-7-[(Z)-2-(5-amino-1,2,4-thiadiazol-3-yl)-2-(hydroxyimino)acetamido-3-[2-(2-L-aspartyl)aminoethylthiomethyl)pyrid-3-ylthio]ceph-3-em-4-carboxylate, bis-trifluoroacetic acid salt. NMR: example 120
This salt was processed to the zwitterion form as described previously in example 121.

### Example 58: Evaluation of Prodrugs of Compound 2:

An aspect of the invention is the formation of prodrugs of Compound 2. These prodrugs display improved solubility, allowing their administration to patients at higher concentrations. Several prodrugs of Compound 2 have been synthesized and their solubility has been determined at near physiological pH. The release of Compound 2 is determined by analyzing the *in vitro* rate of hydrolysis of the prodrug by serum enzymes of three species (rat, monkey, and human).

### A. Solubility protocol:

A small amount of a solid salt (5-8 mg) is weighed into screw cap vial. Water is added to dissolve the solid at a high concentration (50-75 mg/mL). To this solution is added small amounts (1-5 µL increments) of 0.1 N aqueous sodium hydroxide until a faint precipitate remains after mixing. The suspension is left at room temperature for 15 minutes with periodic vortexing. An aliquot of the suspension is then transferred to an Eppendorf tube and centrifuged at 14000 rpm for 1.5 minutes. A portion of the supernatant is diluted with water, filtered and quantified by HPLC against a standard calibration curve. The pH of the supernatant is measured using a fine needle probe. For each subsequent determination, using a new sample, more 0.1 N sodium hydroxide is added to increase the pH just slightly, and the rest of the procedure is repeated. Five to seven determinations are used to prepare the pH vs. concentration curve.

When starting with zwitterion, the solid zwitterion is suspended in water. Either 0.1 N sodium hydroxide or 0.1 N hydrochloric acid is added to begin to dissolve the solid. These suspensions are mixed frequently over the 15 minutes at room temperature, then treated as above (centrifugation, dilution and quantitation).

### B. Determination of prodrug cleavage in serum:

Fresh control human serum, fresh control rat serum, and rhesus monkey plasma (heparinized and stored frozen) were preincubated at 37 °C for 15 min in a shaking water bath. The pH of the serum or plasma was measured using test strips. Then 25 µL aliquots of a solution in water of 2 (2 mg/mL) or its respective prodrug were added to each matrix resulting in a final solution volumn of 1 mL. Solutions were incubated at 37 °C for 1 hr, while removing 100 µL aliquotes at times = 0, 15, 30, and 60 min post dose. The aliquotes were added to 200 µL 4% trichloroacetic acid, vortexed, and centrifuged for 10 min at 14,000 rpm in an Eppendorf microcentrifuge. Then 25 µL of each supernatant was analyzed via HPLC*. The degradation of 2 or its respective prodrug was measured as the percentage of the AUC at time t relative to the AUC at time = 0. The rate of release of 2 from its respective prodrug was measured as percentage of the AUC of 2 observed at time t relative to a theoretical possible AUC of 17 as determined by a comparison to a standard concentration curve.
*HPLC conditions: Beckman Ultrasphere C18 column, 5 micron, 4.6mm x 25cm. 1 mL/min of 95% 0.1 M ammonium acetate, pH = 6, 5% acetonitrile ramping to 25% acetonitrile over 20 min with peak detection at 254 and 280 nM.

### Example 59: Biological Activity

### In vitro antibacterial evaluation

### Susceptibility testing

Results of antimicrobial activity of 7-acylamino-3-heteroarylthio-3-cephem carboxylic acids are presented in Table 1 (MIC in µg/mL).

Compounds were evaluated for antimicrobial activity against a panel of bacterial strains using a broth microdilution assay performed as recommended by the NCCLS (1). The minimum inhibitory concentration (MIC) was defined as the lowest concentration of drug which prevented the growth of the bacteria. The following 16 organisms constituted the primary panel of evaluation:

| **Bacteria** | **Strain** | **Characteristic** |
|---|---|---|
| *Staphylococcus aureus* | MSSA ATCC29213 | Wild type |
| *Staphylococcus aureus* | MSSA COL8A | PBP2A MRSA COL |
| *Staphylococcus aureus* | MSSA PC1 | β-lactamase overexpressor |
| *Staphylococcus aureus* | MSSA Smith | Wild type |
| *Staphylococcus aureus* | MRSA COL | PBP2A constitutive/βla- |
| *Staphylococcus aureus* | MRSA 76 | PBP2A constitutive/βla+ |
| *Staphylococcus aureus* | MRSA ATCC33593 | Wild type |
| *Staphylococcus aureus* | MRSA Spain#356 | Clinical isolate |
| *Staphylococcus haemolyticus* | sh005 | Clinical isolate |
| *Enterococcus faecalis* | ATCC29212 | Wild type |
| *Enterococcus faecium* | ATCC35667 | Wild type |
| *Enterococcus faecium* | VanA | Vancomycin^{R} |
| *Enterococcus faecalis* | VanB | Vancomycin^{R} |
| *Enterococcus faecium* | A491 | Clinical isolate/Ampicillin^{R} |
| *Escherichia coli* | ATCC25922 | Wild type |
| *Pseudomonas aeruginosa* | ATCC27853 | Wild type |

1. National Committee for Clinical Laboratory Standards (NCCLS). 1997. Methods for Dilution of Antimicrobial Susceptibility Tests for Bacteria That Grow Aerobically - Fourth Edition; Approved Standard. NCCLS Document M7-A4, Vol. 17 No.2.

For this primary panel, the assay was performed in Mueller-Hinton Broth (MHB) with a final bacterial inoculum of 5 x 10⁵ CFU/mL (from an early-log phase culture) and a final volume of 100 µL. Control drugs, including imipenem, vancomycin, Penicillin G, dicloxacillin (*vs*. MSSA ATCC29213 only), and new compounds were prepared at a concentration equivalent to 2-fold the desired final concentration. Dilution of compounds were prepared directly in the plates by serial 2-fold dilution using a multichannel pipette. Positive and negative growth control were included in each plate.

The bacterial inocula were prepared as follows. For each strain one isolated colony was used to inoculate a volume of 8 mL of Mueller-Hinton broth and these cultures were incubated overnight (20h) at 35 °C in a shaking incubator. At the exception of *Enterococcus* strains, culture were then diluted 1:10 and allowed to grow for an additional one hour at 35 °C in a shaking incubator. The inocula were prepared by diluting the early log-phase (1h) cultures 1:1000 with fresh Mueller-Hinton broth. *E. faecium* and *E. faecalis* were prepared by diluting overnight cultures 1:333 and 1:666, respectively, with fresh Mueller-Hinton broth . A volume of 50 µL of the inocula was added to each well. This procedure resulted in an inoculum of approximately 5 x 10⁵ CFU/mL. The exact inocula were determined by applying 10 pL of 10-fold serial dilution of the bacterial suspensions on TSA. After overnight incubation at 35 °C, colony forming units were manually counted.

Microtiter plates were incubated during 24 h at 35 °C and were read using a microtiterplate reader (Molecular Devices) at 650 nm as well as by visual observation using a microtiterplate reading mirror. The MIC is defined as the lowest concentration of compound at which the visible growth of the organism is completely inhibited.

### Serum binding determination

The approximate measurement of the degree of binding of the compound to human serum proteins can be achieved by determination of the effect of the addition of human serum to the medium in which the MIC values against a selected bacterial strain is determined. The ratio of the MIC value obtained in presence of added human serum to the MIC value determined without serum addition was used as a measure of serum binding and the results of such determination are presented below in Table 2.

In addition to the standard MIC determination described above, the staphylococcal strain ATCC29213 is also tested in a ratio 1:1, human serum:MHB to obtain a preliminary estimate of the bioactivity of test compounds in serum. The assay was performed in Human Serum and Mueller-Hinton Broth (HSMHB) with a final bacterial inoculum of 1 x 10⁶ CFU/mL (from an early-log phase culture) and a final volume of 100 µL. Control drugs, including imipenem, vancomycin, dicloxacillin, and new compounds were prepared in double-concentrated Mueller-Hinton broth at a concentration equivalent to 4-fold the desired final concentration. Dilution of compounds were prepared directly in the plates by serial 2-fold dilution in double-concentrated Mueller-Hinton using a multichannel pipette. Positive and negative growth controls were included in each plate. A total of 50 µL of human serum was added to all wells. *S. aureus* ATCC29213 was grown overnight in MHB (see MIC determinations above), culture was then diluted 1:10 and allowed to grow for an additional one hour at 35 °C in a shaking incubator. The inoculum was prepared by diluting the early log-phase (1h) culture 1:100 with fresh double-concentrated Mueller-Hinton broth. A volume of 25 µL of the inoculum was added to each well. This procedure resulted in an inoculum of approximately 1 x 10⁶ CFU/mL. The exact inoculum was determined by applying 10 µl of 10-fold serial dilution of the bacterial suspension on TSA. After overnight incubation at 35 °C, colony forming units were manually counted.

The more accurate measurement of binding of selected cephalosporin compounds was performed using HPLC as described below. Corresponding values in Table 2 represent percentage of cephalosporin compound which was found to be bound to serum proteins.
1. Serum is placed in a shaking water bath preheated to 37 ± 2 °C for 5 to 10 minutes to heat serum to 37 °C. Serum pH is measured and recorded, while serum is 37 °C using pH paper. Measurements are taken twice, 3 minutes apart. If the pH is not 7.4 ± 0.4 then the pH is adjusted by blowing 5% CO₂/95% O₂ over the serum. After blowing for several minutes, serum is heated to 37 °C and measurements are taken twice, 3 minutes apart using pH paper to determine the pH. Store at 4 °C. The serum is stable for 7 days. Always check the pH before each use.
2. Before any centrifugation, the centrifuge temperature must be adjusted to 25 ± 2 °C. Preheat the centrifuge by spinning for 45 minutes at the same speed to be used in the assay (1900 g) with two tubes of water in the rotor and the temperature set to 25 °C. When the centrifuge stops immediately check the temperature of the tubes of water with a calibrated thermometer. The temperature of the tubes of water must be 25 ± 2 °C. In the event the temperature does not meet the above criteria adjust the temperature setting appropriately and spin for another 45 minutes. Continue spinning and measuring the temperature and adjusting the setting until the temperature is 25 ± 2 °C.
3. Prepare blank ultrafiltrate by pipetting blank pH 7.4 serum into an Amicon Centrifree Micropartition devices, centrifuge: fixed angle rotor, 1900 g, 25 °C for 20 minutes. Heat ultrafiltrate in a shaking water bath preheated to 37 °C. Measure ultrafiltrate pH and adjust to the pH of the serum (7.4 ± 0.2).
4. Pipette 190 ul of blank ultrafiltrate into a set of labeled 1.7 mL polypropylene microfuge tubes for 3 of each level. Pipette 10 µL of the appropriate standard stock 50, 200, 1000 µg/mL to make standards 2.5, 10, and 50 µg/mL respectively. Vortex each tube well. One set of the standard samples are mixed 1:1 with 0.2 M acetic acid and injected onto the HPLC (MSTD). These samples are the membrane standards and will be used to compare to the other standards. Pipette 190 ul of ultrafiltrate and spike with 10 ul of whatever solution (e.g. water) the standards are prepared in to make the zero standard.
5. Pipette 475 µL of blank heated pH 7.4 serum into a set of labeled 1.7 mL polypropylene microfuge tubes for 2 of each (Serum A and Serum B). Pipette 25 µL of the 1000 µg/mL standard stock into each tube, vortex each tube well to make serum standard 50 µg/mL. Remove 100 µL of serum and deproteinate with 200 µL 4% TCA while vortexing to make serum time 0 (serum 50, T0). Place serum tubes in a shaking water bath at 37 °C for 10 minutes. Pipet 100 µL of serum out and deproteinate with 200 µL 4% TCA, to make serum time 10 min (serum 50, T10). Pipet 200 ul serum standard 50 µg/mL into an ultrafiltration device, which is not treated with TCA, to determine free drug in samples (Rat Serum 50).
6. Transfer serum and standard samples to labeled Amicon Centrifree Micropartition filters, and centrifuge: 1900 g, 25 °C for 10 minutes. The extracted serum samples (T0 and T 10) are spun down and supernatant is loaded into HPLC vials.
7. 100 µL of ultrafiltrate sample and 100 µL of 0.2 M acetic acid were mixed together to stabilize the compound in ultrafiltrate.
8. Transfer entire stabilized ultrafiltrate solution to a labeled autoinjector HPLC vial cap with Teflon septa, and inject on HPLC.

### Calculations:

Peak height or area is used in a weighted linear regression.

High levels of binding to human serum proteins limits the concentration of compound available for antibacterial activity in the body. Therefore compounds with levels of human serum binding ≤ 90% are preferred. Data presented in Table 2 shows that among the 7-acylamino-3-heteroarylthio-3-cephem carboxylic acids which are the subject of the present invention for which human serum binding was detennined, the levels of binding are in the desired range. Even more preferred is the level of binding to human serum proteins ≤ 80% which provides even higher concentration of unbound compound available for antibacterial activity in the body. Data presented in Table 2 shows that compounds 2, 2-R exhibit level of binding to human serum proteins ≤ 80%.

### In vitro stability of compounds in rat serum

In general, compounds from the class of 7-acylamino-3-heteroarylthio-3-cephem carboxylic acids have higher chemical reactivity and lower stability towards chemical or enzymatic decomposition than most typical cephalosporins. Many of them were found to have limited stability *in vitro* in rat serum. Results shown in Table 2 represent percentage loss of the initial amount of cephalosporin compound upon 60 min incubation in rat serum.

Procedure for measuring degree of degradation of cephalosporin compounds in rat serum was as follows:

Fresh control rat serum (heparinized and stored frozen) is preincubated at 37 °C for 15 min in a shaking water bath. pH is measured using test strips. 25 µL of a 2 mg/mL solution cephalosporin compound (in water) of is then added to each matrix to make 1000 µL. Solutions were incubated at 37 °C. 100ul of solution was taken out at time = 0, 15, 30, and 60 min post dose and added 200 µL 4% trichloroacetic acid, vortexed, and centrifuged for 10 min at 14,000 rpm in an Eppendorfmicrocentrifuge. 25 µL of each supernatant was injected onto HPLC. Experiment was repeated using 25 µL of a 0.2 mg/mL cephalosporin compound. Degradation is measured as the percentage of HPLC peak area at time t relative to peak area at time 0.

HPLC conditions: Beckman Ultrasphere C18 column. 5u. 4.6mm x 25cm. 1 mL/min of 95% 0.1 M ammonium acetate, pH 6, 5% ACN going to 75% buffer/organic over 20min. UV monitored at 254 and 280 nm.

Certain combinations of 7-acylamino-substitution and 3-heteroarylthio-substitution at the cephalosporin system result in improved stability of 7-acylamino-3-heteroarylthio-3-cephem carboxylic acid compounds. Examples of such compounds (Table 2) are Compound 1, Compound 2-R, Compound 2-S, Compound 2, Compound 3, and Compound 4. The names and structures of these compounds are listed in section II of the Detailed Description of the Preferred Embodiments, above.

### In vivo evaluation

### Pharmacokinetic evaluation in rat

Improvements in serum stability of certain compounds from the class of 7-acylamino-3-heteroarylthio-3-cephem carboxylic acids over other compounds from this class are the important factors determining improved pharmacokinetic parameters of such compounds.

Although serum stability is recognized as only one of the potential factors influencing clearance of compound from the body, the positive effect of increased serum stability on clearance of certain compounds from the class of 7-acylamino-3-heteroarylthio-3-cephem carboxylic acids determined in rats is substantial.

Determination of clearance of cephalosporin compounds was performed following an example protocol described below which was used for determination of clearance of compound 2.

Four male SD rats with femoral and jugular vein catheters were obtained from Hilltop Lab Animals, Inc. (Scottdale, PA). The catheters in the femoral and jugular veins were filled with 10 units/mL heparin in saline, and kept patent by changing the solution daily. The animals were quarantined for a minimum of two days prior to use, and were maintained on a 12-h light:dark schedule with constant access to food and water before and during the study. On the day of dosing, the animals weighed 212 ± 0.005 g (mean ± s.d.).

All animal experimentation was conducted in accordance with the NIH Guide for the Care and Use of Laboratory Animals.

A 4 mg/mL (active component) dosing solution of mesylate salt of compound 17 was prepared in sterile saline for injection. After a 0.2 mL preinjection blood sample was collected from the jugular catheter, 20 mg/kg over 20 min as a 5 mL/kg infusion was initiated through the femoral catheter. Blood samples (0.2 mL aliquots) were collected from the jugular vein catheter 5, 15, 20, 25, 30, and 45 min, and 1, 2, 3, and 4 h after the start of the infusion. The blood was centrifuged for 10 min and the serum was stored ≤ -70 °C until analysis by HPLC.

The plasma concentrations from individual animals were fit to a two-compartment model with zero order input and first-order elimination from the central compartment using weighted nonlinear regression (WinNonlin, Pharsight, Palo Alto, CA). Pharmacokinetic parameters were calculated from the fitted parameters using standard equations (Gibaldi M, Perrier D. 1982. Pharmacokinetics, 2^{nd} ed., Marcel Dekker Inc., New York.).

### Efficacy Evaluations

Compounds with superior activity in vitro when compared to reference antibiotics, are further evaluated in a murine model for lethal bacteremic peritonitis following the procedure described below.

10 mice/group were injected intraperitoneally with 2.98x10⁷ cfu *S. aureus* ATCC 13709 (Smith diffuse) and treated at 0 and 2hrs post-challenge with serial two-fold doses of new cephalosporin antibiotic, imipenem and vancomycin subcutaneously in order to determine the ED₅₀. Male Swiss-Webster mice weighing originally 24.2 - 26.1 g were obtained from Charles Rivers Labs, Hollister, CA. Mice were housed 10 per cage and given free access to water and mouse chow.

*Staphylococcus aureus* ATCC 13709 (Smith diffuse) was grown overnight at 37 °C in Brain-heart Infusion broth (BHIB). The following morning, it was subcultured to fresh BHIB and incubated for 4-5h at 37 °C. The log-phase growth cells were washed once with physiological saline and adjusted to the desired concentration by correlation of absorbency at OD 600 nm with predetermined plate counts (NCCLS, 1994). The cell suspension was mixed with an equal volume of sterile 14% hog-gastric mucin (2). Inoculum was kept in an ice bath until used (<1h).

Mice were challenged via an injection of 0.5 mL bacterial suspension intraperitoneally. The bacterial challenge was 2.98 x 10⁷ cfu/mouse.

Imipenem (Primaxin IV; Lot 7294A), vancomycin (Vancocin; Lot 8MU60W), and new cephalosporin antibiotic were prepared by serial two-fold dilutions to concentrations of 0.078 - 0.005 mg/mL, 1.2 - 0.075 mg/mL, 0.3 - 0.02 mg/mL, 0.3 - 0.02 mg/mL and 0.3 - 0.02 mg/mL respectively in sterile water (1). All antibiotics were administered subcutaneously in 0.1 mL volumes at 0 and 2 hours post-challenge. Concentrations of all compounds are based on the weight of the bioactive ingredients.

The 50% effective dose (ED₅₀) was calculated by the probit method (3) utilizing a program entitled Probit (Nycomed-Salutar).

The names and structures of the compounds listed by their compound number in Tables 1 and 2, below, are shown in section II of the Detailed Description of the Preferred Embodiments, above.

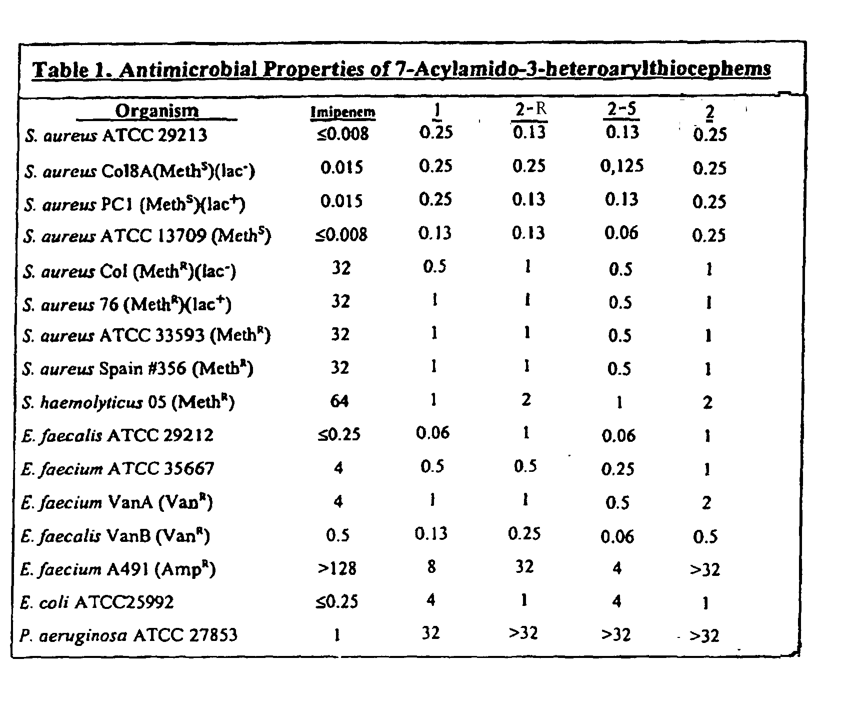

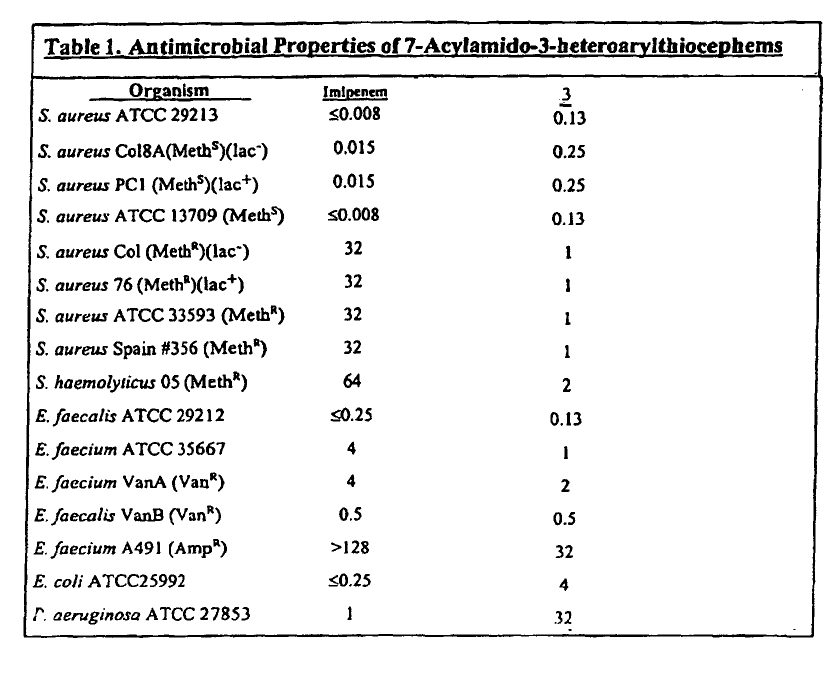

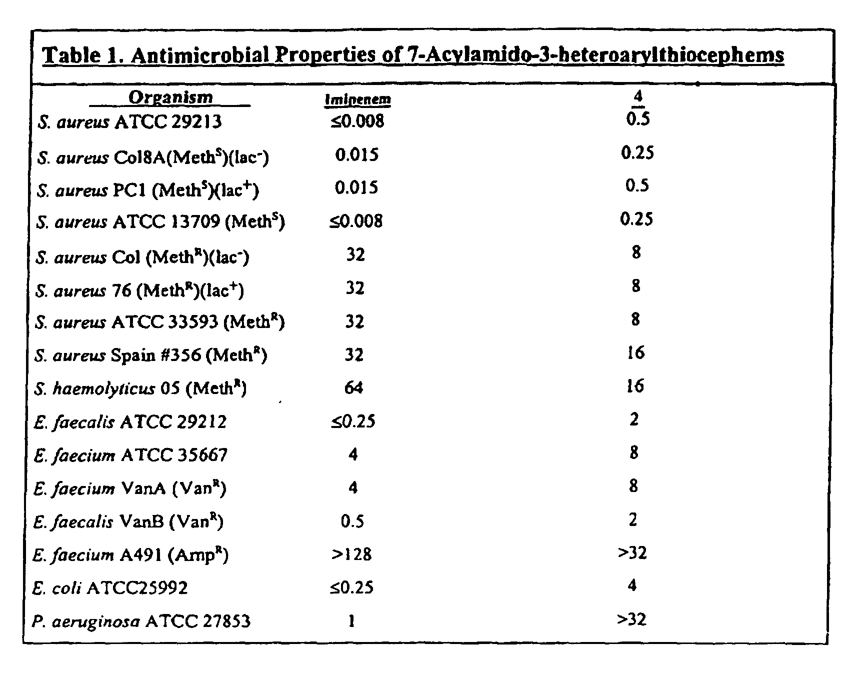

**Table 2.**

| **Serum effects and *in vivo* properties of 7-Acylamido-3- heteroarylthiocephems** | | | | | | | | | |
|---|---|---|---|---|---|---|---|---|---|
| Cmpd | Serum Binding^{*a*} | | | Human serum binding effect^{*b*} | Stability in rat serum^{*c*} | Pharmacokinetics^{*d*} | | Efficacy in murine septicemia^{*e*} | |
| | rat | rhesus | human | | | Clearance (L/h/kg) | Dose (mg/kg) | Dose (mg/kg)/ %survival | ED₅₀ |
| Van^{*f*} | | | 55 | 2 | | | | 10/100 | 2.41 |
| | | | | | | | | 5/100 | |
| | | | | | | | | 2.5/60 | |
| | | | | | | | | 1.25/20 | |
| | | | | | | | | 0.625/10 | |
| 1 | 85 | | 90 | 2 | 9% | 0.79 | 20.0 | 5/100 | <0.31 |
| | | | | | | | | 2.5/100 | |
| | | | | | | | | 1.25/100 | |
| | | | | | | | | 0.625/100 | |
| | | | | | | | | 0.312/30 | |
| 2-R | | | 60 | 2 | 10% | | | 5/100 | <0.31 |
| | | | | | | | | 2.5/100 | |
| | | | | | | | | 1.25/100 | |
| | | | | | | | | 0.625/100 | |
| | | | | | | | | 0.312/90 | |
| 2-S | 76 | 88 | 83 | 2 | 38% | | | | |
| 2 | 74 | 81 | 66 | 2 | 2% | 0.5 | 16.4 | 2.0/100 | 0.39 |
| | | | | | | | | 1.0/100 | |
| | | | | | | | | 0.5/50 | |
| | | | | | | | | 0.25/20 | |
| | | | | | | | | 0.12/10 | |
| | | | | | | | | 1.25/58 | |
| | | | | | | | | 0.625/30 | |
| | | | | | | | | 0.312/10 | |
| | | | | | | | | 0.156/20 | |
| 3 | 97 | 97 | 90 | 2 | 3% | 0.73 | 20.0 | 5/80 | 0.79 |
| | | | | | | | | 2.5/80 | |
| | | | | | | | | 1.25/70 | |
| | | | | | | | | 0.625/0 | |
| | | | | | | | | 0.312/0 | |
| 4 | | | | 4 | | | | | |
| Imip. | | | | 2 | 20% | | | 0.625/100 | 0.07 |
| *g* | | | | | | | | 0.312/100 | |
| | | | | | | | | 0.156/100 | |
| | | | | | | | | 0.078/40 | |
| | | | | | | | | 0.039/20 | |

| | | | | | | | | | |
|---|---|---|---|---|---|---|---|---|---|
| LEGEND a. % of compound bound to serum as determined by HPLC (see "Serum binding determination" for details). | | | | | | | | | |
| b. Ratio of MIC value determined with and without human serum added for *S. aureus* ATCC29213. | | | | | | | | | |
| c. % loss after 60 min exposure to rat serum as determined by HPLC (See "*In vitro* stability of compounds in rat serum" for details). | | | | | | | | | |
| d. Clearance in rat determined at given dose (see "Pharmacokinetic evaluation in rat" for details). | | | | | | | | | |
| e. Efficacy in treatment of experimental infection in mice (see "Efficacy evaluation" for details). | | | | | | | | | |
| f. Vancomycin | | | | | | | | | |
| g. Imipenem | | | | | | | | | |

### CONCLUSION

One skilled in the art would readily appreciate that the present invention is well adapted to carry out the objects and obtain the ends and advantages mentioned, as well as those inherent therein. The molecular complexes and the methods, procedures, treatments, molecules, specific compounds described herein are presently representative of preferred embodiments are exemplary and are not intended as limitations on the scope of the invention.

All patents and publications mentioned in the specification are indicative of the levels of those skilled in the art to which the invention pertains.

The invention illustratively described herein suitably may be practiced in the absence of any element or elements, limitation or limitations which is not specifically disclosed herein. Thus, for example, in each instance herein any of the terms "comprising", "consisting essentially of' and "consisting of" may be replaced with either of the other two terms. The terms and expressions which have been employed are used as terms of description , and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

In addition, where features or aspects of the invention are described in terms of Markush groups, those skilled in the art will recognize that the invention is also thereby described in terms of any individual member or subgroup of members of the Markush group. For example, if X is described as selected from the group consisting of bromine, chlorine, and iodine, claims for X being bromine and claims for X being bromine and chlorine are fully described.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims.

## Claims

1. A compound having the chemical formula: 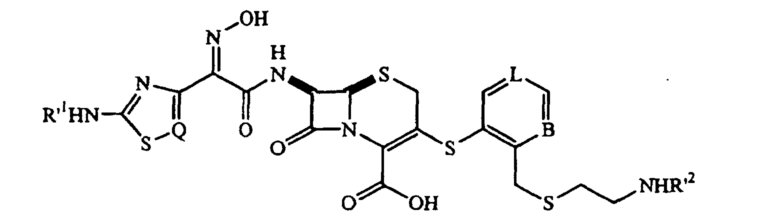 or a pharmaceutically acceptable salt thereof, wherein:
R'¹ is selected from the group consisting of hydrogen and -C (O)CH(NH₂)CH₃;
R'² is hydrogen,
B or L is nitrogen and the other is carbon; and,
Q is selected from the group consisting of nitrogen and -CX, wherein X is selected from the group consisting of hydrogen and chlorine.

2. A prodrug compound having the chemical formula: 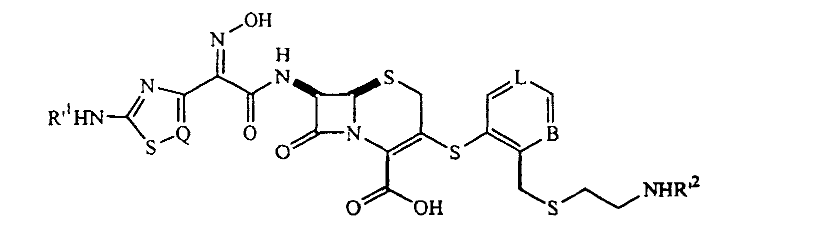 or a pharmaceutically acceptable salt thereof, wherein:
R'¹ is selected from the group consisting of hydrogen and -C (O)CH(NH₂)CH₃;
R'² is an acyl group that is cleaved by an enzyme found in mammals and is selected from the group consisting of -C(O)-R⁸⁸, -C(O)-OR⁸⁹, -C(O)-CH(NHR'³)-alk₄, and 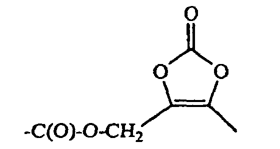
wherein:
R⁸⁸ is 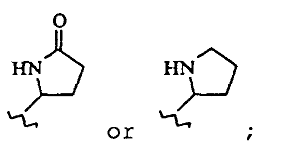 R'³ is selected from the group consisting of hydrogen, alkyl, -C(O)-OR⁸⁹, and -C(O)-CH(NH₂)-alk₄;
alk₄ is selected from the group consisting of hydrogen, and optionally substituted alkyl, wherein said alkyl is optionally substituted with one or more substituents selected from the group consisting of hydrogen, phenyl, -COOH, -C(O)-OR⁸⁹, -C(O)NH₂, -OH, -SH, -NH₂, and 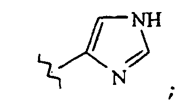 and,
R⁸⁹ is selected from the group consisting of benzhydryl, *t*-butyl, allyl, *p*-nitrobenzyl, benzyl, *p-* or o-nitrobenzyl, 2,2,2-trichloroethyl, allyl, cinnamyl, benzhydryl, 2-chloroallyl, *t*-amyl, trityl, 4-methoxytrityl, 4,4'-dimethoxytrityl, trimethylsilyl, *t*-butyldimethylsilyl, β-(trimethylsilyl)ethyl, 4- or 2-methoxybenzyl, 2,4-dimethoxybenzyl, 3,4-dimethoxybenzyl, 2,4,6-trimethoxybenzyl, methoxymethyl, and 3,3-dimethylallyl.
B or L is nitrogen and the other is carbon; and,
Q is selected from the group consisting of nitrogen and -CX, wherein X is selected from the group consisting of hydrogen and chlorine.

3. The compound of claim 2, wherein R'³ is selected from the group consisting of hydrogen, methyl, and -C(O)-CH(NH₂)CH₃.

4. The compound of any of claims 1-3, wherein alk₄ is selected from the group consisting of hydrogen, -CH₃, -CH(CH₃)₂, -CH₂OH, -CH₂NH₂, -CH₂CH₂NH₂, -CH₂CH₂CH₂NH₂, -CH₂CH₂CH₂CH₂NH₂, -CH₂COOH, -CH₂CH₂COOH, -CH₂-C(O)NH₂, -CH₂CH₂-C(O)NH₂, and

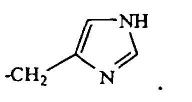

5. A compound according to claim 1 selected from the group consisting of:
(7R)-7-[(Z)-2-(2-amino-5-chlorothiazol-4-yl)-2-(hydroxylamino)acetamido]-3-[2-(2-aminoethylthiomethyl)pyrid-3-ylthio]-3-cephem-4-carboxylic acid,
(7R)-7-[(Z)-2-(5-amino-1,2,4-thiadiazol-3-yl)-2-(hydroxylamino)acetamido]-3-[2-(2-aminoethylthiomethyl)pyrid-3-ylthio]-3-cephem-4-carboxylic acid,
(7R)-7-[(Z)-2-(2-amino-5-chlorothiazol-4-yl)-2-(hydroxylamino)acetamido]-3-[4-(2-aminoethylthiomethyl)pyrid-3-ylthio]-3-cephem-4-carboxylic acid,
(7R)-7-[(Z)-2-(5-amino-1,2,4-thiadiazol-3-yl)-2-(hydroxylamino)acetamido]-3-[4-(2-aminoethylthiomethyl)pyrid-3-ylthio]-3-cephem-4-carboxylic acid,
(7*R*)-7[(Z)-2-(2-amino-5-chlorothiazol-4-yl)-2-(hydroxyimino)acetamido]-3-[2-(2-aminoethylthiomethyl)pyrid-3-ylthio]-3-cephem-4-carboxylic acid,
(7*R*)-7[(Z)-2-(5-amino-1,2,4-thiadiazol-3-yl)-2-(hydroxyimino)acetamido]-3-[2-(2-aminoethylthiomethyl)pyrid-3-ylthio]-3-cephem-4-carboxylic acid,
(7*R*)-7[(Z)-2-(2-amino-5-chlorothiazol-4-yl)-2-(hydroxyimino)acetamido]-3-[4-(2-aminoethylthiomethyl)pyrid-3-ylthio]-3-cephem-4-carboxylic acid,
(7R)-7-[(Z)-2-(5-amino-1,2,4-thiadiazol-3-yl)-2-(hydroxyimino)acetamido]-3-[4-(2-aminoethylthiomethyl)pyrid-3-ylthio]-3-cephem-4-carboxylic acid.

6. A prodrug compound according to claim 2 selected from the group consisting of:
(7R)-7-[(Z)-2-(5-amino-1,2,4-thiadiazol-3-yl)-2-(hydroxylamino)acetamido]-3-[2-(2-guanidinoethylthiomethyl) pyrid-3-ylthio]-3-cephem-4-carboxylic acid,
(7R)-7-[(Z)-2-(2-amino-5-chlorothiazol-4-yl)-2-(hydroxyimino)acetamido]-3-[2-(2-guanidinoethylthiomethyl) pyrid-3-ylthio]-3-cephem-4-carboxylic acid,
(7*R*)-7[(Z)-2-(5-amino-1,2,4-thiadiazol-3-yl)-2-(hydroxyimino)acetamido]-3-[2-(2-guanidinoethylthiomethyl) pyrid-3-ylthio]-3-cephem-4-carboxylic acid,
(7R)-7-[(2)-2-(5-amino-1,2,4-thiadiazol-3-yl)-2-(hydroxyimino)acetamido]-3-{2-[2-N-(L)-ornithylaminoethylthiomethyl]pyrid-3-ylthio)-3-cephem-4-carboxylic acid,
(7R)-7-[(Z)-2-(5-amino-1,2,4-thiadiazol-3-yl)-2-(hydroxyimino)acetamido]-3-{2-[2-N-(L)-prolylaminoethylthiomethyl]pyrid-3-ylthio)-3-cephem-4-carboxylic acid,
(7R)-7-[(Z)-2-(5-N-(L)-alanylamino-1,2,4-thiadiazol-3-yl)-2-(hydroxyimino)-acetamido]-3-{2-[2-aminoethylthiomethyl]pyrid-3-ylthio}-3-cephem-4-carboxylic acid,
(7R)-7-[(Z)-2-(5-amino-1,2,9-thiadiazol-3-yl)-2-(hydroxyimino)acetamido]-3-{2-[2-N-(L,L)-alanylalanylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-glycylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-aspartylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-alanylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-(N_{α}-methyl)alanylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-histidylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-valylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-asparagylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-lysylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-serylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-glutaminylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-(5-methyl-1,3-dioxolan-4-en-2-on-4-yl)methoxycarbonyl)aminoethylthiomethyl]pyrid-3-ylthio]-3-cephem-4-carboxylic acid,
(7R)-7-[(Z)-2-(5-amino-1,2,4-thiadiazol-3-yl)-2-(hydroxyimino)acetamido]-3-[2-(2-N-(L)-pyroglutamylaminoethylthiomethyl]pyrid-3-ylthio}-3-cephem-4-carboxylic acid,
(7R)-7-[(Z)-2-(5-amino-1,2,4-thiadiazol-3-yl)-2-(hydroxyimino)acetamido]-3-{2-[2-N-(L)-aspartylamidoethylthiomethyl]pyrid-3-ylthio}-3-cephem-4-carboxylic acid.

7. A compound having the chemical formula: 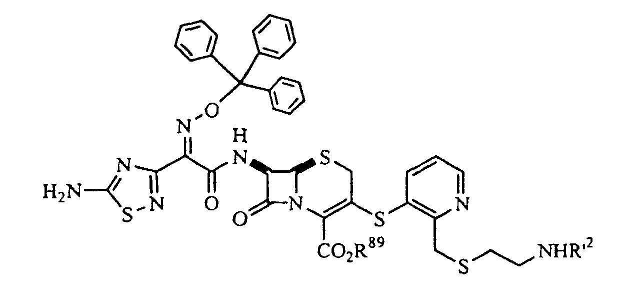 wherein R'² is hydrogen
R⁸⁹ is selected from the group consisting of benzhydryl, *t*-butyl, allyl, *p*-nitrobenzyl, benzyl, *p-* or o-nitrobenzyl, 2,2,2-trichloroethyl, allyl, cinnamyl, benzhydryl, 2-chloroallyl, *t*-amyl, trityl, 4-methoxytrityl, 4,4'-dimethoxytrityl, trimethylsilyl, *t*-butyldimethylsilyl, β-(trimethylsilyl)ethyl, 4- or 2-methoxybenzyl, 2,4-dimethoxybenzyl, 3,4-dimethoxybenzyl, 2,4,6-trimethoxybenzyl, methoxymethyl, and 3,3-dimethylallyl.

8. A prodrug compound having the chemical formula: 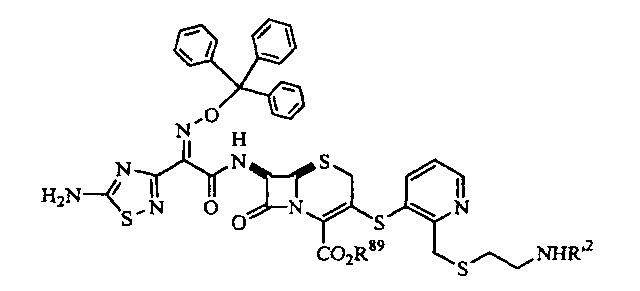 wherein R'² is selected from the group consisting of -C(O)-R⁸⁸, -C(O)-OR⁸⁹, -C(O)-CH (NHR'³)-alk₄, and 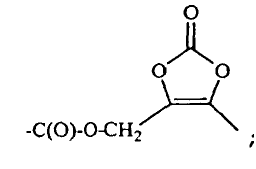 wherein,
R⁸⁸ is 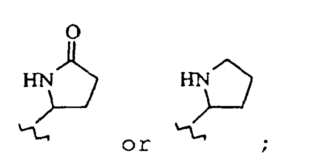 R'³ is selected from the group consisting of hydrogen, -C(O)-OR⁸⁹, and -C(O)-CH(NH₂)-alk₄;
alk₄ is selected from the group consisting of hydrogen, and optionally substituted alkyl, wherein said alkyl is optionally substituted with one or more substituents selected from the group consisting of hydrogen, phenyl, -COOH, -C(O)-OR⁸⁹, -C(O)NH₂, -OH, -SH, -NH₂, and 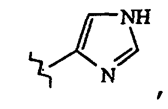 and,
R⁸⁹ is selected from the group consisting of benzhydryl, *t*-butyl, allyl, *p*-nitrobenzyl, benzyl, *p-* or *o*-nitrobenzyl, 2,2,2-trichloroethyl, allyl, cinnamyl, benzhydryl, 2-chloroallyl, *t*-amyl, trityl, 4-methoxytrityl, 4,4'-dimethoxytrityl, trimethylsilyl, *t*-butyldimethylsilyl, β-(trimethylsilyl)ethyl, 4- or 2-methoxybenzyl, 2,4-dimethoxybenzyl, 3,4-dimethoxybenzyl, 2,4,6-trimethoxybenzyl, methoxymethyl, and 3,3-dimethylallyl.

9. The compound or salt of any of claims 1-8, wherein the compound is active against methicillin-resistant Staphylococcal bacteria selected from the group consisting of *S. aureus* Col (Meth^{R})(lac-), *S. aureus* 76 (Meth^{R})(lac+), *S. aureus* ATCC 33593 (Meth^{R}), *S. aureus* Spain #356 (Meth^{R}), and *S. haemolyticus* 05 (Meth^{R}).

10. An antibacterial composition for treating a methicillin-resistant Staphylococcal bacterial infection, comprising a therapeutically effective amount of a compound or salt of any of claims 1-8 in a pharmaceutically acceptable carrier.

11. Use of the compound of any of claims 1-8 for the manufacture of an antibacterial composition for treating a methicillin-resistant Staphylococcal bacterial infection.

## Patentansprüche

1. Eine Verbindung mit der chemischen Formel: 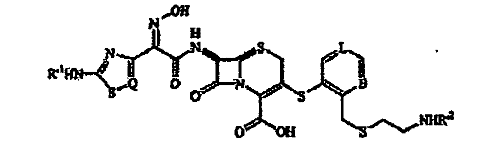 oder ein pharmazeutisch annehmbares Salz davon, wobei:
R'¹ aus der Gruppe, die aus Wasserstoff und -C(O)CH(NH₂)CH₃ besteht, ausgewählt wird;
R'² Wasserstoff ist,
B oder L Stickstoff ist, wobei das jeweils andere Kohlenstoff ist; und
Q aus der Gruppe, die aus Stickstoff und -CX besteht ausgewählt wird, wobei X aus der Gruppe, die aus Wasserstoff und Chlor besteht, ausgewählt wird.

2. Eine Prodrug-Verbindung mit der chemischen Formel: 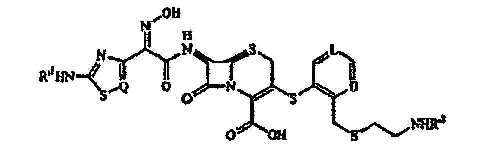 oder ein pharmazeutisch annehmbares Salz davon, wobei:
R'¹ aus der Gruppe, die aus Wasserstoff und -C(O)CH(NH₂)CH₃ besteht, ausgewählt wird;
R'² eine Acylgruppe ist, die durch ein Enzym, das in Säugetieren gefunden wird, gespalten wird und aus der Gruppe, die aus -C(O)-R⁸⁸, -C(O)-OR⁸⁹, -C(O)-CH(NHR'³)-alk₄, besteht, ausgewählt wird, und 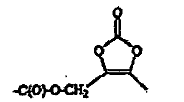
wobei:
R⁸⁸ 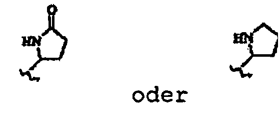 ist;
R'³ aus der Gruppe, die aus Wasserstoff, Alkyl, -C(O)-OR⁸⁹ und -C(O)-CH(NH₂)-alk₄ besteht, ausgewählt wird;
alk₄ aus der Gruppe, die aus Wasserstoff und optional substituiertem Alkyl besteht, wobei besagtes Alkyl optional mit einem oder mehreren Substituenten ausgewählt aus der Gruppe bestehend aus Wasserstoff, Phenyl, -COOH, -C(O)-OR⁸⁹, -C(O)NH₂, -OH, -SH, -NH₂, und 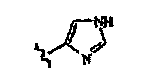 ausgewählt wird; und
R⁸⁹ aus der Gruppe, die aus Benzhydryl, *t*-Butyl, Allyl, *p*-Nitrobenzyl, Benzyl, *p*- oder *o*-Nitrobenzyl, 2,2,2-Trichlorethyl, Allyl, Cinnamyl, Benzhydryl, 2-Chlorallyl, *t*-Amyl, Trityl, 4-Methoxytrityl, 4,4'-Dimethoxytrityl, Trimethylsilyl, *t*-Butyldimethylsilyl, β-(Trimethylsilyl) ethyl, 4- oder 2-Methoxybenzyl, 2,4-Dimethoxybenzyl, 3,4-Dimethoxybenzyl, 2,4,6-Trimethoxybenzyl, Methoxymethyl und 3,3-Dimethylallyl besteht, ausgewählt wird;
B oder L Stickstoff ist, wobei das jeweils andere Kohlenstoff ist; und
Q aus der Gruppe, die aus Stickstoff und -CX besteht ausgewählt wird, wobei X aus der Gruppe, die aus Wasserstoff und Chlor besteht, ausgewählt wird.

3. Die Verbindung gemäß Anspruch 2, wobei R'³ aus der Gruppe, die aus Wasserstoff, Methyl und -C(O)-CH(NH₂)CH₃ besteht, ausgewählt wird.

4. Die Verbindung gemäß einem der Ansprüche 1-3, wobei alk₄ aus der Gruppe, die aus Wasserstoff, -CH₃, -CH(CH₃)₂, -CH₂OH, -CH₂NH₂, -CH₂CH₂NH₂, -CH₂CH₂CH₂NH₂, -CH₂CH₂CH₂CH₂NH₂, CH₂COOH, -CH₂CH₂COOH, -CH₂-C(O)NH₂, -CH₂CH₂-C(O)NH₂, und 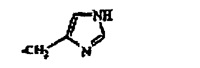 besteht, ausgewählt wird.

5. Eine Verbindung gemäß Anspruch 1, ausgewählt aus der Gruppe, die besteht aus:
(7R)-7-[(Z)-2-(2-amino-5-chlorothiazol-4-yl)-2-(hydroxylamino)acetamido]-3-[2-(2-aminoethylthiomethyl) pyrid-3-ylthio]-3-cephem-4-carbonsäure,
(7R)-7-[(Z)-2-(5-amino-1,2,4-thiadiazol-3-yl)-2-(hydroxylamino)acetamido]-3-(2-(2-aminoethylthiomethyl) pyrid-3-ylthio]-3-cephem-4-carbonsäure,
(7R)-7-[(Z)-2-(2-amino-5-chlorothiazol-4-yl)-2-(hydroxylamino)acetamido]-3-[4-(2-aminoethylthiomethyl) pyrid-3-ylthio]-3-cephem-4-carbonsäure,
(7R)-7-[(Z)-2-(5-amino-1,2,4-thiadiazol-3-yl)-2-(hydroxylaniino)acetamido]-3-[4-(2-aminoethylthiomethyl) pyrid-3-ylthio]-3-cephem-4-carbonsäure,
(7R)-7-[(Z)-2-(2-amino-5-chlorothiazol-4-yl)-2-(hydroxyimino)acetamido]-3-[2-(2-aminoethylthiomethyl) pyrid-3-ylthio]-3-cephem-4-carbonsäure,
(7R)-7-[(Z)-2-(5-amino-1, 2, 4-thiadiazol-3-yl)-2-(hydroxyimino)acetamido]-3-[2-(2-aminoethylthiomethyl) pyrid-3-ylthio]-3-cephem-4-carbonsäure,
(7R)-7-[(Z)-2-(2-amino-5-chlorothiazol-4-yl)-2-(hydroxyimino)acetamido]-3-[4-(2-aminoethylthiomethyl) pyrid-3-ylthio]-3-cephem-4-carbonsäure,
(7R)-7-[(Z)-2-(5-amino-1, 2, 4-thiadiazol-3-yl)-2-(hydroxyimino)acetamido]-3-[4-(2-aminoethylthiomethyl) pyrid-3-ylthio]-3-cephem-4-carbonsäure

6. Eine Prodrug-Verbindung gemäß Anspruch 2, ausgewählt aus der Gruppe, die besteht aus:
(7R)-7-[(Z)-2-(5-amino-1, 2, 4-thiadiazol-3-yl)-2-(hydroxylamino)acetamido]-3-[2-(2-guanidinoethylthiomethyl) pyrid-3-ylthio]-3-cephem-4-carbonsäure,
(7R)-7-[(Z)-2-(2-amino-5-chlorothiazol-4-yl)-2-(hydroxyimino)acetamido]-3-[2-(2-guanidinoethylthiomethyl)pyrid-3-ylthio]-3-cephem-4-carbonsäure,
(7R)-7-[(Z)-2-(5-amino-1, 2, 4-thiadiazol-3-yl)-2-(hydroxyimino)acetamido]-3-[2-(2-guanidinoethylthiomethyl)pyrid-3-ylthio]-3-cephem-4-carbonsäure,
(7R)-7-[(Z)-2-(5-amino-1,2,4-thiadiazol-3-yl)-2-(hydroxyimino)acetamido]-3-{2-[2-N-(L)-ornithylaminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-(2-[2-N-(L)-prolylaminoethylthiomethyl]pyrid-3-ylthio)-3-cephem-4-carbonsäure,
(7R)-7-[(Z)-2-(5-N-(14-alanylamino-1, 2, 4-thiadiazol-3-yl)-2-(hydroxyimino)-acetamido]-3-{2-[2-aminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-{2-[2-N-(L,L)-alanylalanylaminoethylthiomethyl]pyrid-3-ylthio}-3-cephem-4-carbonsäure,
(7R)-7-((Z)-2-(5-amino-1, 2, 4-thiadiazol-3-yl)-2-(hydroxyimino)acetamido]-3-{ 2-[2-N-glycylaminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-{2[2-N-(L)-aspartylaminoethylthiomethyl]pyrid-3-ylthio}-3-cephem-4-carbonsäure,
(7R)-7-[(Z)-2-(5-amino-1, 2, 4-thiadiazol-3-yl)-2-(hydroxyimino)acetamido]-3-(2-[2-N-(L)-alanylaminoethylthiomethyl]pyrid-3-ylthio}-3-cephem-4-carbonsäure,
(7R)-7-((Z)-2-(5-amino-1,2,4-thiadiazol-3-yl)-2-(hydroxyimino)acetamido]-3-{2-[2-N-(L)-(N_{α}-methyl)alanylaminoethylthiomethyl]pyrid-3-ylthio)-3-cephem-4-carbonsäure,
(7R)-7-[(Z)-2-(5-amino-1, 2, 4-thiadiazol-3-yl)-2-(hydroxyimino)acetamido]-3-{2-[2-N-(L)-histidylaminoethylthiomethyl]pyrid-3-ylthio]-3-cephem-4-carbonsäure,
(7R)-7-[(Z)-2-(5-amino-1, 2, 4-thiadiazol-3-yl)-2-(hydroxyimino)acetamido]-3-{2-[2-N-(L)-valylaminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-{2-N-(L)-asparagylaminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-{2-(2-N-(L)-lysylaminoethylthiomethyl]pyrid-3-ylthio)-3-cephem-4-carbonsäure,
(7R)-7-((Z)-2-(5-amino-1,2,4-thiadiazol-3-yl)-2-(hydroxyimino)acetamido]-3-{2-[2-N-(L)-serylaminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-{2-[2-N-(L)-glutaminylaminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-{2-[2-(5-methyl-1,3-dioxolan-4-en-2-on-4-yl)methoxycarbonyl)aminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-{2-(2-N-(L)-pyroglutamylaminoethylthiomethyl]pyrid-3-ylthio}-3-cephem-4-carbonsäure,
(7R)-7-[(Z)-2-(5-amino-1,2,4-thiadiazol-3-yl)-2-(hydroxyimino)acetamido]-3-{2-[2-N-(L)-aspartylamidoethylthiomethyl]pyrid-3-ylthio}-3-cephem-4-carbonsäure.

7. Eine Verbindung mit der chemischen Formel: 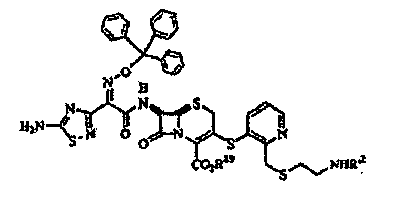 wobei R'² Wasserstoff ist
R⁸⁹ aus der Gruppe, die aus Benzhydryl, *t*-Butyl, Allyl, *p*-Nitrobenzyl, Benzyl, *p*- oder *o*-Nitrobenzyl, 2,2,2-Trichlorethyl, Allyl, Cinnamyl, Benzhydryl, 2-Chlorallyl, *t*-Amyl, Trityl, 4-Methoxytrityl, 4,4'-Dimethoxytrityl, Trimethylsylyl, *t*-Butyldimethylsilyl, β-(Trimethylsilyl) ethyl, 4- oder 2-Methoxybenzyl, 2,4-Dimethoxybenzyl, 3,4-Dimethoxybenzyl, 2,4,6-Trimethoxybenzyl, Methoxymethyl und 3,3-Dimethylallyl besteht, ausgewählt wird.

8. Eine Prodrug-Verbindung mit der chemischen Formel: 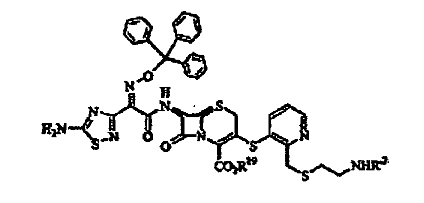 wobei R'² aus der Gruppe ausgewählt wird, die besteht aus -C(O)-R⁸⁸, -C(O)-OR⁸⁹, -C(O)-CH(NHR'³)-alk₄, und 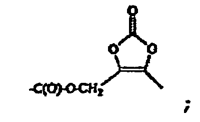 wobei
R⁸⁸ 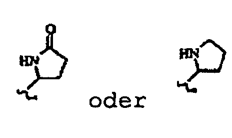 ist;
R'³ aus der Gruppe ausgewählt wird, die besteht aus Wasserstoff, -C(O)-OR⁸⁹ und -C(O)-CH(NH₂)-alk₄;
alk⁴ aus der Gruppe ausgewählt wird, die besteht aus Wasserstoff und optional substituiertem Alkyl, wobei besagtes Alkyl optional mit einem oder mehreren Substituenten substituiert ist, die aus der Gruppe bestehend aus Wasserstoff, Phenyl, -COOH, -C(O)-OR⁸⁹, -C(O)NH₂, -OH, -SH, -NH₂, und 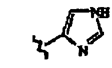 ausgewählt werden, und
R⁸⁹ aus der Gruppe, die aus aus Benzhydryl, *t*-Butyl, Allyl, *p*-Nitrobenzyl, Benzyl, *p*- oder *o*-Nitrobenzyl, 2,2,2-Trichlorethyl, Allyl, Cinnamyl, Benzhydryl, 2-Chlorallyl, *t*-Amyl, Trityl, 4-Methoxytrityl, 4,4'-Dimethoxytrityl, Trimethylsylyl, *t*-Butyldimethylsilyl, β-(Trimethylsilyl)ethyl, 4- oder 2-Methoxybenzyl, 2,4-Dimethoxybenzyl, 3,4-Dimethoxybenzyl, 2,4,6-Trimethoxybenzyl, Methoxymethyl und 3,3-Dimethylallyl, besteht, ausgewählt wird.

9. Die Verbindung oder das Salz gemäß einem der Ansprüche 1-8, wobei die Verbindung gegen Methicillinresistente Staphylococcen, die aus der Gruppe bestehend aus *S. aureus* Col (Meth^{R})(lac-), *S. aureus* 76 (Meth^{R})(lac+), *S. aureus* ATCC 33593 (Meth^{R}), *S. aureus* Spain #356 (Meth^{R}), und *S. haemolyticus* 05 (Meth^{R}) ausgewählt werden, aktiv ist.

10. Eine antibakterielle Zusammensetzung zur Behandlung einer Methicillin-resistenten bakteriellen Staphylococcen-Infektion, die eine therapeutisch wirksame Menge einer Verbindung oder Salzes gemäß einem der Ansprüche 1-8 in einem pharmazeutisch akzeptablen Träger umfasst.

11. Verwendung der Verbindung gemäß einem der Ansprüche 1-8 zur Herstellung einer antibakteriellen Zusammensetzung zur Behandlung einer Methicillin-resistenten bakteriellen Staphylococcen-Infektion.

## Revendications

1. Composé répondant à la formule chimique : 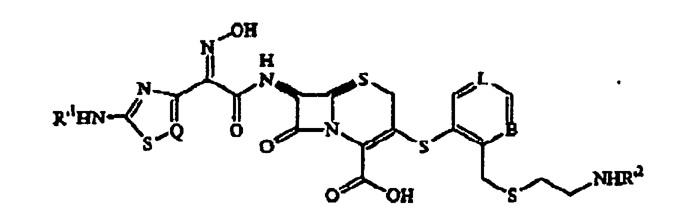 ou un sel pharmaceutiquement acceptable de celui-ci, dans laquelle :
R'¹ est choisi dans le groupe constitué par un atome d'hydrogène et -C(O)CH(NH₂)CH₃ ;
R'² est un atome d'hydrogène ;
B ou L est un atome d'azote et l'autre est un atome de carbone ; et
Q est choisi dans le groupe constitué par un atome d'azote et -CX, où X est choisi dans le groupe constitué par un atome d'hydrogène et de chlore.

2. Composé précurseur de médicament répondant à la formule chimique : 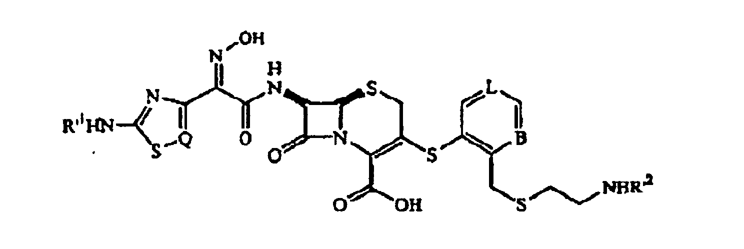 ou un sel pharmaceutiquement acceptable de celui-ci, dans laquelle :
R'¹ est choisi dans le groupe constitué par un atome d'hydrogène et -C(O)CH(NH₂)CH₃ ;
R'² est un groupe acyle qui est coupé par une enzyme présente chez les mammifères et est choisi dans le groupe constitué par -C(O)-R⁸⁸, -C(O)-R⁸⁹,
-C(O)-CH(NHR'³)-alk₄, et 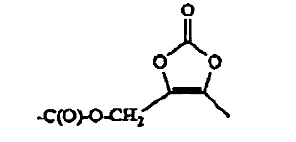 où
R⁸⁸ est 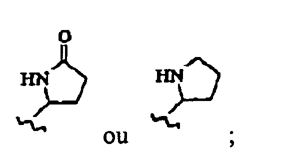
R'³ est choisi dans le groupe constitué par un atome d'hydrogène, un groupe alkyle, -C(O)-OR⁸⁹ et -C(O)-CH(NH₂)-alk₄ ;
alk₄ est choisi dans le groupe constitué par un atome d'hydrogène, un groupe alkyle éventuellement substitué, où ledit groupe alkyle est éventuellement substitué par un ou plusieurs substituants choisis dans le groupe constitué par un atome d'hydrogène, un groupe phényle, -COOH, -C(O)-R⁸⁹, -C(O)NH₂, -OH, -SH, -NH₂ et 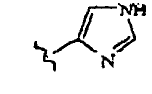 et
R⁸⁹ est choisi dans le groupe constitué par un groupe benzhydryle, t-butyle, allyle, p-nitrobenzyle, benzyle, p- ou o-nitrobenzyle, 2,2,2-trichloroéthyle, allyle, cinnamyle, benzhydryle, 2-chloroallyle, t-amyle, trityle, 4-méthoxytrityle, 4,4'-diméthoxytrityle, triméthylsilyle, t-butyldiméthylsilyle, β-(triméthylsilyl)éthyle, 4- ou 2-méthoxybenzyle, 2,4-diméthoxybenzyle, 3,4-diméthoxybenzyle, 2,4,6-triméthoxybenzyle, méthoxyméthyle et 3,3-diméthylallyle ; B ou L est un atome d'azote et l'autre est un atome de carbone ; et
Q est choisi dans le groupe constitué par un atome d'azote et -CX, où X est choisi dans le groupe constitué par un atome d'hydrogène et de chlore.

3. Composé selon la revendication 2, dans lequel R'³ est choisi dans le groupe constitué par un atome d'hydrogène, un groupe méthyle et -C(O)-CH(NH₂)CH₃.

4. Composé selon l'une quelconque des revendications 1 à 3, dans lequel alk₄ est choisi dans le groupe constitué par un atome d'hydrogène, -CH₃, -CH(CH₃)₂, -CH₂OH, -CH₂NH₂, -CH₂CH₂NH₂, -CH₂CH₂CH₂NH₂, -CH₂CH₂CH₂CH₂NH₂, -CH₂COOH, -CH₂CH₂COOH, -CH₂-C(O)NH₂, -CH₂CH₂-C(O)NH₂, et

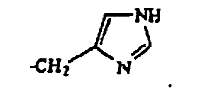

5. Composé selon la revendication 1 choisi dans le groupe constitué par :
l'acide (7R)-7-[(Z)-2-(2-amino-5-chlorothiazol-4-yl)-2-(hydroxylamino)-acétamido]-3-[2-(2-aminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylamino)-acétamido]-3-[2-(2-aminoéthylthiométhyl)pyrid-3-ylthio]-3-céphèm-4-carboxylique,
l'acide (7R)-7-[(Z)-2-(2-amino-5-chlorothiazol-4-yl)-2-(hydroxylamino)-acétamido]-3-[4-(2-aminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylamino)-acétamido]-3-[4-(2-aminoéthylthiométhyl)pyrid-3-ylthio]-3-céphèm-4-carboxylique,
l'acide (7R)-7-[(Z)-2-(2-amino-5-chlorothiazol-4-yl)-2-(hydroxylimino)-acétamido]-3-[2-(2-aminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-aminoéthylthiométhyl)pyrid-3-ylthio]-3-céphèm-4-carboxylique,
l'acide (7R)-7-[(Z)-2-(2-amino-5-chlorothiazol-4-yl)-2-(hydroxylimino)-acétamido]-3-[4-(2-aminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[4-(2-aminoéthylthiométhyl)pyrid-3-ylthio]-3-céphèm-4-carboxylique,

6. Composé précurseur de médicament selon la revendication 2 choisi dans le groupe constitué par :
l'acide (7R)-7-[(Z)-2-(5-amino-1,2,4-thiadiazol-3-yl)-2-(hydroxylamino)-acétamido]-3-[2-(2-guanidinoéthylthiométhyl)pyrid-3-ylthio]-3-céphèm-4-carboxylique,
l'acide (7R)-7-[(Z)-2-(2-amino-5-chlorothiazol-4-yl)-2-(hydroxylimino)-acétamido]-3-[2-(2-guanidinoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-guanidinoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-ornithylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-propylaminoéthylthiométhyl)pyrid-3-ylthio]-3-céphèm-4-carboxylique,
l'acide (7R)-7-[(Z)-2-(5-(N)-(L)-alanylamino-1,2,4-thiadiazol-3-yl)-2-(hydroxylimino)-acétamido]-3-[2-(2-aminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L,L)-alanylalanylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-glycylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-aspartylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-alanylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-(N_{α}-méthyl)alanylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-histidylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-valylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-asparagylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-lysylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-sérylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-glutaminylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-{2-[2-(5-méthyl-1,3-dioxolan-4-én-2-on-4-yl)méthoxycarbonyl)-aminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-pyroglutamylaminoéthylthiométhyl)pyrid-3-ylthio]-3-céphèm-4-carboxylique,
l'acide (7R)-7-[(Z)-2-(5-amino-1,2,4-thiadiazol-3-yl)-2-(hydroxylimino)-acétamido]-3-[2-(2-N-(L)-aspartylaminoéthylthiométhyl)pyrid-3-ylthio]-3-céphèm-4-carboxylique.

7. Composé répondant à la formule chimique : 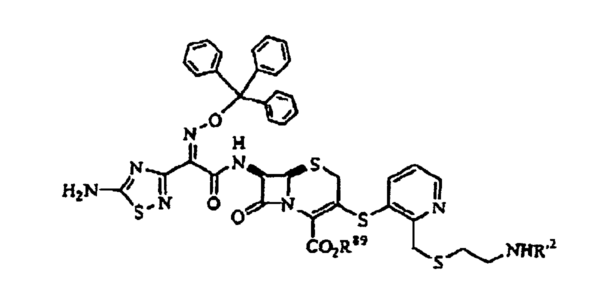 dans laquelle :
R'² est un atome d'hydrogène ;
R⁸⁹ est choisi dans le groupe constitué par un groupe benzhydryle, t-butyle, allyle, p-nitrobenzyle, benzyle, p- ou o-nitrobenzyle, 2,2,2-trichloroéthyle, allyle, cinnamyle, benzhydryle, 2-chloroallyle, t-amyle, trityle, 4-méthoxytrityle, 4,4'-diméthoxytrityle, triméthylsilyle, t-butyldiméthylsilyle, β-(triméthylsilyl)éthyle, 4- ou 2-méthoxybenzyle, 2,4-diméthoxybenzyle, 3,4-diméthoxybenzyle, 2,4,6-triméthoxybenzyle, méthoxyméthyle et 3,3-diméthylallyle.

8. Composé précurseur de médicament répondant à la formule chimique : 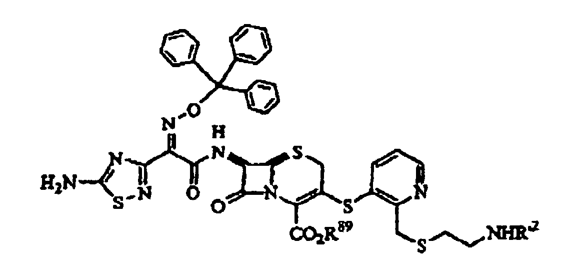 dans laquelle :
R'² est choisi dans le groupe constitué par -C(O)-R⁸⁸, -C(O)-R⁸⁹, -C(O)-CH(NHR'³)-alk₄, et 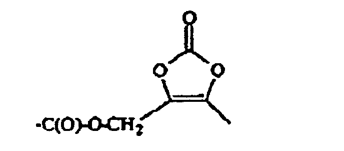 où
R⁸⁸ est 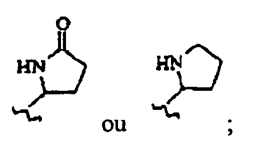
R'³ est choisi dans le groupe constitué par un atome d'hydrogène, -C(O)-OR⁸⁹ et -C(O)-CH(NH₂)-alk₄ ;
alk₄ est choisi dans le groupe constitué par un atome d'hydrogène, un groupe alkyle éventuellement substitué, où ledit groupe alkyle est éventuellement substitué par un ou plusieurs substituants choisis dans le groupe constitué par un atome d'hydrogène, un groupe phényle, -COOH, -C(O)-R⁸⁹, -C(O)NH₂, -OH, -SH, -NH₂ et 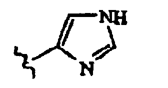 et
R⁸⁹ est choisi dans le groupe constitué par un groupe benzhydryle, t-butyle, allyle, p-nitrobenzyle, benzyle, p- ou o-nitrobenzyle, 2,2,2-trichloroéthyle, allyle, cinnamyle, benzhydryle, 2-chloroallyle, t-amyle, trityle, 4-méthoxytrityle, 4,4'-diméthoxytrityle, triméthylsilyle, t-butyldiméthylsilyle, β-(triméthylsilyl)éthyle, 4- ou 2-méthoxybenzyle, 2,4-diméthoxybenzyle, 3,4-diméthoxybenzyle, 2,4,6-triméthoxybenzyle, méthoxyméthyle et 3,3-diméthylallyle.

9. Composé ou sel selon l'une quelconque des revendications 1 à 8, dans lequel le composé est actif vis à vis de bactéries Staphylococcales résistantes à la méthicilline choisies dans le groupe constitué par S. aureus Col (Meth^{R})(lac-), S. aureus 76 (Meth^{R})(lac+), S. aureus ATCC 33593 (Meth^{R}), S. aureus Spain #356 (Meth^{R}) et S. haemolyticus 05 (Meth^{R}).

10. Composition antibiotique pour le traitement d'une infection par des bactéries staphylococcales résistantes à la méthicilline, comprenant une quantité efficace au plan thérapeutique d'un composé ou d'un sel selon l'une quelconque des revendications 1 à 8 dans un vecteur pharmaceutiquement acceptable.

11. Utilisation du composé selon l'une quelconque des revendications 1 à 8 pour la fabrication d'une composition antibiotique pour le traitement d'une infection par des bactéries staphylococcales résistantes à la méthicilline.
